(19) Europäisches Patentamt
European Patent Office
Office européen des brevets

(11) Veröffentlichungsnummer : **0 413 666 B1**

(12) # EUROPÄISCHE PATENTSCHRIFT

(45) Veröffentlichungstag der Patentschrift :
**13.04.94 Patentblatt 94/15**

(21) Anmeldenummer : **90810603.2**

(22) Anmeldetag : **09.08.90**

(51) Int. Cl.$^5$ : **C07C 53/21,** C07C 53/50, C07C 69/63, C07C 51/00, C07C 67/00, C07C 233/00, C07C 231/00, A01N 37/02, A01N 37/18

(54) **Buttersäurederivate.**

(30) Priorität : **18.08.89 CH 3012/89**
**07.03.90 CH 727/90**

(43) Veröffentlichungstag der Anmeldung :
**20.02.91 Patentblatt 91/08**

(45) Bekanntmachung des Hinweises auf die Patenterteilung :
**13.04.94 Patentblatt 94/15**

(84) Benannte Vertragsstaaten :
**AT BE CH DE DK ES FR GB GR IT LI LU NL**

(56) Entgegenhaltungen :
**CHEMICAL ABSTRACTS, Band 94, Nr. 3, 19. JUnner 1981, Columbus, Ohio, USA HUFF, ROGER K. et al. "Syn- thesis and thermolysis of highly halogenated delta 1- -pyrazolines" Seite 437, Spalte 2, Zusammenfassung-Nr. 15 674n**
**CHEMICAL ABSTRACTS SERVICE, 1965-1971, Columbus, Ohio, USA "Registry Handbook" Seite 927R, Spalte 2, Reg.-Nr. 2016-74-2**

(56) Entgegenhaltungen :
**CHEMICAL ABSTRACTS, Band 94, Nr. 19, 11. Mai 1981, Columbus, Ohio, USA MARTIN, PIERRE et al. "Copper(I)-catalyzed radical additions of polyhalogenated compounds to olefins. Part 1. Synthesis of polyhalogenated butyryl chlorides, precursors of pyrethroid haloketenes" Seite 600, Spalte 2, Zu- sammenfassung-Nr. 156 258s**
**CHEMICAL ABSTRACTS, Band 96, Nr. 5, 1 Februar 1982, Columbus, Ohio, USA HEINZER, FRANZ et al. "A new synthesis of DL-armentomycin and related 2-amino-4- -polyhalobutyric acids" Seite 601, Spalte 1, Zusammenfassung-Nr 34 492 j**

(73) Patentinhaber : **CIBA-GEIGY AG**
**Klybeckstrasse 141**
**CH-4002 Basel (CH)**

(72) Erfinder : **Maienfisch, Peter, Dr.**
**Traugott Meyer-Strasse 5**
**CH-4147 Aesch (CH)**
Erfinder : **Steiner, Eginhard, Dr.**
**Obere Hofackerstrasse 3**
**CH-4414 Füllinsdorf (CH)**
Erfinder : **Böger, Manfred**
**Wilhelm Glockstrasse 14**
**D-7858 Weil am Rhein 5 (DE)**

EP 0 413 666 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Derivate von 4-Chlor-4,4-difluorbuttersäuren, Verfahren und Zwischenprodukte zu ihrer Herstellung, Schädlingsbekämpfungsmittel, welche diese Verbindungen enthalten, sowie ihre Verwendung bei der Kontrolle von Schädlingen.

Die erfindungsgemässen 4-Chlor-4,4-difluorbuttersäurederivate entsprechen der Formel I

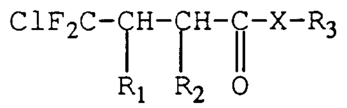

(I)

worin

$R_1$ und $R_2$ unabhängig voneinander Wasserstoff, $C_1$-$C_4$-Alkyl oder $C_1$-$C_4$-Halogenalkyl,
$R_3$ Wasserstoff oder einen organischen Rest und
X Sauerstoff oder -$NR_4$- bedeuten,
wobei $R_4$ für Wasserstoff oder $C_1$-$C_6$-Alkyl steht.

Aus der Literatur ist die Klasse der polyhalogenierten Buttersäurechloride als Zwischenprodukte für pyrethroide Haloketone aus Helv. Chim. Acta 63, p. 1947-1957 (1980) bekannt.

Der in der Definition von $R_3$ genannte organischen Rest steht für ein beliebiges organisches Radikal, das in Form eines Alkohols oder eines Amins an die Carbonylgruppe der 4-Chlor-4,4-difluorbuttersäure gebunden werden kann. Vorzugsweise ist die Alkohol- oder Aminofunktion dieses organischen Radikals an ein Kohlenstoffatom gebunden. Damit wird der Rest $R_3$ vorzugsweise über ein Kohlenstoffatom an die -CO-X-Gruppe gebunden. Beispielsweise steht für $R_3$ jeweils substituiertes oder unsubstituiertes $C_1$-$C_{20}$-Alkyl, $C_3$-$C_7$-Cycloalkyl, $C_3$-$C_{20}$-Alkenyl, $C_3$-$C_{20}$-Alkinyl, Benzyl oder Aryl. Vorzugsweise steht $R_3$ im Rahmen der vorliegenden Erfindung für $C_1$-$C_{20}$-Alkyl, $C_3$-$C_7$-Cycloalkyl, $C_3$-$C_{20}$-Alkenyl, $C_3$-$C_{20}$-Alkinyl, Aryl, $C_3$-$C_{20}$-Halogenalkenyl, $C_3$-$C_{20}$-Halogenalkinyl; durch Halogen oder $C_1$-$C_4$-Alkyl substituiertes $C_3$-$C_7$-Cycloalkyl; durch Halogen, $C_1$-$C_4$-Alkyl, $C_1$-$C_4$-Halogenalkyl, $C_1$-$C_{12}$-Alkoxy, $C_1$-$C_4$-Halogenalkoxy, $C_1$-$C_4$-Alkylthio, Nitro, Cyan, Benzoyl, Halogenbenzoyl, Phenoxy, Halogenphenoxy, $C_1$-$C_4$-Alkylphenoxy, $C_1$-$C_4$-Halogenalkylphenoxy, Tri-$C_1$-$C_4$-alkylsilyl, N-Pyrrolidinyl, N-Piperidinyl, N-Pyrrolidin-2-onyl, N-Piperidin-2-onyl, $C_1$-$C_4$-Alkylamino, Di-$C_1$-$C_4$-alkylamino, Anilino, N-$C_1$-$C_4$-Alkylanilino, N-Formylanilino, N-$C_1$-$C_6$-Alkylcarbonylanilino, Phenylthio oder Halogenphenylthio substituiertes Aryl; durch einen über Sauerstoff oder Schwefel gebundenen unsubstituierten oder substituierten aromatischen oder nichtaromatischen, mono- oder bicyclischen Heterocyclus substituiertes Phenyl, wobei sowohl der Heterocyclus als auch der Phenylring jeweils durch Halogen, $C_1$-$C_4$-Alkyl, Nitro, $C_1$-$C_4$-Halogenalkyl, $C_1$-$C_4$-Halogenalkoxy, $C_1$-$C_4$-Alkoxy, $C_1$-$C_4$-Alkylthio oder Cyclopropyl substituiert sein können; oder durch Hydroxy, Halogen, Di-$C_1$-$C_4$-alkylamino, $C_1$-$C_4$-Alkoxy, $C_1$-$C_4$-Halogenalkoxy, $C_2$-$C_6$-Alkoxyalkoxy, $C_1$-$C_4$-Halogenalkylthio, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy, $C_3$-$C_7$-Cycloalkyl, Aryl, Aryloxy, Arylthio, Arylsulfonyl, Arylsulfinyl, Arylsulfonyloxy, Arylcarbonyl oder Pyridyl substituiertes $C_1$-$C_{20}$-Alkyl; wobei die Aryl- und Pyridylgruppen jeweils durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Nitro, Cyan, Phenoxy, Halogenphenoxy, Phenylthio oder Halogenphenylthio substituiert sein können.

In der Definition der erfindungsgemässen Formel I sollen die einzelnen generischen Begriffe wie folgt verstanden werden:

Bei den als Substituenten in Betracht kommenden Halogenatomen handelt es sich sowohl um Fluor und Chlor als auch um Brom und Jod, wobei Fluor, Chlor und Brom bevorzugt sind. Halogen ist dabei als selbständiger Substituent oder als Teil eines Substituenten zu verstehen wie im Halogenalkyl, Halogenalkylthio, Halogenalkoxy, Halogenphenylthio oder Halogenphenoxy.

Die als Substituenten in Betracht kommenden Alkyl-, Alkylthio-, Alkoxyalkoxy- und Alkoxyreste können geradkettig oder verzweigt sein. Als Beispiele solcher Alkyle seien Methyl, Aethyl, Propyl, Isopropyl, Butyl, i-Butyl, sek.Butyl, tert.Butyl oder Pentyl, Hexyl, Octyl, Decyl, Dodecyl und ihre Isomeren genannt. Als geeignete Alkoxyreste seien unter anderem genannt: Methoxy, Aethoxy, Propoxy, Isopropoxy oder Butoxy und ihre Isomeren. Alkylthio steht beispielsweise für Methylthio, Aethylthio, Isopropylthio, Propylthio oder die isomeren Butylthio.

Sind die als Substituenten in Betracht kommenden Alkyl-, Alkoxy-, Alkenyl-, Alkinyl- oder Arylgruppen durch Halogen substituiert, so können sie nur teilweise oder auch perhalogeniert sein. Dabei gelten für Halogen, Alkyl und Alkoxy die oben gegebenen Definitionen. Beispiele der Alkylelemente dieser Gruppen sind das

ein- bis dreifach durch Fluor, Chlor und/oder Brom substituierte Methyl wie beispielsweise $CHF_2$ oder $CF_3$; das ein- bis fünffach durch Fluor, Chlor und/oder Brom substituierte Aethyl wie zum Beispiel $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl wie z.B. $CH_2CHBrCH_2Br$, $CF_2CHFCF_3$, $CH_2CF_2CF_3$ oder $CH(CF_3)_2$; das ein- bis neunfach durch Fluor, Chlor und/oder Brom substituierte Butyl oder eines seiner Isomeren wie zum Beispiel $CF(CF_3)CHFCF_3$ oder $CH_2(CF_2)_2CF_3$.

Sind die unter $R_3$ definierten Alkyl-, Cycloalkyl- oder Arylgruppen durch andere Substituenten substituiert, so können sie ein- oder mehrfach durch den gleichen oder verschiedene der aufgezählten Substituenten substituiert sein. Vorzugsweise sind in dem substituierten Gruppen ein oder zwei weitere Substituenten vorhanden.

Bei den als Substituenten in Betracht kommenden Cycloalkylresten handelt es sich beispielsweise um Cyclopropyl, Cyclobutyl, Cyclopentyl oder Cyclohexyl.

Alkenyl- und Alkinylgruppen enthalten eine oder mehrere, vorzugsweise nicht mehr als drei, ungesättigte Kohlenstoff-Kohlenstoff-Bindungen. Die Doppel- oder Dreifachbindungen sind von der Verknüpfungsstelle zur Brücke X durch mindestens ein gesättigtes Kohlenstoffatom getrennt. Typische Vertreter sind Allyl, Methalllyl, 2-Butenyl, 3-Butenyl) Propargyl, 2-Butinyl oder 3-Butinyl.

Aryl steht für einen aromatischen Kohlenwasserstoffrest. Vorzugsweise wird unter Aryl Phenyl oder Naphthyl verstanden.

Beispiele für Alkoxyalkoxyreste sind Methoxymethoxy, Methoxyäthoxy, Aethoxyäthoxy, Aethoxymethoxy, Propoxymethoxy, Propoxyäthoxy, Methoxypropoxy, Butoxymethoxy oder Propoxyäthoxy.

Beispiele für Alkoxycarbonylreste sind Methoxycarbonyl, Aethoxycarbonyl, Propoxycarbonyl, Isopropoxycarbonyl oder Butoxycarbonyl. Alkylcarbonyl steht beispielsweise für Acetyl, Propionyl, Butyryl oder Valeryl, sowie Isomere davon. Alkylcarbonyloxy steht zum Beispiel für Acetoxy, Propionyloxy oder Butyryloxy.

Beispiele für aromatische und nichtaromatische, mono- oder bicyclische Heterocyclen, welche an unter $R_3$ definierte Phenylkerne über ein Sauerstoff- oder Schwefelatom gebunden sind, sind die folgenden cyclischen Grundkörper: Pyridin, Pyrazin, Pyridazin, Pyrimidin, Pyrrolidin, Thiazol, Thiadiazol, Oxazol, Benzothiazol, Triazin, Oxadiazol, Chinolin, Chinoxalin, Chinazolin, Isochinolin, Phthalazin, Naphthyridin, Cinnolin, Pteridin, Triazol, Piperidin oder Benzoxazol. Diese Heterocyclen sind vorzugsweise über eine Sauerstoff- oder Schwefelbrücke an die 4-Position des Phenylkerns gebunden. Die Sauerstoff- oder Schwefelbrücke selbst wird von einem Kohlenstoffatom des Heterocyclus getragen. Sowohl der Phenylkern als auch der Heterocyclus können weitere Substituenten tragen, beispielsweise je bis zu drei Reste aus der Gruppe Halogen, Alkyl, Halogenalkyl und Alkoxy oder je ein oder zwei Substituenten aus der Gruppe Nitro, Halogenalkoxy, Alkylthio und Cycloalkyl. Insgesamt ist die Anzahl der Substituenten am Phenylring und am Heterocyclus zusammen in der Regel nicht grösser als vier. Vorzugsweise tragen diese beiden Gruppen zusammen nicht mehr als 3 weitere Substituenten aus der Reihe Chlor, Brom, Methyl, Aethyl und Trifluormethyl, und sind über ein Sauerstoffatom miteinander verbunden.

Unter diesen Verbindungen sind besonders solche erwähnenswert, worin $R_3$ für in 4-Stellung durch einen über Sauerstoff gebundenen aromatischen mono- oder bicyclischen Heterocyclus aus der Reihe Pyridin, Pyrimidin oder Benzothioazol substituiertes Phenyl steht, wobei beide aromatischen Ringe unsubstituiert sind oder zusammen höchstens drei weitere Substituenten aus der Reihe Chlor, Brom, Methyl, Aethyl und Trifluormethyl tragen.

Insbesondere können die durch einen Heterocyclus über Sauerstoff oder Schwefel substituierten Phenylreste unter der Definition von $R_3$ beispielsweise folgende individualisierte Bedeutungen haben:

-4-(3-Methylthiadiazol-5-yloxy)-phenyl,
-4-(4-Bromthiazol-2-yloxy)-phenyl,
-4-(5-Trifluormethylpyrid-2-yloxy)-phenyl,
-4-(3,5-Dichlorpyrid-2-yloxy)-phenyl,
-4-(3-Chlor-5-trifluormethylpyrid-2-yloxy)-phenyl,
-3-(5-Trifluormethylpyrid-2-yloxy)-phenyl,
-2,6-Dimethyl-4-(4-trifluormethylphenoxy)-phenyl,
-2,6-Diisopropyl-4-(N-formylanilino)-phenyl,
-2,6-Diisopropyl-4-(N-methylanilino)-phenyl,
-4-Phenylthio-phenyl,
-4-(2-Cyclopropyl-4-trifluormethyl-pyrimidin-6-yloxy)-phenyl,
-4-(2-tert.Butyl-4-trifluormethyl-pyrimidin-6-yloxy)-phenyl,
-4-(2-Methylthio-4-methyl-pyrimidin-6-yloxy)-phenyl,
-4-(2-Methyl-4-trifluormethyl-pyrimidin-6-yloxy)-phenyl,
-4-(6-Trifluormethoxy-benzothiazol-2-yloxy)-phenyl,

-4-(6-Chlorbenzothiazol-2-yloxy)-phenyl,
-4-(6-Nitrobenzothiazol-2-yloxy)-phenyl,
-4-(6-Methoxybenzothiazol-2-yloxy)-phenyl,
-4-(5-Trifluormethyl-benzothiazol-2-yloxy)-phenyl,
-3,5-Dichlor-4-(3-chlor-5-trifluormethylpyrid-2-yloxy)-phenyl,
-2,6-Dimethyl-4-(3-chlor-5-trifluormethylpyrid-2-yloxy)-phenyl,
-4-Chlor-3-(3,5-bis-trifluormethylpyrid-2-yloxy)-phenyl,
-4-Chlor-3-(5-chlor-3-fluorpyrid-2-yloxy)-phenyl,
-2,6-Dimethyl-4-[3-chlor-5-(2,2-dichlor-1,1,2-trifluoräthyl)-pyrid-2-yloxy]-phenyl,
-4-[3-Chlor-5-(2,2-dichlor-1,1,2-trifluoräthyl)-pyrid-2-yloxy]-phenyl,
-4-(N-Acetyl-pyrid-3-ylamino)-phenyl,
-2,4-Dichlor-3-(3-chlor-5-trifluormethylpyrid-2-yloxy)-phenyl,
-4-Aethoxycarbonyl-3-(3-chlor-5-trifluormethylpyrid-2-yloxy)-phenyl,
-3-Methyl-4-(3-chlor-5-trifluormethylpyrid-2-yloxy)-phenyl,
-3,5-Dichlor-4-(5-trifluormethylpyrid-2-yloxy)-phenyl,
-4-(6-Chlor-4-trifluormethylpyrid-2-yloxy)-phenyl,
-4-Brom-3-(3-chlor-5-trifluormethylpyrid-2-yloxy)-phenyl,
-3-(5-Trifluormethylpyrid-2-yloxy)-phenyl,
-2,6-Dimethyl-4-(3,5-dichlorpyrid-2-ylthio)-phenyl,
-2,6-Dimethyl-4-(2-chlorpyridazin-6-yloxy)-phenyl,
-2,6-Dimethyl-4-(pyrimidin-2-yloxy)-phenyl,
-2,6-Dimethyl-4-(pyrazin-2-yloxy)-phenyl,
-2,6-Dimethyl-4-(6-chlor-chinoxalin-2-yloxy)-phenyl,
-2,6-Dimethyl-4-(6-trifluormethyl-chinoxalin-2-yloxy)-phenyl,
-2,6-Dimethyl-4-(chinolin-2-yloxy)-phenyl,
-4-Chlor-2-fluor-5-(3-methyl-1,2,4-thiadiazol-5-yloxy)-phenyl,
-2,6-Dimethyl-4-(4-brom-thiazol-2-yloxy)-phenyl oder
-4-Methyl-3-(3-chlor-5-trifluoromethylpyrid-2-yloxy)-phenyl.

Unter den Verbindungen der Formel I sind solche Untergruppen herauszuheben, in denen entweder

a) $R_1$ und $R_2$ unabhängig voneinander Wasserstoff oder $C_1$-$C_4$-Alkyl bedeuten, oder

b) X Sauerstoff, -NH-, $-NCH_3$- oder $-NC_2H_5$- bedeutet, oder

c) $R_3$ für Wasserstoff, $C_1$-$C_{20}$-Alkyl, $C_3$-$C_7$-Cycloalkyl, $C_3$-$C_{20}$-Alkenyl, $C_3$-$C_{20}$-Alkinyl, Phenyl, Naphthyl, $C_3$-$C_{20}$-Halogenalkenyl, $C_3$-$C_{20}$-Halogenalkinyl; durch Fluor, Chlor, Brom oder $C_1$-$C_3$-Alkyl substituiertes $C_3$-$C_7$-Cycloalkyl; durch Fluor, Chlor, Brom oder $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, $C_1$-$C_4$-Alkylphenoxy, $C_1$-$C_4$-Halogenalkylphenoxy, Tri-$C_1$-$C_4$-alkylsilyl, N-Pyrrolidinyl, N-Piperidinyl, N-Pyrrolidin-2-onyl, N-Piperidin-2-onyl, $C_1$-$C_4$-Alkylamino, Di-$C_1$-$C_4$-alkylamino, Anilino, N-$C_1$-$C_4$-Alkylanilino, N-Formylanilino, N-$C_1$-$C_6$-Alkylcarbonylanilino, Phenylthio oder Halogenphenylthio substituiertes Phenyl oder Naphthyl, oder durch Hydroxy, Fluor, Chlor, Brom, Di-$C_1$-$C_4$-alkylamino, $C_1$-$C_4$-Alkoxy, $C_1$-$C_4$-Halogenalkoxy, $C_2$-$C_6$-Alkoxyalkoxy, $C_1$-$C_4$-Halogenalkylthio, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy, $C_3$-$C_7$-Cycloalkyl, Phenyl, Phenoxy, Phenylthio, Phenylsulfonyloxy oder Pyridyl substituiertes $C_1$-$C_{20}$-Alkyl, wobei die Phenyl- und Pyridylgruppen jeweils durch Fluor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, Phenylthio oder Halogenphenylthio substituiert sein können, steht oder

d) $R_3$ für durch in 4-Stellung durch einen über Sauerstoff gebundenen aromatischen mono- oder bicyclischen Heterocyclus aus der Reihe Pyridin, Pyrimidin oder Benzothioazol substituiertes Phenyl steht, wobei beide aromatischen Ringe unsubstituiert sind oder zusammen höchstens drei weitere Substituenten aus der Reihe Chlor, Brom, Methyl, Aethyl und Trifluormethyl tragen.

Unter den Verbindungen der Untergruppe a) sind diejenigen bevorzugt, worin $R_1$ und $R_2$ für Wasserstoff stehen.

Von den Verbindungen der Untergruppe b) sind solche bevorzugt, worin X Sauerstoff oder -NH- bedeutet.

Eine besonders herauszuhebende Gruppe von Wirkstoffen der Formel I ist dadurch gekennzeichnet, dass $R_1$ und $R_2$ unabhängig voneinander Wasserstoff oder $C_1$-$C_4$-Alkyl, X Sauerstoff oder $-NR_4$- und $R_4$ Wasserstoff, Methyl oder Aethyl bedeuten. Wenn $R_1$ und $R_2$ Wasserstoff bedeuten, werden ganz besonders bevorzugte Verbindungen der Formel charakterisiert.

Aus der Untergruppe c) der Verbindungen der Formel I sind einerseits diejenigen herauszustellen, worin $R_3$ Phenyl, Benzyl, Naphthyl, 3-Pyridylmethyl, oder jeweils durch Fluor, Chlor, Brom, $C_1$-$C_3$-Alkyl, $C_1$-$C_3$-Ha-

logenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, Phenylthio oder Halogenphenylthio substituiertes Phenyl, Benzyl, Naphthyl oder 3-Pyridylmethyl bedeutet. Von diesen Verbindungen sind wiederum solche bevorzugt, worin $R_1$ und $R_2$ unabhängig voneinander Wasserstoff oder $C_1$-$C_4$-Alkyl, X Sauerstoff oder -$NR_4$- und $R_4$ Wasserstoff, Methyl oder Aethyl bedeuten.

Andererseits sind unter der Untergruppe c) der Verbindungen der Formel I auch diejenigen besonders hervorzuheben, worin $R_3$ $C_1$-$C_{12}$-Alkyl oder durch Hydroxy, Fluor, Chlor, Brom, Dimethylamino, Methoxy, Aethoxy, Methoxyäthoxy, Aethoxyäthoxy, Methylthio, Aethylthio, Cyclopropyl, Cyclopentyl, Cyclohexyl, Phenyl oder Phenoxy substituiertes $C_1$-$C_{12}$-Alkyl bedeutet, wobei der Phenyl- oder Phenoxyrest jeweils durch Fluor, Chlor, Brom, Phenoxy, Halogenphenoxy oder Phenylthio substituiert sein kann. Von dieser Gruppe von Verbindungen sind solche bevorzugt, worin $R_1$ und $R_2$ unabhängig voneinander Wasserstoff oder $C_1$-$C_4$-Alkyl, X Sauerstoff oder -$NR_4$- und $R_4$ Wasserstoff oder $C_1$-$C_4$-Alkyl bedeuten. Insbesondere bedeuten $R_1$ und $R_2$ in bevorzugten Ausführungsformen jeweils Wasserstoff.

Eine sehr interessante Gruppe von Wirkstoffen aus der Untergruppe c) der Formel I zeichnet sich dadurch aus, dass $R_3$ $C_3$-$C_{12}$-Alkenyl, $C_3$-$C_{12}$-Alkinyl oder jeweils durch Fluor, Chlor oder Brom substituiertes $C_3$-$C_{12}$-Alkenyl oder $C_3$-$C_{12}$-Alkinyl bedeutet. Aus dieser Gruppe geniessen solche Verbindungen eine Vorzugsstellung, worin $R_1$ und $R_2$ unabhängig voneinander Wasserstoff oder $C_1$-$C_4$-Alkyl, X Sauerstoff oder -$NR_4$- und $R_4$ Wasserstoff oder $C_1$-$C_4$-Alkyl bedeuten. Insbesondere die Bedeutung Wasserstoff ist für $R_1$ und $R_2$ bevorzugt.

Als bevorzugte Einzelverbindungen der Formel I sind zu nennen:

4-Chlor-4,4-difluorbuttersäure-methylester,
4-Chlor-4,4-difluorbuttersäure-äthylester,
4-Chlor-4,4-difluorbuttersäure-isopropylester,
4-Chlor-4,4-difluorbuttersäure-tert.butylester,
4-Chlor-4,4-difluorbuttersäure-n-butylester,
4-chlor-4,4-difluorbuttersäure-(2,2-dimethylpropyl)-ester,
4-Chlor-4,4-difluorbuttersäure-benzylester,
4-Chlor-4,4-difluorbuttersäure-phenylester,
4-Chlor-4,4-difluorbuttersäure-[2-(4-phenoxyphenoxy)-äthyl]-ester,
4-Chlor-4,4-difluorbuttersäure-cyclohexylester,
4-Chlor-4,4-difluorbuttersäure-cyclohexylmethylester,
4-Chlor-4,4-difluorbuttersäure-cyclopropylmethylester,
4-Chlor-4,4-difluor-2-trifluormethylbuttersäure-äthylester,
4-Chlor-4,4-difluorbuttersäure-N-methylamid,
4-Chlor-4,4-difluorbuttersäure-N,N-dimethylamid,
4-Chlor-4,4-difluorbuttersäure-N,N-dihexylamid,
4-Chlor-4,4-difluorbuttersäure-N-äthylamid,
4-Chlor-4,4-difluorbuttersäure-N-isopropylamid,
4-Chlor-4,4-difluorbuttersäure-N-butylamid,
4-Chlor-4,4-difluorbuttersäure-N-tert.butylamid,
4-Chlor-4,4-difluorbuttersäure-N-benzylamid,
4-Chlor-4,4-difluorbuttersäure-anilid,
4-Chlor-4,4-difluorbuttersäure-N-methyl-N-pyrid-3-ylmethylamid,
4-Chlor-4,4-difluorbuttersäure-N-pyrid-3-ylmethylamid,
4-Chlor-4,4-difluorbuttersäure-(4-chlor-anilid),
4-Chlor-4,4-difluorbuttersäure-(4-phenoxy-anilid),
4-Chlor-4,4-difluorbuttersäure-(2-chloranilid),
4-Chlor-4,4-difluorbuttersäure-(4-methoxy-anilid),
4-Chlor-4,4-difluorbuttersäure-(4-methyl-anilid),
4-Chlor-4,4-difluorbuttersäure-(3-methylmercapto-anilid),
4-Chlor-4,4-difluorbuttersäure-(4-fluor-anilid),
4-Chlor-4,4-difluorbuttersäure-(4-chlor-2-nitro-anilid),
4-Chlor-4,4-difluorbuttersäure-(3-phenoxy-benzyl)-ester,
4-Chlor-4,4-difluorbuttersäure-[4-(4-fluor-phenoxy)-phenyl]-ester,
4-Chlor-4,4-difluorbuttersäure-[4-(4-fluorphenoxy)-phenoxy-äthyl]-ester,
4-Chlor-4,4-difluorbuttersäure-(4-nitro-phenyl)-ester,
4-Chlor-4,4-difluorbuttersäure-[4-(3,5-difluor-phenoxy)-phenyl]-ester,
4-Chlor-4,4-difluorbuttersäure-[4-(5-trifluormethyl-pyrid-2-yloxy)-phenyl]-ester,
4-Chlor-4,4-difluorbuttersäure und
4-Chlor-4,4-difluorbuttersäureamid.

Die erfindungsgemässen Verbindungen der Formel I können in Analogie zu bekannten Verfahren hergestellt werden. Beispielsweise erhält man die Verbindung der Formel I, indem man entweder

a) ein 4-Chlor-4,4-difluorbuttersäurehalogenid der Formel II

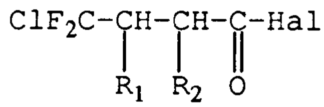

(II)

worin $R_1$ und $R_2$ die unter Formel I gegebenen Bedeutungen haben und Hal für Halogen, vorzugsweise Chlor oder Brom steht, in Gegenwart einer Base mit einer Verbindung der Formel III

$$H\text{-}X\text{-}R_3 \qquad (III)$$

worin X und $R_3$ die unter Formel I gegebenen Bedeutungen haben, umsetzt, oder

b) 4-Chlor-4,4-difluorbuttersäure der Formel Ic

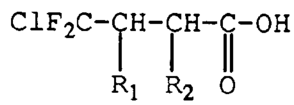

(Ic)

worin $R_1$ und $R_2$ die unter Formel I gegebenen Bedeutungen haben, in Gegenwart eines wasserabspaltenden Mittels mit einer Verbindung der Formel III umsetzt.

Die Umsetzung des Verfahrens a) (II+III → I) erfolgt vorzugsweise in einem inerten, hydroxylgruppenfreien Lösungsmittel in Anwesenheit einer organischen Base, wie zum Beispiel Pyridin, 4-Dimethylaminopyridin, Lutidin, Collidin, Trialkylamin, N,N-Dialkylanilin, oder bicyclische, nicht nucleophile Basen wie 1,4-Diazabicyclo[2.2.2]-octan (DABCO), 1,5-Diazabicyclo[4.3.0]non-5-en (DBN) oder 1,8-Diazabicyclo [5.4.0]undec-7-en (1,5-5) (DBU). Die Reaktion wird im allgemeinen bei Temperaturen von -30° bis +70°C, vorzugsweise von -10° bis +50°C durchgeführt. Man arbeitet dabei zweckmässigerweise in Gegenwart eines reaktionsinerten Lösungsmittels oder Lösungsmittelgemisches. Es eignen sich hierfür beispielsweise aliphatische und aromatische Kohlenwasserstoffe wie Benzol, Toluol, Xylole, Petroläther, Hexan; halogenierte Kohlenwasserstoffe wie Chlorbenzol, Methylenchlorid, Aethylenchlorid, Chloroform, Tetrachlorkohlenwasserstoff, Tetrachloräthylen; Aether und ätherartige Verbindungen wie Dialkyläther (Diäthyläther, Düsopropyläther, tert.-Butylmethyläther usw.), Anisol, Dioxan, Tetrahydrofuran; Nitrile wie Acetonitril, Propionitril; Ester wie Aethylacetat (Essigsäureäthylester), Propylacetat oder Butylacetat; Ketone wie Aceton, Diäthylketon, Methyläthylketon; Verbindungen wie Dimethylsulfoxid (DMSO), Dimethylformamid (DMF) und Gemische solcher Lösungsmittel untereinander. Man kann die Reaktion aber auch im Ueberschuss einer der oben genannten Basen durchführen, oder im Falle, dass die Verbindung der Formel III ein Amin darstellt ($X = NR_4$) kann anstelle der Base auch ein zweites Aequivalent oder auch ein grösserer Ueberschuss der Verbindung der Formel III eingesetzt werden.

Bei der Verfahrensvariante b) (Ic + III → I) wird die Reaktion vorteilhafterweise in Gegenwart von für Veresterungen üblichen, wasserabspaltenden Reagenzien durchgeführt, wie zum Beispiel in Anwesenheit eines Carbodiimids [Dicyclohexylcarbodiimid (DCC)] oder eines 1-Alkyl-2-halogen-pyridiniumsalzes wie 1-Methyl-2-chlorpyridiniumjodid. Man arbeitet dabei zweckmässigerweise in Gegenwart eines reaktionsinerten Lösungsmittels oder Lösungsmittelgemisches bei Temperaturen von -30°C bis +70°C, vorzugsweise -10°C bis +50°C. Man arbeitet bevorzugt in Gegenwart einer Base wie beispielsweise in Gegenwart eines organischen Amins wie eines Trialkylamins (Trimethylamin, Triäthylamin, Tripropylamin oder Diisopropyläthylamin), eines Pyridins (Pyridin selbst, 4-Dimethylaminopyridin oder 4-Pyrrolidinopyridin), eines Morpholins (N-Methylmorpholin) oder eines N,N-Dialkylanilins (N,N-Dimethylanilin oder N-Methyl-N-äthylanilin). Als Lösungsmittel eignen sich hierfür beispielsweise aliphatische und aromatische Kohlenwasserstoffe wie Benzol, Toluol, Xylole, Petroläther, Hexan; halogenierte Kohlenwasserstoffe wie Chlorbenzol, Methylenchlorid, Aethylenchlorid, Chloroform, Tetrachlorkohlenstoff, Tetrachloräthylen; Aether und ätherartige Verbindungen wie Dialkyläther (Diäthyläther, Diisopropyläther), tert.-Butylmethyläther usw.), Anisol, Dioxan, Tetrahydrofuran; Nitrile wie Acetonitril, Propionitril; Ester wie Aethylacetat (Essigsäureäthylester), Propylacetat oder Butylacetat; und Gemische solcher Lösungsmittel untereinander.

Stellt die Verbindung der Formel III einen Alkohol dar (X = O) so kann die Verfahrensvariante b) auch in Gegenwart eines Säurekatalysators, wie zum Beispiel $H_2SO_4$, HCl oder einer Sulfonsäure, wie Methansulfonsäure oder p-Toluolsulfonsäure, durchgeführt werden. Man arbeitet dabei vorteilhafterweise mit einem Ueberschuss des Alkohols der Formel III. Bei diesem Verfahren kann freiwerdendes Wasser kontinuierlich aus dem

Reaktionsgemisch entfernt werden. Eine übliche Methode dazu ist das Entfernen des Reaktionsproduktes Wasser durch Abdestillieren eines Azeotrops des Lösungsmittels mit Wasser. Dazu geeignete Lösungsmittel sind Benzol, Toluol, Xylol, Methylenchlorid oder Chloroform.

Grundsätzlich sind die verschiedenen Derivate der Formel I auch aus Umesterung oder Amidierung aus den leicht zugänglichen Niederalkylestern der 4-Chlor-4,4-difluorbuttersäure erhältlich.

Beispielsweise können die Derivate des Estertyps der Formel I (X = O) durch basen- oder säurekatalysierte Umesterung der Niederalkylester der Formel Ia

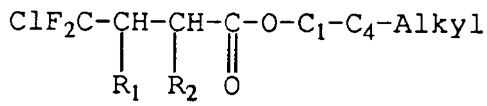 (Ia)

mit den Alkoholen der Formel IIIa

$$H\text{-}O\text{-}R_3 \qquad \text{(IIIa)}$$

worin $R_3$ mit Ausnahme von $C_1$-$C_4$-Alkyl die unter Formel I gegebene Bedeutung hat, erhalten werden. Als Säurekatalysatoren besonders geeignet sind HCl, $H_2SO_4$ oder eine Sulfonsäure. Bei der basenkatalysierten Umesterung verwendet man vorzugsweise als Base das Natrium- oder Kaliumalkoholat des Alkohols der Formel IIIa welche aus IIIa beispielsweise durch Zugabe von Natrium- oder Kaliumhydrid zugänglich sind. Die Umesterungsreaktion wird vorzugsweise bei Temperaturen zwischen -20°C und +120°C, insbesondere zwischen 0°C und +100°C durchgeführt. Die Alkoholkomponente IIIa wird mit Vorteil im Ueberschuss verwendet. Als Lösungsmittel kommen Aether, wie Diäthyläther, Diisopropyläther, Dioxan oder Tetrahydrofuran, halogenierte Kohlenwasserstoffe oder aliphatische oder aromatische Kohlenwasserstoffe in Frage.

Derivate des Amidtyps der Formel I (X = $NR_4$) werden aus den Niederalkylestern der Formel Ia erhalten, indem man diese mit Aminen der Formel IIIb

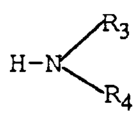 (IIIb)

worin $R_3$ und $R_4$ die unter Formel I gegebenen Bedeutungen haben, umsetzt. Die Amidierungsreaktionen führt man zwischen Temperaturen zwischen 0°C und +120°C durch. Mit Vorteil werden die Reaktanden in einem inerten Lösungsmittel oder Lösungsmittelgemisch umgesetzt. Es eignen sich hierfür beispielsweise aliphatische und aromatische Kohlenwasserstoffe wie Benzol, Toluol, Xylole, Petroläther, Hexan; halogenierte Kohlenwasserstoffe wie Chlorbenzol, Methylenchlorid, Aethylenchlorid, Chloroform, Tetrachlorkohlenstoff, Tetrachloräthylen; Aether und ätherartige Verbindungen wie Dialkyläther (Diäthyläther, Diisopropyläther, tert.-Butylmethyläther usw.), Anisol, Dioxan, Tetrahydrofuran; Nitrile wie Acetonitril, Propionitril; Alkohole wie Methanol, Aethanol, Propanol, Isopropanol; oder Wasser. Die Aminkomponente IIIb wird mit Vorteil im Ueberschuss verwendet.

Die Verbindungen der Formel III sind bekannt und teilweise im Handel erhältlich oder sie können analog zu bekannten Herstellungsverfahren hergestellt werden.

Die Säurehalogenide der Formel II können auf übliche Weise durch Umsetzung mit Halogenierungsmitteln aus den 4-Chlor-4,4-difluorbuttersäuren der Formel Ic erhalten werden. Als Halogenierungsmittel eignen sich besonders $SOCl_2$, Oxalylchlorid, $PCl_3$, $POCl_3$ oder $PCl_5$. Man arbeitet dabei im allgemeinen bei Temperaturen zwischen -20°C und +120°C, vorzugsweise zwischen 0°C und +100°C. Die Umsetzung kann ohne Lösungsmittel oder im Gemisch mit einem reaktionsinerten Lösungsmittel erfolgen. Als Lösungsmittel eignen sich hierfür beispielsweise aromatische Kohlenwasserstoff wie Benzol oder Toluol oder halogenierte Kohlenwasserstoffe wie Methylenchlorid, Chloroform oder Chlorbenzol. Die Reaktion wird häufig unter Zugabe einer katalytischen Menge von DMF durchgeführt.

Die Zwischenprodukte der Formel II sind neu. Sie wurden speziell für die Synthese der Wirkstoffe der Formel I entwickelt. Sie bilden daher einen Gegenstand der vorliegenden Erfindung.

Die 4-Chlor-4,4-difluorbuttersäuren der Formel Ic lassen sich aus Verbindungen der Formel Ia durch saure oder basische Hydrolyse erhalten. Die Reaktion wird im allgemeinen bei Temperaturen zwischen -20°C und +120°C, vorzugsweise zwischen +10°C und +100°C durchgeführt. Bei der sauren Hydrolyse verwendet man vorzugsweise HCl oder $H_2SO_4$ und bei der basischen Hydrolyse NaOH oder KOH. Man arbeitet dabei zweckmässigerweise in Wasser oder in einem Lösungsmittelgemisch aus Wasser und einem organischen Lösungsmittel. Als organische Lösungsmittel besonders geeignet sind Alkohole, wie Methanol oder Aethanol; Aether

wie Dioxan oder Tetrahydrofuran; Dimethylsulfoxid; oder Dimethylformamid.

Die Verbindungen der Formel Ia, die eine Untergruppe der Verbindungen der Formel I darstellen und gleichzeitig als Zwischenprodukte für die Herstellung der diversen Ester- und Amidtypen der Formel I dienen können, sind nach dem folgenden Verfahren erhältlich:

Die Verbindungen der Formel Ia können erhalten werden, indem man einen α-Halogen-4-chlor-4,4-difluorbuttersäureester der Formel IV

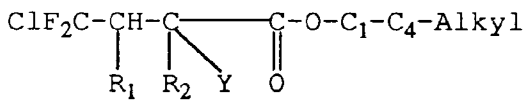 (IV)

worin $R_1$ und $R_2$ die unter Formel I gegebenen Bedeutungen haben und Y für Chlor oder Brom steht, mit Wasserstoff in der α-Position katalytisch dehalogeniert.

Die Verbindungen der engeren Unterformel Ib

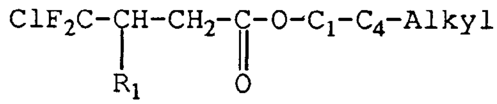 (Ib)

worin $R_1$ die unter Formel I gegebene Bedeutung hat, können durch katalytische α-Dehalogenierung mit Wasserstoff aus den α,α-Dihalogen-4-chlor-4,4-difluorbuttersäureestern der Formel V

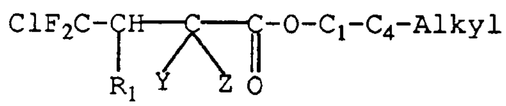 (V)

worin $R_1$ die unter Formel I gegebene Bedeutung hat und Y und Z unabhängig voneinander Chlor oder Brom bedeuten, erhalten werden.

Bei geeigneter Wahl des Katalysators und der Reaktionsbedingungen ist es auch möglich, die α-Halogenatome Y und Z stufenweise durch Wasserstoff zu ersetzen. Es können so durch mono-α-Dehalogenierung die Verbindungen der Formel IV, worin $R_2$ für Wasserstoff steht, aus den Verbindungen der Formel V hergestellt werden.

Die katalytische Dehalogenierungsverfahren mit Hilfe von Wasserstoff (IV → Ia, V → Ib und V → IV) wird mit Wasserstoff in Gegenwart eines Edelmetallkatalysators oder RaneyNickel, gegebenenfalls in Gegenwart eines Halogenwasserstofffängers und eines Lösungsmittels bei einer Temperatur zwischen 0°C und +150°C und bei Normaldruck oder einem Druck bis zu 150 bar durchgeführt. Das Lösungsmittel wird bevorzugt aus der Gruppe der Kohlenwasserstoffe, Halogenkohlenwasserstoffe, Aether, Ketone, Alkohole, Carbonsäureester, Sulfone, N,N-Dialkylcarbonsäureamide, N-Alkyllactame und Lactone ausgewählt. Einige Beispiele sind Petroläther, Pentan, Hexan, Cyclohexan, Methylcyclohexan, Benzol, Toluol, Xylol, Chlorbenzol, Methylenchlorid, Chloroform, 1,2-Dichloräthan, 1,1,2,2-Tetrachloräthan, Diäthyläther, Dibutyläther, Aethylenglykoldimethyläther, Tetrahydrofuran, Dioxan, Aceton, Methylisobutylketon, Essigsäuremethyl-oder -äthylester, Tetramethylensulfon, Dimethylformamid, N-Methylpyrrolidon, γ-Valerolacton, Butyrolacton, Methanol, Aethanol oder Isopropanol. Vorzugsweise ist das Lösungsmittel polarer Natur. Besonders bevorzugte Lösungsmittel sind Essigsäureäthylester, Tetrahydrofuran oder Alkohole. Die Menge des Lösungsmittels kann in weiten Grenzen gewählt werden. Zweckmässig wird die gleiche bis zehnfache Menge Lösungsmittel bezogen auf die Menge der Verbindung der Formel IV oder V verwendet. Halogenwasserstoffänger sind allgemein bekannt. Es kann sich dabei um tertiäre Stickstoffbasen mit bevorzugt insgesamt 3 bis 20, besonders 3 bis 12 Kohlenstoffatome handeln, um Alkalimetall- oder Erdalkalimetallsalze organischer Säuren oder der Kohlensäure, oder um Alkalimetall- oder Erdalkalimetalloxide oder -hydroxide. Einige Beispiele sind Natriumcarbonat, Natrium- oder Calciumhydrogencarbonat, Natriumacetat, NaOH, KOH, MgO und CaO; oder aromatische, aliphatische oder cyclische tertiäre Stickstoffbasen wie Trimethyl-, Triäthyl-, Tripropyl-, Tributyl-, Triäthanol oder Butyldimethylamin, Pyridin, 2,6-Dimethylpyridin, N-Methylpyrrolin und N-Methylmorpholin. Bevorzugte Halogenwasserstoffänger sind tertiäre Stickstoffbasen und MgO. Besonders bevorzugt ist 2,6-Dimethylpyridin. Der Halogenwasserstoffänger kann in geringem Unterschuss oder Ueberschuss, bezogen auf die Menge der Verbindung der Formel IV oder V, eingesetzt werden. Bevorzugt werden äquimolare Mengen verwendet. Geeignete Edelmetallkatalysatoren sind beispielsweise Iridium, Rhodium, Platin, Ruthenium und Palladium. Besonders bevorzugt wird Palladium verwendet. Das Edelmetall wird bevorzugt als Katalysator auf einem Träger eingesetzt. Beispiele für Träger sind $BaSO_4$, $SiO_2$, $Al_2O_3$ und insbesondere Aktivkohle. Der angewendete Katalysator enthält neben dem Träger im allgemeinen 0,1 bis 20 Gew.-% des Edelmetalls. Es können auch sulfidierte Katalysatoren verwendet werden. Die eingesetzte Menge kann 0,1 bis 20 Gew.-% betragen, bezogen auf die Menge der Verbindungen der Formel IV oder V. Es kann zweckmässig sein, während der Reaktion regenerierten oder ungebrauchten Katalysator zuzugeben. Die Reaktionstemperatur beträgt bevorzugt zwischen 0°C und +80°C, besonders 0°C bis +30°C. Der Druck beträgt bevorzugt bis zu 20 bar. Bevorzugt wird die Reaktion bei Normaldruck durchgeführt.

Die Verbindungen der Formel V sind aus der EP-A-2206 bekannt oder können analog zum dort beschriebenen Verfahren erhalten werden. Die Zwischenprodukte der Formel IV können hergestellt werden, indem man an einen Chloressigsäureester der Formel VI

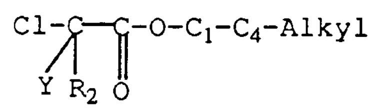

(VI)

worin $R_2$ die unter Formel I gegebene Bedeutung hat und Y für Chlor oder Brom steht, in Gegenwart eines Cu-(I)-Katalysators 1,1-Difluoräthylen der Formel VII

$$F_2C=CH_2 \qquad (VII)$$

addiert. Dieses Verfahren ist für Y = Chlor in Helv. Chim. Acta 63, p. 1947-1957 (1980) beschrieben.

Die einsetzbaren Katalysatoren enthalten als wesentliches Element Kupfer in der Oxidationsstufe 1. Beispiele dafür sind Kupfer(I)chlorid und -bromid (CuCl, CuBr), Kupfercyanid (CuCN). Bevorzugt sind CuCl und CuBr sowie deren Gemische. Die Katalysatoren werden im allgemeinen in Mengen von etwa 0,01 bis 10 Mol%, bevorzugt 0,1 bis 5 Mol%, bezogen auf die Verbindung der Formel VII, verwendet. Die Umsetzung wird in einem organischen Lösungsmittel vorgenommen. Geeignete organische Lösungsmittel sind solche, in denen die Katalysatoren ausreichend löslich sind oder die mit den Katalysatoren Komplexe bilden können, die aber gegenüber den Ausgangsverbindungen der Formel VI und VII inert sind. Beispiele solcher Lösungsmittel sind Alkylnitrile, insbesondere solche mit 2 bis 5 Kohlenstoffatomen, wie Acetonitril, Propionitril und Butyronitril; 3-Alkoxypropionitrile mit 1 oder 2 Kohlenstoffatomen im Alkoxyteil, wie 3-Methoxypropionitril und 3-Aethoxypropionitril; aromatische Nitrile, vor allem Benzonitril; aliphatische Ketone mit bevorzugt insgesamt 3 bis 8 Kohlenstoffatomen, wie Aceton, Diäthylketon, Methyl-isopropylketon, Di-isopropylketon, Methyl-tert-butylketon; Alkyl- und Alkoxyalkylester von aliphatischen Monocarbonsäuren mit insgesamt 2 bis 6 Kohlenstoffatomen, wie Ameisensäuremethyl- und -äthylester, Essigsäuremethyl-, -äthyl-, -n-butyl- und -isobutylester sowie 1 -Acetoxy-2-methoxyäthan; cyclische Aether, wie Tetrahydrofuran, Tetrahydropyran und Dioxan; Dialkyläther mit je 1 bis 4 Kohlenstoffatomen in den Alkylteilen, wie Diäthyläther, Di-n-propyläther und Di-isopropyläther; N,N-Dialkylamide von aliphatischen Monocarbonsäuren mit 1 bis 3 Kohlenstoffatomen im Säureteil, wie N,N-Dimethylformamid, N,N-Dimethylacetamid, N,N-Diäthylacetamid und N,N-Dimethylmethoxy-acetamid; Aethylenglykol- und Diäthylenglykoldialkyläther mit je 1 bis 4 Kohlenstoffatomen in den Alkylteilen, wie Aethylenglykoldimethyl-, -diäthyl- und -di-n-butyläther, Diäthylenglykoldiäthyl- und -di-n-butyläther oder Hexamethylphosphorsäuretriamid. Bevorzugte Lösungsmittel sind Alkylnitrile mit 2 bis 6 Kohlenstoffatomen und 3-Alkoxypropionitrile mit 1 oder 2 Kohlenstoffatomen im Alkoxyteil, insbesondere Acetonitril und 3-Methoxypropionitril. Die Reaktionstemperatur ist im allgemeinen nicht kritisch und kann innerhalb weiter Grenzen variieren. Bevorzugt liegen die Reaktionstemperaturen zwischen etwa +60°C und +200°C, insbesondere zwischen etwa +80°C und +170°C. Die Reaktion kann unter Druck oder drucklos durchgeführt werden.

Die Verbindungen der engeren Formel Id

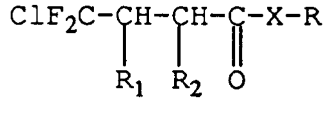

(Id)

worin $R_1$, $R_2$ und X die unter Formel I gegebenen Bedeutungen haben und R für Wasserstoff oder $C_1$-$C_6$-Alkyl steht, können durch katalytische Hydrierung mit Wasserstoff aus den 4-Chlor-4,4-difluorcrotonsäurederivaten der Formel VIII

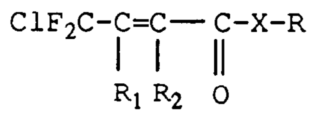

(VIII)

worin $R_1$, $R_2$ und X die unter Formel I gegebenen Bedeutungen haben und R für Wasserstoff oder $C_1$-$C_6$-Alkyl steht, erhalten werden.

Die so erhaltenen Produkte der engeren Unterformeln Ia, Ib, Ic und Id können durch übliche Derivatisierungsreaktionen wie Hydrolyse, Umesterung oder Umamidierung in die übrigen Derivate der Formel I überführt werden.

Die katalytische Hydrierung der Crotonsäuren der Formel VIII wird unter für diesen Reaktionstyp üblichen Bedingungen ausgeführt. So führt man die Reaktion in Gegenwart eines Edelmetallkatalysators oder von Raney-Nickel, vorzugsweise in einem inerten Lösungsmittel, unter einer Wasserstoffatomsphäre bei einem Druck zwischen 1 und 150 bar durch.

Geeignete Edelmetallkatallysatoren sind beispielsweise Iridium, Rhodium, Platin, Ruthenium und Palladium. Besonders bevorzugt wird Palladium verwendet. Das Edelmetall wird bevorzugt als Katalysator auf einem Träger eingesetzt. Beispiele für Träger sind $BaSO_4$, $SiO_2$, $Al_2O_3$ und insbesondere Aktivkohle. Der angewendete Katalysator enthält neben dem Träger im allgemeinen 0,1 bis 20 Gew.-% des Edelmetalls. Es können auch sulfidierte Katalysatoren verwendet werden. Die eingesetzte Menge kann 0,1 bis 20 Gew.-% betragen, bezogen auf die Menge der Verbindungen der Formel VIII. Es kann zweckmässig sein, während der Reaktion regenerierten oder ungebrauchten Katalysator zuzugeben. Die Reaktionstemperatur beträgt bevorzugt zwischen 0°C und +80°C, besonders 0°C bis +30°C. Der Druck beträgt bevorzugt bis zu 20 bar. Insbesondere wird die Reaktion vorzugsweise bei Normaldruck durchgeführt. Das Lösungsmittel wird bevorzugt aus der Gruppe der Kohlenwasserstoffe, Halogenkohlenwasserstoffe, Aether, Ketone, Carbonsäureester, Sulfone, N,N-Dialkylcarbonsäureamide, N-Alkyllactame und Lactone ausgewählt. Einige Beispiele sind Petroläther, Pentan, Hexan, Cyclohexan, Methylcyclohexan, Benzol, Toluol, Xylol, Chlorbenzol, Methylenchlorid, Chloroform, 1,2-Dichloräthan, 1,1,2,2-Tetrachloräthan, Diäthyläther, Dibutyläther, Aethylenglykoldimethyläther, Tetrahydrofuran, Dioxan, Aceton, Methylisobutylketon, Essigsäuremethyl- oder -äthylester, Tetramethylensulfon, Dimethylformamid, N-Methylpyrrolidon, γ-Valerolacton, Butyrolacton, Methanol. Vorzugsweise ist das Lösungsmittel polarer Natur. Besonders bevorzugte Lösungsmittel sind Essigsäureäthylester und Tetrahydrofuran. Die Menge des Lösungsmittels kann in weiten Grenzen gewählt werden. Zweckmässig wird die gleiche bis zehnfache Menge Lösungsmittel bezogen auf die Menge der Verbindung der Formel VIII verwendet.

Die Verbindungen der Formel VIII sind mit Ausnahme von 4-Chlor-4,4-difluorcrotonsäure und mit Ausnahme derjenigen Verbindungen VIII, woring X Sauerstoff ist, R Äthyl ist, $R_2$ Wasserstoff ist und $R_1$ $CF_2H$, $CF_2Cl$ oder $CF_3$ ist, neu. Die neuen Verbindungen wurden speziell für die Synthese der Verbindungen der Formel I als Zwischenprodukte entwickelt. Sie bilden einen Teil der vorliegenden Erfindung. Die neuen Verbindungen der Formel VIII können analog zum beschriebenen Herstellungsverfahren für die freie Säure erhalten werden (Izvestiya Akademii Nauk SSSR, Ser. Khim, No. 2 (1965), 300-307) oder aus diesen Produkten durch übliche Derivatisierungsreaktionen wie Veresterung, Umesterung oder Amidierung hergestellt werden.

Verbindungen der Formel VIII, worin $R_1$ und $R_2$ Wasserstoff bedeuten (Verbindungen VIII a), können auch aus Verbindungen der Formel IX,

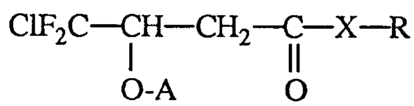

(IX)

worin A Wasserstoff oder eine Acylgruppe bedeutet, durch β-Elimination nach bekannten Methoden erhalten werden, z.B. Houben Weyl 6/1b 939 (1984).

Verbindungen der Formel IX können aus Verbindungen der Formel X

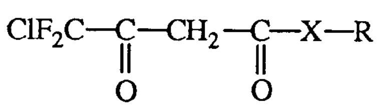

(X)

durch Reduktion, z.B. katalytisch mit Wasserstoff nach der von Reuben G. Jones in J. Amer. Chem. Soc., 70 (1948) 144 beschriebenen Methode, hergestellt werden.

Die Verbindungen der Formel X sind teilweise bekannt oder können nach bekannten Methoden wie z.B. Claisen Kondensation (Huang Weiynan et al.; Huaxne Xuebao 1983, 41(8) 723; C.A. 100, (1984) 22308s) hergestellt werden.

Weiter erhält man Verbindungen der Formel X, indem man Chlordifluoracetylchlorid $ClF_2C$-CO-Cl mit Keten $H_2C=C=O$ umsetzt und das erhaltene 4-Chlor-4,4-difluoracetessigsäurechlorid der Formel XI

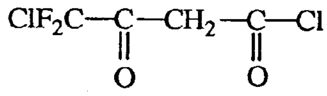

(XI)

zur freien Säure hydrolysiert und durch Reaktion mit den geeigneten Alkoholen oder Aminen in die Ester oder Amide der Formel X überführt. Mit Vorteil kann man diese Ester und Amide auch direkt durch Umsetzung des Säurechlorids der Formel XI mit einem Alkohol oder Amin erhalten.

Beispielsweise ist für Verbindungen der Formel I, worin $R_1$ und $R_2$ Wasserstoff, X Sauerstoff und $R_3$ für Alkyl steht, eine Synthese nach folgendem Schema 1 als vorteilhaft anzusehen:

Schema 1:

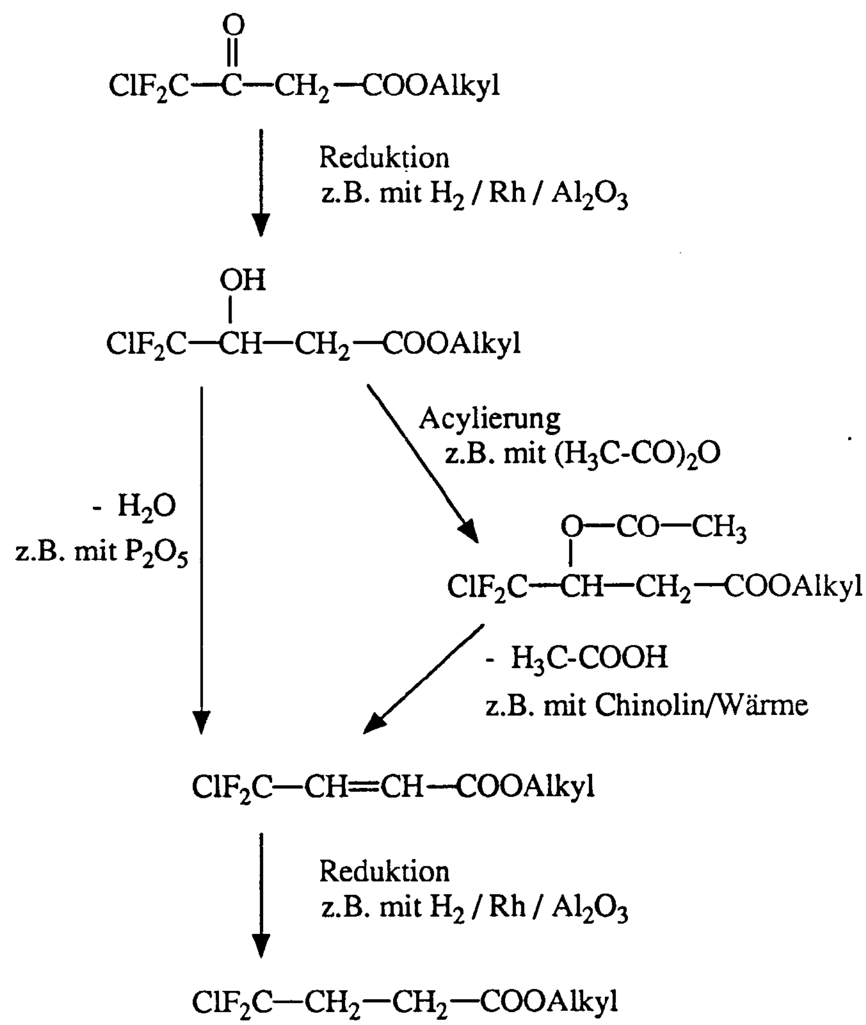


Es wurde nun gefunden, dass die erfindungsgemässen Verbindungen der Formel I bei günstiger Warmblüter-, Fisch- und Pflanzenverträglichkeit wertvolle Wirkstoffe in der Schädlingsbekärnpfung sind. Insbesondere betrifft die Anwendung der erfindungsgemässen Wirkstoffe Insekten und Spinnentiere, die in Nutz- und

Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen. Sie sind gegen alle oder einzelne Entwicklungsstadien von normal sensiblen aber auch resistenten Arten wirksam. Dabei kann sich ihre Wirkung in unmittelbarer Abtötung der Schädlinge oder erst nach einiger Zeit, beispielsweise bei einer Häutung, oder in einer verminderten Eiablage und/oder Schlupfrate zeigen. Zu den oben erwähnten Schädlingen gehören:
aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp. Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.; aus der Ordnung der Orthoptera zum Beispiel Blatta spp., Blattella spp., Gryllotalpa spp., Leucophaea maderae, Löcusta spp., Periplaneta spp. und Schistocerca spp.;
aus der Ordnung der Isoptera zum Beispiel
Reticulitermes spp.; aus der Ordnung der Psocoptera zum Beispiel Liposcelis spp.; aus der Ordnung der Anoplura zum Beispiel Haematopinus spp., Linognathus spp. Pediculus spp., Pemphigus spp. und Phylloxe ra spp.; aus der Ordnung der Mallophaga zum Beispiel Damalinea spp. und Trichodectes spp.;
aus der Ordnung der Thysanoptera zum Beispiel
Frankliniella spp., Hercinothrips spp., Taeniothrips spp., Thrips palmi, Thrips tabaci und Scirtothrips aurantii;
aus der Ordnung der Heteroptera zum Beispiel
Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung der Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., leecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung der Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung der Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp.) Orseolia spp. Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung der Siphonaptera z.B.
Ceratophyllus spp., Xenopsylla cheopis,
aus der Ordnung der Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp.,

Sarcoptes spp., Tarsonemus spp. und Tetranychus spp.; und
aus der Ordnung der Thysanura zum Beispiel
Lepisma saccharina.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen und der sie enthaltenden Mittel lässt sich durch Zusatz von anderen Insektiziden und/oder Akariziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnlk üblichen Hilfsmitteln eingesetzt und können daher beispielsweise zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, das heisst die den Wirkstoff der Formel I, beziehungsweise Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckmitteln, wie beispielsweise mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, beispielsweise das Stearyltrimethylammoniumchlorid oder das Benzyl-di-(2-chloräthyl)-äthylammoniumbromid.

Die in der Formulierungstechnik gebräuchlichen Tenside sind beispielsweise in folgenden Publikationen beschrieben:
"1985 International Mc Cutcheon's Emulsifiers & Detergents", Glen Rock NJ USA, 1985",
H. Stache, "Tensid-Taschenbuch", 2. Aufl., C. Hanser Verlag München, Wien 1981,
M. and J. Ash. "Encyclopedia of Surfactants", Vol. I-III, Chemical Publishing Co., New York, 1980-1981.

Die pestiziden Zubereitungen enthalten in der Regel 0,1 bis 99 %, insbesondere 0,1 bis 95 % Wirkstoff der Formel I oder Kombinationen dieses Wirkstoffs mit andern Insektiziden oder Akariziden, 1 bis 99,9 eines festen oder flüssigen Zusatzstoffes und 0 bis 25 insbesondere 0,1 bis 20 %, eines Tensides. Während als Handelsware eher konzentrierte Mittel bevorzugt werden, verwendet der Endverbraucher in der Regel verdünnte Zübereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000g Wirkstoff pro Hektar, vorzugsweise 25 bis 500 g/ha.

Insbesondere setzen sich bevorzugte Formulierungen folgendermassen zusammen: (% = Gewichtsprozent)

Emulgierbare Konzentrate:

| | |
|---|---|
| Aktiver Wirkstoff: | 1 bis 20 %, bevorzugt 5 bis 10 % |
| oberflächenaktives Mittel: | 5 bis 30 %, vorzugsweise 10 bis 20 % |
| flüssiges Trägermittel: | 50 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspension-Konzentrate:

| | |
|---|---|
| Aktiver Wirkstoff: | 5 bis 75 % vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30% |

Benetzbare Pulver:

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

Granulate:

| | |
|---|---|
| Aktiver Wirkstoff: | 0,5 bis 30 %, vorzugsweise 3 bis 15 % |

festes Trägermittel: 99,5 bis 70 %, vorzugsweise 97 bis 85%

Die Mittel können auch weitere Zusätze wie Stabilisatoren, Entschäumer, Konservierungmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

Herstellungsbeispiele

Beispiel H1: 4-Chlor-4,4-difluor-2-trifluormethylbuttersäureäthylester

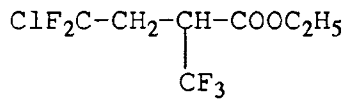

a) 2,4-Dichlor-4,4-difluor-2-trifluormethylbuttersäure-äthylester.

22,5 g 2,2-Dichlor-3,3,3-trifluor-propionsäureäthylester werden mit 0,5 g Cu(I)-chlorid und 100 ml Acetonitril in einem Autoklaven vorgelegt. Nach dem Aufpressen von 16,0 g 1,1-Difluoräthylen wird die Mischung für 8 Stunden auf +160°C erhitzt. Nach dem Erkalten wird das Reaktionsgemisch vom Lösungsmittel befreit und das zurückbleibende Oel am Vakuum rektifiziert. Man erhält 7,4 g 2,4-Dichlor4,4-difluor-2-trifluormethylbuttersäure-äthylester in Form eines farblosen bei 54-56°C/11 mbar siedenden Oels.

Analyse: $C_7H_7Cl_2F_5O_2$ (289,03)
Ber.: C 29,09 % H 2,44 % Cl 24,53 % F 32,87 %
Gef.: C 29,2 % H 2,4 % Cl 24,4 % F 32,9 %

b) 28,9 g 2,4-Dichlor-4,4-difluor-2-trifluormethyl-buttersäure-äthylester werden in 200 ml Aethanol in Gegenwart von 2 g 5%-igem Platin/Kohle-Katalysator bei Normaldruck und Raumtemperatur mit Wasserstoffgas, bis zur Aufnahme von 1 Aequivalent Wasserstoff behandelt. Nach dem Abfiltrieren des Katalysators wird das Lösungsmittel bei Normaldruck abdestilliert und der Rückstand rektifiziert. Man erhält 15 g 4-Chlor-4,4-difluor-2-trifluormethylbuttersäure-äthylester in Form eines farblosen Oels vom Siedepunkt 41-43°C/16 mbar.

Analyse: $C_7H_8ClF_5O_2$ (254,58)
Ber.: C 33,03 % H 3,17 % Cl 13,92 % F 37,31 %
Gef.: C 33,2 % H 3,1 % Cl 14,1 % F 37,0 %

Beispiel H2: 4-Chlor-4,4-difluorbuttersäure-äthylester

$$ClF_2C-CH_2-CH_2-COOC_2H_5$$

22,1 g 2,4-Dichlor-4,4-difluorbuttersäure-äthylester werden in 200 ml absolutem Aethanol gelöst. Nach der Zugabe von 8,2 g wasserfreiem Natriumacetat und 2,0 g 5%-igem Platin/Kohle-Katalysator wird bei Normaldruck Wasserstoffgas eingeleitet, bis die Wasserstoffaufnahme 100 % der Theorie beträgt. Nach dem Abfiltrieren des Katalysators und dem Abdestillieren des Aethanols wird das zurückbleibende Oel auf Wasser aufgegossen, die organische Phase abgetrennt, mit Natriumsulfat getrocknet, filtriert und bei Normaldruck rektifiziert. Man erhält 14,0 g 4-Chlor-4,4-difluorbuttersäure-äthylester in Form eines farblosen Oels mit dem Siedepunkt 154-156°C.

Analyse: $C_6H_9ClF_2O_2$ (186,59)
Ber.: C 38,62 % H 4,86 % Cl 19,00 % F 20,36 %
Gef.: C 38,6 % H 4,8 % Cl 19,1 % F 19,9 %

Beispiel H3: 4-Chlor-4,4-difluorbuttersäure-äthylester

$$ClF_2C\text{-}CH_2\text{-}CH_2\text{-}COOC_2H_5$$

22,1 g 2,4-Dichlor-4,4-difluorbuttersäure-äthylester werden in 100 ml absolutem Tetrahydrofuran gelöst. Nach der Zugabe von 10,7 g 2,6-Dimethylpyridin und 2,0 g 5%-igem Palladium/Kohle-Katalysator wird bei Normaldruck Wasserstoffgas eingeleitet, bis die Wasserstoffaufnahme 100 % der Theorie beträgt. Nach dem Abfiltrieren des Katalysators und dem Abdestillieren des Tetrahydrofurans wird das zurückbleibende Oel auf Wasser aufgegossen, die organische Phase abgetrennt, mit Natriumsulfat getrocknet, filtriert und bei Normaldruck rektifiziert. Man erhält 14,0g 4-Chlor-4,4-difluorbuttersäure-äthylester in Form eines farblosen Oels mit dem Siedepunkt 154-156°C.

Analyse: $C_6H_9ClF_2O_2$ (186,59)
Ber.: C 38,62 % H 4,86 % Cl 19,00 % F 20,36 %
Gef.: C 38,6 % H 4,8 % Cl 19,1 % F 19,9 %

Beispiel H4: 4-Chlor-4,4-difluorbuttersäure-äthylester

$$ClF_2C\text{-}CH_2\text{-}CH_2\text{-}COOC_2H_5$$

25,5 g 2,2,4-Trichlor-4,4-difluorbuttersäure-äthylester werden in 200 ml absolutem Aethanol gelöst. Nach der Zugabe von 16,4 g wasserfreiem Natriumacetat und 2,0 g 5%-igem Platin/Kohle-Katalysator wird bei Normaldruck Wasserstoffgas eingeleitet, bis die Wasserstoffaufnahme 2 Aequivalente beträgt. Nach dem Abfiltrieren des Katalysators und dem Abdestillieren des Aethanols wird das zurückbleibende Oel auf Wasser aufgegossen, die organische Phase abgetrennt, mit Natriumsulfat getrocknet, filtriert und bei Normaldruck rektifiziert. Man erhält 14,0 g 4-Chlor-4,4-difluorbuttersäure-äthylester in Form eines farblosen Oels mit dem Siedepunkt 154-156°C.

Analyse: $C_6H_9ClF_2O_2$ (186,59)
Ber.: C 38,62 % H 4,86 % Cl 19,00 % F 20,36 %
Gef.: C 38,6 % H 4,8 % Cl 19,1 % F 19,9 %

Beispiel H5: 4-Chlor-4,4-difluorbuttersäure-N-methylamid

$$ClF_2C\text{-}CH_2\text{-}CH_2\text{-}CO\text{-}NH\text{-}CH_3$$

2,0 g 4-Chlor-4,4-difluorbuttersäure-äthylester werden bei 0°C in 10 ml einer 33%-igen Lösung von Methylamin in Aethanol gelöst. Das erhaltene Reaktionsgemisch wird 3 Tage bei Raumtemperatur gerührt und dann eingedampft. Der erhaltene Rückstand wird aus Hexan umkristallisiert. Man erhält 1,5 g 4-Chlor-4,4-difluorbuttersäure-N-methylamid mit einem Schmelzpunkt von 61-63°C.

Beispiel H6: 4-Chlor-4,4-difluorbuttersäure-benzylester

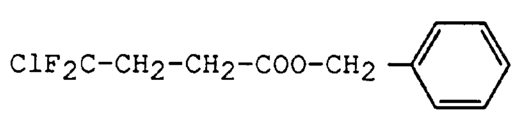

a) 4-Chlor-4,4-difluorbuttersäure.

18,6 g 4-Chlor-4,4-difluorbuttersäure-äthylester werden mit 100 ml 2N-NaOH bei Raumtemperatur gerührt bis eine homogene Lösung entstanden ist. Die Lösung wird hierauf auf 150 ml 2N-HCl aufgegossen. Die ausgeschiedene organische Phase wird in Diäthyläther aufgenommen, mit Natriumsulfat getrocknet und nach dem Abdestillieren des Aethers im Vakuum rektifiziert. Man erhält 12,6 g 4-Chlor-4,4-difluorbuttersäure in Form eines farblosen Oels vom Siedepunkt 88-90°C/11 mbar.

Analyse: $C_4H_5ClF_2O_2$ (158,53)
Ber.: C 30,31 % H 3,18 % Cl 22,36 % F 23,97 %
Gef.: C 30,3 % H 3,2 % Cl 22,4 % F 24,2 %

b) 4-Chlor-4,4-difluorbuttersäurechlorid.

15,8 g 4-Chlor-4,4-difluorbuttersäure werden mit 50 ml Thionylchlorid und 0,2 ml Dimethylformamid gemischt und innerhalb von 2 Stunden auf +70°C erwärmt und darauf noch 30 Minuten bei +70°C gehalten. Das Reaktionsgemisch wird im Vakuum rektifiziert und die beim Siedepunkt von 35-37°C/21 mbar übergehende Flüssigkeit aufgefangen. Man erhält 10,5 g 4-Chlor-4,4-difluorbuttersäurechlorid in Form einer klaren, farblosen Flüssigkeit.

Analyse: $C_4H_4Cl_2F_2O$ (176,98)
Ber.: C 27,15 % H 2,28 % Cl 40,06 % F 21,47 %
Gef.: C 27,3 % H 2,3 % Cl 40,1 % F 21,4 %

c) Zu einer Lösung von 3,35 g 4-Chlor-4,4-difluorbuttersäurechlorid in 15 ml Benzol wird bei 0°C innerhalb von 30 Minuten eine Lösung von 1,84 g Benzylalkohol und 3,74 g Pyridin in 6 ml Methylenchlorid gegeben. Das erhaltene Reaktionsgemisch wird für 16 Stunden bei 0°C gerührt, dann auf 50 ml 1N HCl-Lösung gegossen und mit 150 ml Diäthyläther extrahiert. Die organische Phase wird mit 50 ml gesättigter $NaHCO_3$-Lösung und 50 ml gesättigter NaCl-Lösung gewaschen, mit Magnesiumsulfat getrocknet und eingedampft. Das erhaltene Rohprodukt wird im Kugelrohr bei 190-210°C/120 mbar destilliert. Man erhält 3,65 g 4-Chlor-4,4-difluorbuttersäurebenzylester.

MS: m/e: 248 ($M^+$, $C_{11}H_{11}ClF_2O_2$)

Beispiel H7: 4-Chlor-4,4-difluorbuttersäure-N-isopropylamid

$ClF_2C\text{-}CH_2\text{-}CH_2\text{-}CO\text{-}NH\text{-}C_3H_7\text{-}i$

Zu einer Lösung von 3,35 g 4-Chlor-4,4-difluorbuttersäurechlorid in 15 ml Toluol wird bei 0°C eine Lösung von 3,35 g Isopropylamin in 5 ml Methylenchlorid gegeben. Das erhaltene Reaktionsgemisch wird für 16 Stunden bei 0°C gerührt, dann auf 50 ml gesättigte $NaHCO_3$-Lösung gegossen und mit 150 ml Diäthyläther extrahiert. Die organische Phase wird mit 50 ml gesättigter NaCl-Lösung gewaschen, mit Magnesiumsulfat getrocknet und eingedampft. Man erhält 3,18 g 4-Chlor-4,4-difluorbuttersäure-N-isopropylamid, Smp.: 84-85°C.

Beispiel H8: 2,4-Dichlor-4,4-difluorbuttersäuremethylester

$ClF_2C\text{-}CH_2\text{-}CHCl\text{-}COOCH_3$

Eine Lösung von 2,4 g 2,2,4-Trichlor-4,4-difluorbuttersäure-methylester in 20 ml absolutem Aethanol wird unter Zusatz von 0,2 g 5%-igem Platin/Kohle-Katalysator bis zur Aufnahme von 1 Aequivalent Wasserstoff bei Raumtemperatur hydriert (ca. 1 Stunde). Nach dem Abfiltrieren des Katalysators wird das erhaltene Rohprodukt im Kugelrohr bei +70°C/20 mbar destilliert. Man erhält 1,5 g 2,4-Dichlor-4,4-difluorbuttersäuremethylester als farbloses Oel.

Beispiel H9: 4-Chlor-4,4-difluorbuttersäure

$ClF_2C\text{-}CH_2\text{-}CH_2\text{-}COOH$

15,6 g 4-Chlor-4,4-difluorcrotonsäure werden in 160 ml Tetrahydrofuran gelöst und nach Zugabe von 0,8 g 5% $Pd/BaSO_4$-Katalysator bei Raumtemperatur und Normaldruck mit Wasserstoffgas behandelt, bis die Wasserstoff-Aufnahme 100 % der Theorie beträgt. Nach dem Abfiltrieren des Katalysators und dem Abdestillieren des Lösungsmittels wird das zurückbleibende Oel im Vakuum rektifiziert. Man erhält 12 g eines farblosen Oels vom Siedepunkt 88-90°C/11 mbar, das mit der nach Beispiel H6a hergestellten 4-Chlor-4,4-difluorbuttersäure identisch ist.

Beispiel H10: 4-Chlor-4,4-difluor-3-hydroxy-buttersäure-äthylester

$$ClF_2C\text{-}CH\text{-}CH_2\text{-}COOC_2H_5$$
|
OH

20,0 g 4-Chlor-4,4-difluor-acetessigsäure-äthylester werden in 200 ml Tetrahydrofuran gelöst und nach Zugabe von 2,0 g eines 5 %igen Rhodium/$Al_2O_3$-Katalysators bei Normaldruck und Raumtemperatur mit Wasserstoffgas bis zur Aufnahme der theoretischen Menge Wasserstoff hydriert. Eventuell muss während der Hydrierung zusätzlich noch frischer Katalysator zugefügt werden. Anschliessend wird der Katalysator abfiltriert und das Lösungsmittel im Wasserstrahlvakuum abgedampft. Die Rektifikation des Rohprodukts ergibt 18,0g reinen 4-Chlor-4,4-difluor-3-hydroxy-buttersäure-äthylester in Form eines bei 93-95 °C und 17 mbar siedenden Oels, das zu langen Nadeln erstarrt.

| Analyse: | $C_6H_9ClF_2O_3$ (202,6) | | | |
|---|---|---|---|---|
| Ber.: | C 35,6 % | H 4,5 % | Cl 17,5 % | F 18,8 % |
| Gef.: | C 35,5 % | H 4,5 % | Cl 17,1 % | F 18,7 % |

Beispiel H11: 4-Chlor-4,4-difluor-3-acetoxy-buttersäure-äthylester

$$ClF_2C\text{-}CH\text{-}CH_2\text{-}COOC_2H_5$$
|
$O\text{-}CO\text{-}CH_3$

20,2 g 4-Chlor-4,4-difluor-3-hydroxybuttersäure-äthylester und 0,1 g wasserfreies Natriumacetat werden in 50 ml Essigsäureanhydrid bei +100°C gelöst und für 1 Stunde bei dieser Temperatur gerührt. Nach dem Aufgiessen des Reaktionsgemisches auf 150 ml Wasser wird das Produkt in Diäthyläther aufgenommen. Man trocknet die ätherische Phase mit Natriumsulfat und dampft anschliessend den Aether im Vakuum ab. Die Rektifikation des Rohproduktes ergibt 20,1 g 4-Chlor-4,4-difluor-3-acetoxy-buttersäureäthylester in Form eines bei 95-97 °C und 17 mbar siedenden farblosen Oels.

| Analyse: | $C_8H_{11}ClF_2O_4$ (244,6) | | | |
|---|---|---|---|---|
| Ber.: | C 39,3 % | H 4,5 % | Cl 14,5 % | F 15,5 % |
| Gef.: | C 39,2 % | H 4,6 % | Cl 14,4 % | F 15,5 % |

Beispiel H12: 4-Chlor-4,4-difluor-crotonsäure-äthylester

$$ClF_2C\text{-}CH{=}CH\text{-}COOC_2H_5$$

24,4 g 4-Chlor-4,4-difluor-3-acetoxy-buttersäure-äthylester und 40 ml Chinolin werden gemischt und in einer Destillationsapparatur erhitzt. Bei einer Badtemperatur von ca. 200°C destilliert ein farbloses, stechend riechendes Oel vom Siedepunkt 140- 145°C ab. Das Rohprodukt wird in Diäthyläther aufgenommen , mit 1 N-Salzsäure und mit Wasser gewaschen. Nach dem Trocknen der Aetherphase mit Natriumsulfat und dem Abdampfen des Aethers erhält man 14,0 g 4-Chlor-4,4-difluor-crotonsäure-äthylester in Form eines farblosen, bei 41-43°C und 12 mbar siedenden Oels.

| | | | | |
|---|---|---|---|---|
| Analyse: $C_6H_7ClF_2O_2$ (184,6) | | | | |
| Ber.: | C 39,0 % | H 3,8 % | Cl 19,2 % | F 20,6 % |
| Gef.: | C 39,3 % | H 3,8 % | Cl 19,0 % | F 20,4 % |

Das gleiche Produkt wird erhalten , wenn man 4-Chlor-4,4-difluor-3-hydroxy-buttersäure-äthylester mit Phosphorpentoxyd nach der von McBee et al. in J. Amer. Chem. Soc. 76 (1954) 3722 für 4,4,4-Trifluor-3-hydroxy-buttersäure-äthylester angegebenen Methode behandelt.

Beispiel H13: 4-Chlor-4,4-difluorbuttersäure-äthylester

$$ClF_2C\text{-}CH_2\text{-}CH_2\text{-}COOC_2H_5$$

18,4 g 4-Chlor-4,4-difluor-crotonsäure-äthylester werden in 200 ml Essigsäure-äthylester gelöst und nach Zugabe von 2,0 g eines 5 %igen Palladium/Bariumsulfat-Katalysators bei Normaldruck und Raumtemperatur mit Wasserstoffgas behandelt. Die Hydrierung ist nach kurzer Zeit beendet. Nach dem Abfiltrieren des Katalysators wird das Lösungsmittel im Vakuum abgedampft und der Rückstand rektifiziert. Das bei 154-156°C destillierende Produkt ist mit dem nach den Beispielen H2,H3 und H4 hergestellten 4-Chlor-4,4-difluorbuttersäure-äthylester identisch.

Analog zu den beschriebenen Arbeitsweisen werden auch die in den nachfolgenden Tabellen genannten Verbindungen hergestellt.

Tabelle 1:

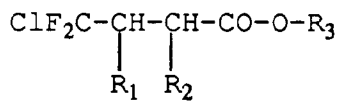

| Verb. Nr. | $R_1$ | $R_2$ | $R_3$ | Phys. Daten |
|---|---|---|---|---|
| 1.01 | H | H | $CH_3$ | MS:($M^+$, $C_5H_7ClF_2O_2$) m/e: 172 |
| 1.02 | H | H | $C_2H_5$ | MS:($M^+$, $C_6H_9ClF_2O_2$) m/e: 186 |
| 1.03 | H | H | $C_3H_7$-n | |
| 1.04 | H | H | $C_3H_7$-i | MS:($M^+$, $C_7H_{11}ClF_2O_2$) m/e: 200 |
| 1.05 | H | H | $C_4H_9$-t | MS:($M^+$-15, $C_7H_{11}ClF_2O_2$) m/e: 199 |
| 1.06 | H | H | $C_4H_9$-n | MS:($M^+$+1, $C_8H_{14}ClF_2O_2$) m/e: 215 |
| 1.07 | H | H | $C_6C_{13}$-n | MS:($M^+$, $C_{10}H_{17}ClF_2O_2$) m/e: 242 |
| 1.08 | H | H | $C_{10}H_{21}$-n | MS:($M^+$, $C_{14}H_{25}ClF_2O_2$) m/e: 298 |
| 1.09 | H | H | $C_{20}H_{41}$-n | SMP: 33-35°C |
| 1.10 | H | H | $CH_2C(CH_3)_3$ | MS:($M^+$, $C_9H_{15}ClF_2O_2$) m/e: 228 |
| 1.11 | H | H | $CH_2CH_2OH$ | MS:($M^+$, $C_6H_9ClF_2O_3$) m/e: 202 |
| 1.12 | H | H | $CH_2CH_2O$-$COCH_3$ | |
| 1.13 | H | H | $CH_2CH_2O$-$COOCH_3$ | |
| 1.14 | H | H | $CH_2CH_2OCONHC_6H_5$ | |
| 1.15 | H | H | $CH_2CH_2OC_2H_5$ | |
| 1.16 | H | H | $CH_2CH_2OCH_2CH_2OCH_3$ | MS:($M^+$, $C_9H_{15}ClF_2O_4$) m/e: 260 |
| 1.17 | H | H | $CH_2CCl_3$ | |
| 1.18 | H | H | $CH_2CBr_3$ | $n_D^{23}$ = 1,5050 |
| 1.19 | H | H | $CH_2CH_2SCH_3$ | |
| 1.20 | H | H | $CH_2CH_2OCH_2C_6H_5$ | |
| 1.21 | H | H | $CH_2CH_2Cl$ | |
| 1.22 | H | H | $CH_2$-$C_6H_5$ | MS:($M^+$, $C_{11}H_{11}ClF_2O_2$) m/e: 248 |

Tabelle 1 (Fortsetzung)

| Verb. Nr. | $R_1$ | $R_2$ | $R_3$ | Phys. Daten |
|---|---|---|---|---|
| 1.23 | H | H | $CH(CH_3)C_6H_5$ | |
| 1.24 | H | H | $CH(CH_3)C_6H_5$ (R) | |
| 1.25 | H | H | $CH(CH_3)C_6H_5$ (S) | |
| 1.26 | H | H | $CH_2$-$C_6H_4$-$NO_2$-(4) | |
| 1.27 | H | H | $CH_2$-$C_6H_4$-$NO_2$-(2) | |
| 1.28 | H | H | $CH_2$-$C_6H_4$-Cl-(2) | |
| 1.29 | H | H | $CH_2$-$C_6H_5$-F-(4) | |
| 1.30 | H | H | $CH_2$-$C_6H_4$-O-$C_6H_5$-(3) | $n_D^{23}$ = 1,5270 |
| 1.31 | H | H | -$C_6H_5$ | MS:($M^+$, $C_{10}H_9ClF_2O_2$) m/e: 234 |
| 1.32 | H | H | -$C_6H_4$-O-$C_6H_5$-(4) | $n_D^{23}$ = 1,530 |
| 1.33 | H | H | -$C_6H_4$-$NO_2$-(4) | Smp. 81-82°C |
| 1.34 | H | H | -$C_6H_4$-F-(4) | |
| 1.35 | H | H | 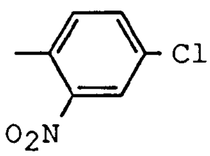 | $n_D^{23}$ = 1,5109 |
| 1.36 | H | H | 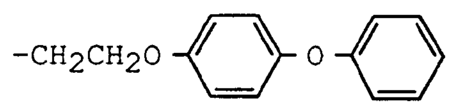 | MS:($M^+$, $C_{18}H_{17}ClF_2O_4$) m/e: 370 |
| 1.37 | H | H | 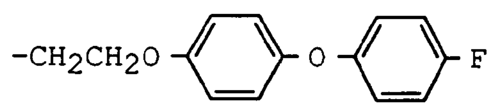 | Smp. 62-63°C |
| 1.38 | H | H | 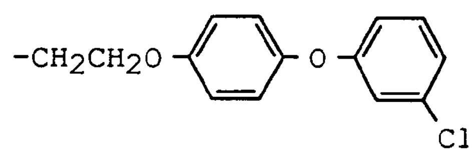 | $n_D^{20}$ = 1,5380 |
| 1.39 | H | H | 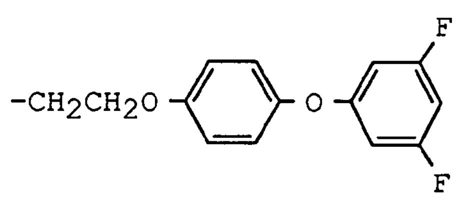 | $n_D^{20}$ = 1,5047 |
| 1.40 | H | H | $C_6H_{11}$-cycl. | MS:($M^+$-35,$C_{10}H_{15}F_2O_2$) m/e: 205 |
| 1.41 | H | H | $C_5H_9$-cycl. | |

Tabelle 1 (Fortsetzung)

| Verb. Nr. | $R_1$ | $R_2$ | $R_3$ | Phys. Daten |
|---|---|---|---|---|
| 1.42 | H | H | $C_3H_5$-cycl. | |
| 1.43 | H | H | 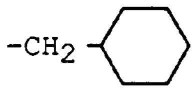 | |
| 1.44 | H | H | 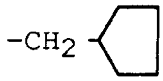 | |
| 1.45 | H | H | 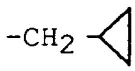 | MS:($M^+$-35, $C_8H_{11}F_2O_2$) m/e: 177 |
| 1.46 | H | $CF_3$ | $-C_2H_5$ | MS:($M^+$, $C_7H_8ClF_5O_2$) m/e: 254 |
| 1.47 | H | H | 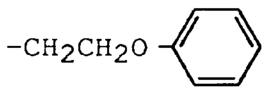 | |
| 1.48 | H | H | 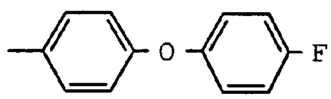 | $n_D^{23}$ = 1,5150 |
| 1.49 | H | H | H | Sdp. 88-90°/11 mbar |
| 1.50 | H | H | $-C_{16}H_{33}n$ | |
| 1.51 | H | H | $-CH(CH_2CH_2CH_2CH_3)_2$ | |
| 1.52 | H | H | $-CH_2-(CH_2)_4CH_2Cl$ | |
| 1.53 | H | H | 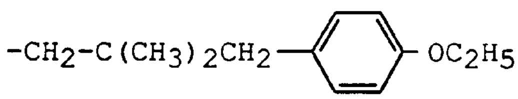 | |
| 1.54 | H | H | 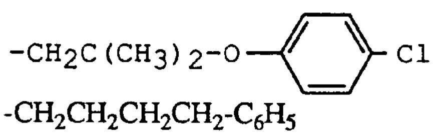 | |
| 1.55 | H | H | $-CH_2CH_2CH_2CH_2-C_6H_5$ | |
| 1.56 | H | H | 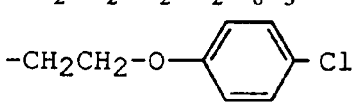 | |
| 1.57 | H | H | $-CH_2-CH=CH_2$ | |
| 1.58 | H | H | $-CH_2CH=C(CH_3)CH_2CH_2CH=C(CH_3)_2$ | |
| 1.59 | H | H | $-CH_2CH=C(CH_3)CH_2CH_2CH=C(CH_3)-CH_2CH_2CH=C(CH_3)_2$ | |
| 1.60 | H | H | $-CH_2-CH\equiv CH$ | |

Tabelle 1 (Fortsetzung)

| Verb. Nr. | $R_1$ | $R_2$ | $R_3$ | Phys. Daten |
|---|---|---|---|---|
| 1.61 | H | H | $-CH_2CH_2-C{\equiv}C-(CH_2)_4CH_3$ | |
| 1.62 | H | H | $-CH_2CH_2O-SO_2CH_3$ | |
| 1.63 | H | H | 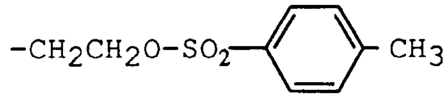 | |
| 1.64 | H | H | $-CH_2CH_2O-COC_6H_5$ | |
| 1.65 | H | H | 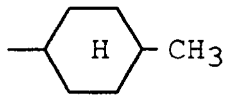 | |
| 1.66 | H | H | 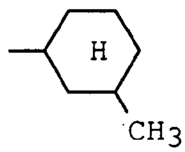 | |
| 1.67 | H | H | 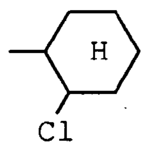 | |
| 1.68 | H | H | 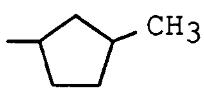 | |
| 1.69 | H | H | 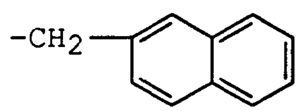 | |
| 1.70 | H | H | 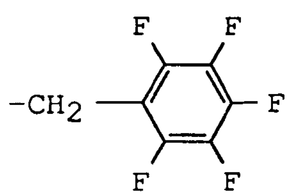 | |
| 1.71 | H | H | 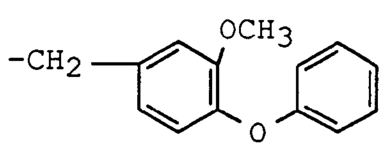 | |

Tabelle 1 (Fortsetzung)

| Verb. Nr. | $R_1$ | $R_2$ | $R_3$ | Phys. Daten |
|---|---|---|---|---|
| 1.72 | H | H | 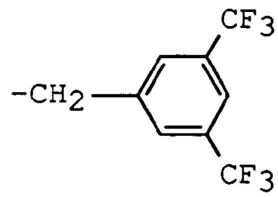 | |
| 1.73 | H | H | $-CH_2-C_6H_4-Br-(4)$ | |
| 1.74 | H | H | 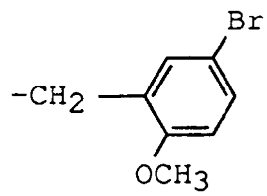 | |
| 1.75 | H | H | 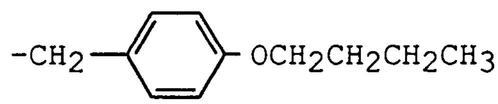 | |
| 1.76 | H | H | 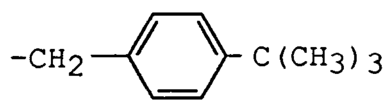 | |
| 1.77 | H | H | 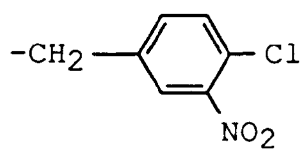 | |
| 1.78 | H | H | $-CH_2-C_6H_4-Cl-(3)$ | |
| 1.79 | H | H | $-CH_2-C_6H_4-Cl-(4)$ | |
| 1.80 | H | H | 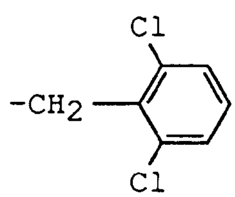 | |
| 1.81 | H | H | 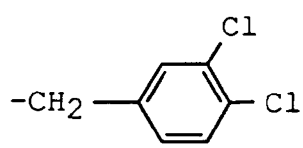 | |
| 1.82 | H | H | 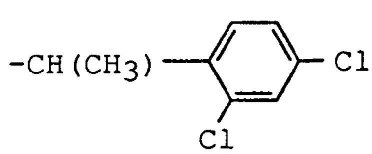 | |

Tabelle 1 (Fortsetzung)

| Verb. Nr. | $R_1$ | $R_2$ | $R_3$ | Phys. Daten |
|---|---|---|---|---|
| 1.83 | H | H | 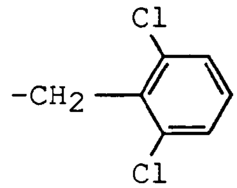 | |
| 1.84 | H | H | 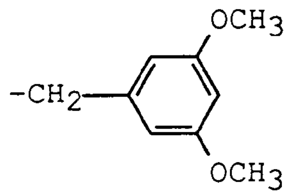 | |
| 1.85 | H | H | 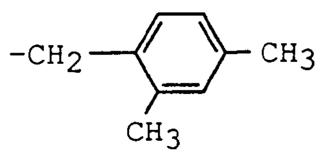 | |
| 1.86 | H | H | 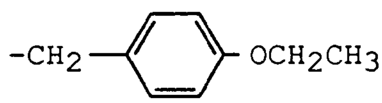 | |
| 1.87 | H | H | $-CH_2-C_6H_4-C_2H_5-(4)$ | |
| 1.88 | H | H | $-CH_2-C_6H_4-F-(3)$ | |
| 1.89 | H | H | $-CH_2-C_6H_4-OH-(4)$ | |
| 1.90 | H | H | $-CH_2-C_6H_4-J-(4)$ | |
| 1.91 | H | H | 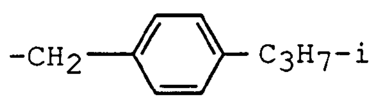 | |
| 1.92 | H | H | $-CH_2-C_6H_4-OCH_3-(4)$ | |
| 1.93 | H | H | $-CH_2-C_6H_4-CH_3-(4)$ | |
| 1.94 | H | H | $-CH_2(C_2H_5)-C_6H_5$ | |
| 1.95 | H | H | $-CH_2-C_6H_4-CF_3-(4)$ | |
| 1.96 | H | H | $-CH_2-C_6H_4-CF_3-(3)$ | |
| 1.97 | H | H | 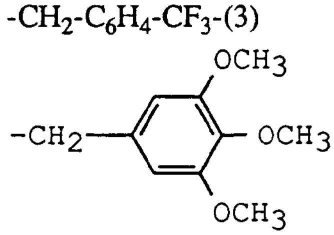 | |

Tabelle 1 (Fortsetzung)

| Verb. Nr. | $R_1$ | $R_2$ | $R_3$ | Phys. Daten |
|---|---|---|---|---|
| 1.98 | H | H | 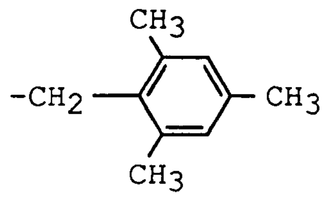 | |
| 1.99 | H | H | 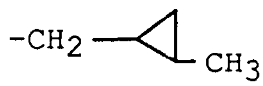 | |
| 1.100 | H | H | $-C_6H_4$-Br-(4) | |
| 1.101 | H | H | 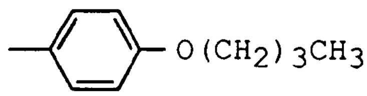 | |
| 1.102 | H | H | 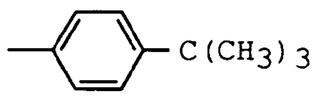 | |
| 1.103 | H | H | 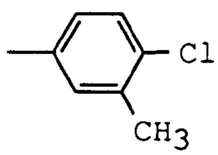 | |
| 1.104 | H | H | $-C_6H_4$-Cl-(2) | |
| 1.105 | H | H | $-C_6H_4$-Cl-(3) | |
| 1.106 | H | H | $-C_6H_4$-Cl-(4) | |
| 1.107 | H | H | 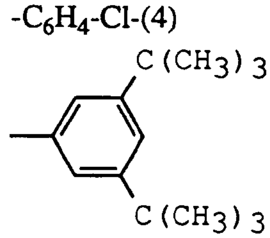 | |
| 1.108 | H | H | 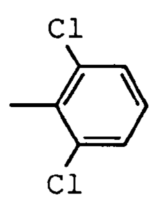 | |
| 1.109 | H | H | 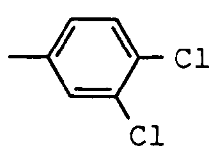 | |

Tabelle 1 (Fortsetzung)

| Verb. Nr. | R1 | R2 | R3 | Phys. Daten |
|---|---|---|---|---|
| 1.110 | H | H | 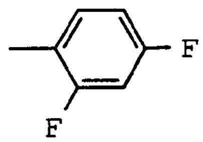 | |
| 1.111 | H | H | 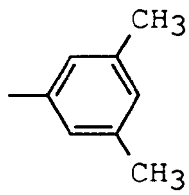 | |
| 1.112 | H | H | 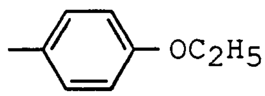 | |
| 1.113 | H | H | 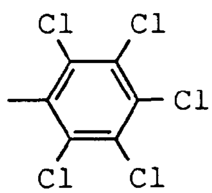 | |
| 1.114 | H | H | 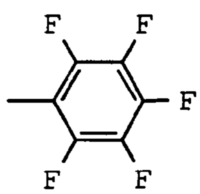 | |
| 1.115 | H | H | 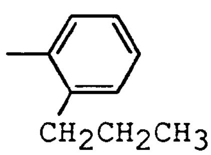 | |
| 1.116 | H | H | 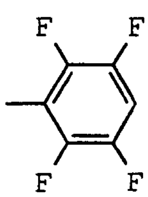 | |

Tabelle 1 (Fortsetzung)

| Verb. Nr. | $R_1$ | $R_2$ | $R_3$ | Phys. Daten |
|---|---|---|---|---|
| 1.117 | H | H | 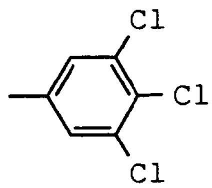 | |
| 1.118 | H | H | 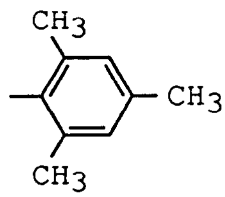 | |
| 1.119 | H | H | 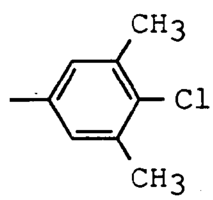 | |
| 1.120 | $CH_3$ | H | -H | |
| 1.121 | $CH_3$ | H | $-C_6H_5$ | |
| 1.122 | $C_2H_5$ | H | $-C_6H_5$ | |
| 1.123 | $C_2H_5$-n | H | $-C_6H_5$ | |
| 1.124 | $CH_3$ | H | 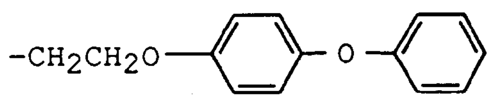 | |
| 1.125 | $CH(CH_3)_2$ | H | 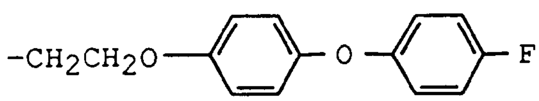 | |
| 1.126 | $CH_3$ | H | $-CH_2C_6H_5$ | |
| 1.127 | $C_3H_7$-n | H | -H | |
| 1.128 | H | $CF_3$ | 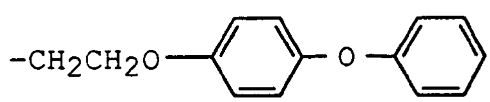 | |

Tabelle 1 (Fortsetzung)

| Verb. Nr. | $R_1$ | $R_2$ | $R_3$ | Phys. Daten |
|---|---|---|---|---|
| 1.129 | H | H | 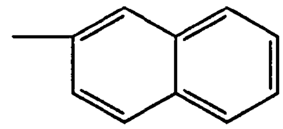 | |
| 1.130 | H | H | 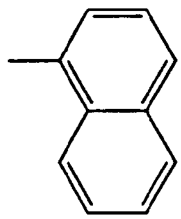 | |
| 1.131 | H | H | 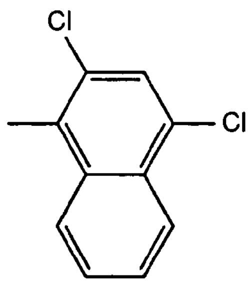 | |
| 1.132 | H | H | 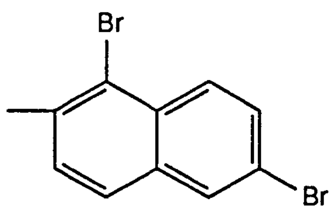 | |
| 1.133 | H | H | 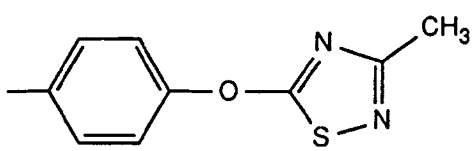 | |
| 1.134 | H | H | 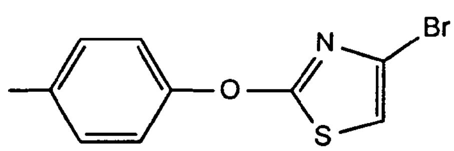 | |

Tabelle 1 (Fortsetzung)

| Verb. Nr. | $R_1$ | $R_2$ | $R_3$ | Phys. Daten |
|---|---|---|---|---|
| 1.135 | H | H | 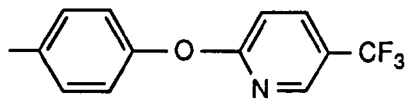 | Smp. 70°C |
| 1.136 | H | H | 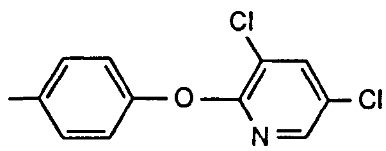 | |
| 1.137 | H | H | 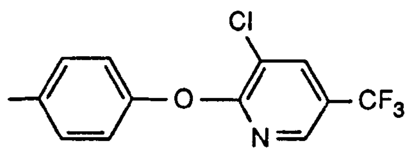 | |
| 1.138 | $C_3H_7$-n | H | $C_2H_5$ | |
| 1.139 | H | H | 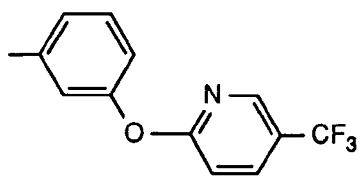 | |
| 1.140 | H | H | 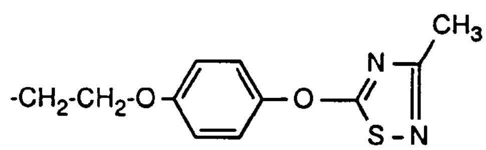 | |

Tabelle 2:

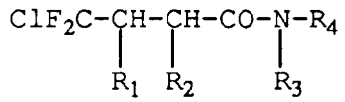

| Verb. Nr. | $R_1$ | $R_2$ | $R_4$ | $R_3$ | Phys. Daten |
|---|---|---|---|---|---|
| 2.01 | H | H | H | $-CH_3$ | Smp. 61-63°C |
| 2.02 | H | H | $CH_3$ | $-CH_3$ | MS:($M^+$, $C_6H_{10}ClF_2NO$) m/e: 185 |
| 2.03 | H | H | $C_6H_{13}$-n | $-C_6H_{13}$-n | |
| 2.04 | H | $CF_3$ | H | $-CH_3$ | |
| 2.05 | H | H | H | $-C_2H_5$ | Smp. 50-51°C |
| 2.06 | H | H | H | $-C_3H_7$-n | |
| 2.07 | H | H | H | $-C_3H_7$-i | Smp. 84-85°C |
| 2.08 | H | H | H | $-C_4H_9$-n | Smp. 30°C |
| 2.09 | H | H | H | $-C_4H_9$-t | Smp. 97-98°C |
| 2.10 | H | H | H | $-C_6H_{13}$-n | |
| 2.11 | H | H | H | $-C_{10}H_{21}$-n | Smp. 40-41°C |
| 2.12 | H | H | H | $-C_{20}H_{41}$-n | |
| 2.13 | H | H | H | $-CH_2C(CH_3)_3$ | |
| 2.14 | H | H | H | $-CH_2CH_2OH$ | |
| 2.15 | H | H | H | $-CH_2CH_2O-COC_6H_5$ | |
| 2.16 | H | H | H | $-CH_2CH_2O-COOCH_3$ | |
| 2.17 | H | H | H | $-CH_2CH_2O-CONHC_6H_5$ | |
| 2.18 | H | H | H | $-CH_2CH_2OC_2H_5$ | |
| 2.19 | H | H | H | $-CH_2CF_3$ | Smp. 55-56°C |
| 2.20 | H | H | H | $-CH_2CCl_3$ | |
| 2.21 | H | H | H | $-CH(CH_3)C_6H_5$ | Smp. 60-61°C |
| 2.22 | H | H | H | $-CH(CH_3)C_6H_5$ (R) | |
| 2.23 | H | H | H | $-CH(CH_3)C_6H_5$ (S) | |
| 2.24 | H | H | H | $-CH_2-C_6H_5$ | Smp. 74-75°C |
| 2.25 | H | H | H | $-CH_2-C_6H_4-NO_2$-(4) | |
| 2.26 | H | H | H | $-CH_2-C_6H_4-F$-(4) | |
| 2.27 | H | H | H | $-CH_2-C_6H_4-Cl$-(2) | |
| 2.28 | H | H | H | $-C_6H_5$ | Smp. 121-122°C |
| 2.29 | H | H | H | $-C_6H_4-NO_2$-(4) | |

Tabelle 2 (Fortsetzung)

| Verb. Nr. | $R_1$ | $R_2$ | $R_4$ | $R_3$ | Phys. Daten |
|---|---|---|---|---|---|
| 2.30 | H | H | H | $-C_6H_4-F-(4)$ | Smp. 96-97°C |
| 2.31 | H | H | H | 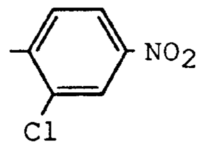 | Smp. 110-111°C |
| 2.32 | H | H | H | 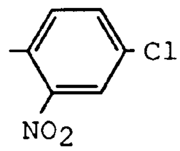 | Smp. 122-123°C |
| 2.33 | H | H | $CH_3$ | $-CH_2$ 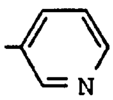 | MS:($M^+$,$C_{11}H_{13}ClF_2N_2O$) m/e: 262 |
| 2.34 | H | H | $CH_3$ | $-CH_2$ 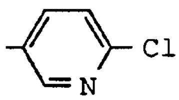 | |
| 2.35 | H | H | H | $-CH_2$ 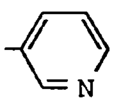 | Smp. 47-49°C |
| 2.36 | H | H | H | $-C_6H_4-CN-(4)$ | Smp. 122-123°C |
| 2.37 | H | H | H | $-C_6H_4-CF_3-(2)$ | Smp. 106-107°C |
| 2.38 | H | H | H | $-C_6H_4-C_6H_5-(4)$ | Smp. 186-187°C |
| 2.39 | H | H | H | $-C_6H_4-CF_3-(3)$ | Smp. 77-79°C |
| 2.40 | H | H | H | 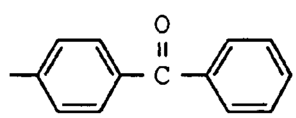 | Smp. 143-144°C |
| 2.41 | H | H | H | 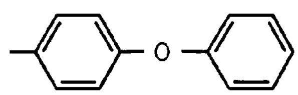 | Smp. 120-121°C |
| 2.42 | H | H | H | $-C_6H_4-Cl-(2)$ | Smp. 105-106°C |
| 2.43 | H | H | H | $-C_6H_4-Cl-(4)$ | Smp. 150-151°C |
| 2.44 | H | H | H | $-C_6H_4-Cl-(3)$ | Smp. 93-94°C |
| 2.45 | H | H | H | $-C_6H_4-OCH_3-(4)$ | Smp. 113-114°C |
| 2.46 | H | H | H | $-C_6H_4-CH_3-(4)$ | Smp. 120-121°C |

Tabelle 2 (Fortsetzung)

| Verb. Nr. | $R_1$ | $R_2$ | $R_4$ | $R_3$ | Phys. Daten |
|---|---|---|---|---|---|
| 2.47 | H | H | H | $-C_6H_4-SCH_3-(3)$ | Smp. 88-89°C |
| 2.48 | H | H | H | H | Smp. 92-93°C |
| 2.49 | H | H | H | $-C_{16}H_{33}-n$ | |
| 2.50 | H | H | H | $-C_{18}H_{37}-n$ | Smp. 71-72°C |
| 2.51 | H | H | H | $-C_{12}H_{25}-n$ | |
| 2.52 | H | H | H | $-C_9H_{19}-n$ | |
| 2.53 | H | H | H | $-C_8H_{17}-n$ | |
| 2.54 | H | H | H | $-CH_2(CH_3)-CH_2CH_2CH_3$ | |
| 2.55 | H | H | H | $-CH_2CH_2CH_2CH_2OH$ | |
| 2.56 | H | H | H | $-CH_2CH_2CH_2CH_2OCH_3$ | |
| 2.57 | H | H | H | 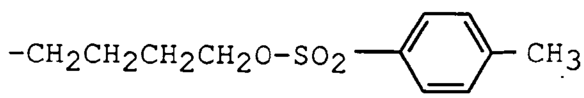 | |
| 2.58 | H | H | H | $-CH_2CH_2CH_2CH_2O-SO_2CH_3$ | |
| 2.59 | H | H | H | 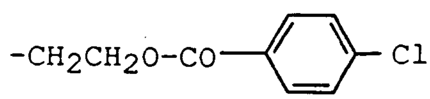 | |
| 2.60 | H | H | H | $-CH_2CH_2O-CH_2CF_3$ | |
| 2.61 | H | H | H | $-CH_2CH_2N(CH_3)_2$ | |
| 2.62 | H | H | H | $-CH_2(CH_3)-CH_2CH_2CH_2-N(CH_2CH_3)_2$ | |
| 2.63 | H | H | H | 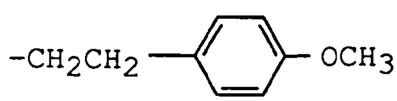 | |
| 2.64 | H | H | H | 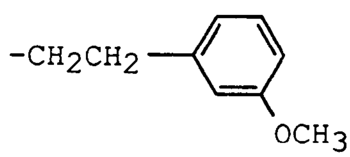 | |
| 2.65 | H | H | H | $-CH_2(CH_3)-C(CH_3)_3$ | |
| 2.66 | H | H | H | $-CH_2CH(OCH_2CH_3)_2$ | |
| 2.67 | H | H | H | $-CH_2-CO-C_6H_5$ | |
| 2.68 | H | H | H | 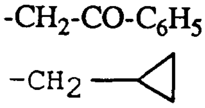 | |

Tabelle 2 (Fortsetzung)

| Verb. Nr. | $R_1$ | $R_2$ | $R_4$ | $R_3$ | Phys. Daten |
|---|---|---|---|---|---|
| 2.69 | H | H | H | 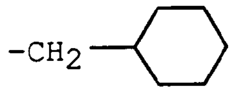 | |
| 2.70 | H | H | H | $-CH_2(CH_3)-CH_2OCH_3$ | |
| 2.71 | H | H | H | $-CH_2CH_2-CH(CH_3)_2$ | |
| 2.72 | H | H | H | $-CH_2(CH_2)_7-CH=CH-(CH_2)_7CH_3$ | |
| 2.73 | H | H | $CH_3$ | $-C_{18}H_{37}$-n | |
| 2.74 | H | H | $CH_3$ | $-C_3H_7$-n | |
| 2.75 | H | H | $CH_3$ | $-CH_2CH_2-C_6H_5$ | |
| 2.76 | H | H | H | $-CH_2-C_6H_4-OCH_3$-(4) | Smp. 106-108°C |
| 2.77 | H | H | H | $-CH_2-C_6H_4-F$-(2) | |
| 2.79 | H | H | H | $-CH_2-C_6H_3-F_2$-(2,6) | |
| 2.80 | H | H | H | $-CH_2-C_6H_3-F_2$-(2,4) | |
| 2.80 | H | H | H | $-CH_2-C_6H_3-F_2$-(3,4) | |
| 2.81 | H | H | H | $-CH_2-C_6H_4-Cl$-(4) | Smp. 102-103°C |
| 2.82 | H | H | H | $-CH_2-C_6H_3-Cl_2$-(3,4) | |
| 2.83 | H | H | H | $-CH_2-C_6H_4-CF_3$-(4) | |
| 2.84 | H | H | H | $-CH_2-C_6H_4-NO_2$-(3) | |
| 2.85 | H | H | H | $-CH(C_6H_5)_2$ | |
| 2.86 | H | H | H | 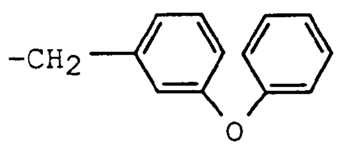 | |
| 2.87 | H | H | H | 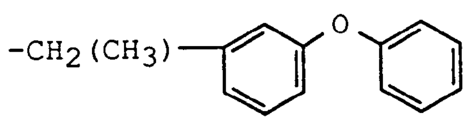 | |
| 2.88 | H | H | H | 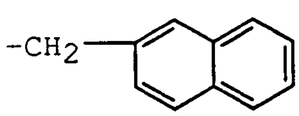 | |

Tabelle 2 (Fortsetzung)

| Verb. Nr. | R1 | R2 | R4 | R3 | Phys. Daten |
|---|---|---|---|---|---|
| 2.89 | H | H | H | 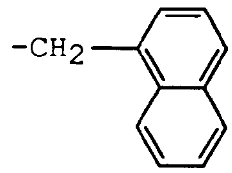 | |
| 2.90 | H | H | H | 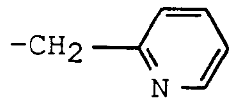 | |
| 2.91 | H | H | H | 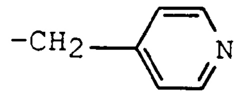 | |
| 2.92 | H | H | $CH_3$ | $-CH_2-C_6H_5$ | |
| 2.93 | H | H | $C_2H_5$ | $-CH_2-C_6H_5$ | |
| 2.94 | H | H | $C_3H_7$-i | $-CH_2-C_6H_5$ | |
| 2.95 | H | H | $C_4H_9$-n | $-CH_2-C_6H_5$ | |
| 2.96 | H | H | H | $-C_6H_{11}$-cyclo | |
| 2.97 | H | H | H | $-C_3H_5$-cyclo | |
| 2.98 | H | H | H | 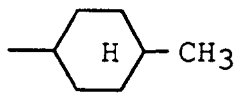 | |
| 2.99 | H | H | H | 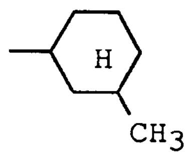 | |
| 2.100 | H | H | H | $-CH_2-C{\equiv}CH$ | |
| 2.101 | H | H | $CH_3$ | $-CH_2-C{\equiv}CH$ | |
| 2.102 | H | H | H | $-CH_2-CH{\equiv}CH_2$ | |
| 2.103 | H | H | H | $-C_6H_4-COCH_3-(4)$ | |
| 2.104 | H | H | H | $-C_6H_4-CONH_2-(3)$ | |
| 2.105 | H | H | H | $-C_6H_4-CN-(3)$ | |

Tabelle 2 (Fortsetzung)

| Verb. Nr. | $R_1$ | $R_2$ | $R_4$ | $R_3$ | Phys. Daten |
|---|---|---|---|---|---|
| 2.106 | H | H | H | 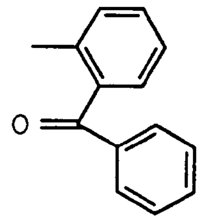 | |
| 2.107 | H | H | H | $-C_6H_4-Br-(4)$ | |
| 2.108 | H | H | H | 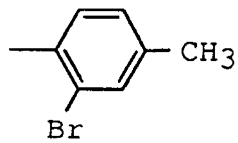 | |
| 2.109 | H | H | H | 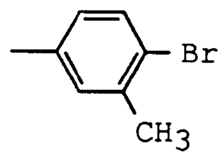 | |
| 2.110 | H | H | H | 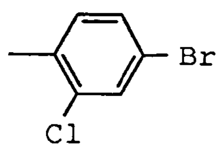 | |
| 2.111 | H | H | H | 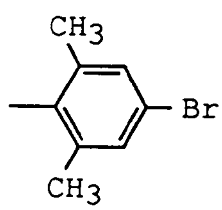 | |
| 2.112 | H | H | H | 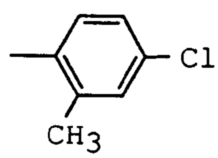 | |
| 2.113 | H | H | H | 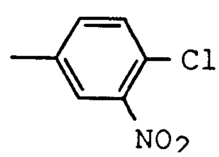 | |

Tabelle 2 (Fortsetzung)

| Verb. Nr. | $R_1$ | $R_2$ | $R_4$ | $R_3$ | Phys. Daten |
|---|---|---|---|---|---|
| 2.114 | H | H | H | 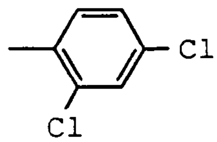 | |
| 2.115 | H | H | H | 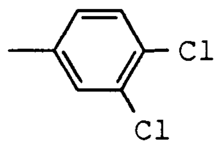 | |
| 2.116 | H | H | H | 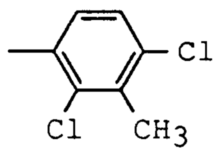 | |
| 2.117 | H | H | H | 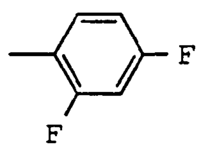 | |
| 2.118 | H | H | H | 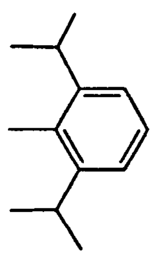 | |
| 2.119 | H | H | H | 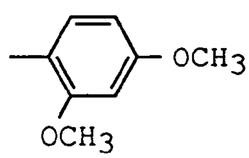 | |
| 2.120 | H | H | H | 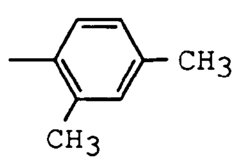 | |
| 2.121 | H | H | H | 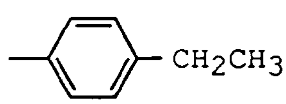 | |

Tabelle 2 (Fortsetzung)

| Verb. Nr. | $R_1$ | $R_2$ | $R_4$ | $R_3$ | Phys. Daten |
|---|---|---|---|---|---|
| 2.122 | H | H | H | 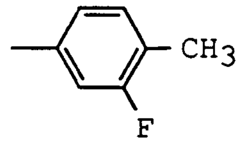 | |
| 2.123 | H | H | H | 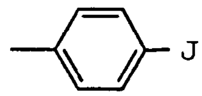 | |
| 2.124 | H | H | H | 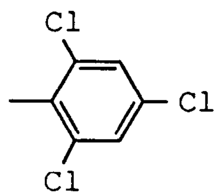 | |
| 2.125 | H | H | H | 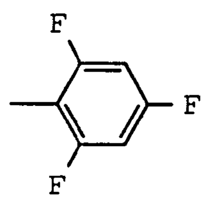 | |
| 2.126 | H | H | H | 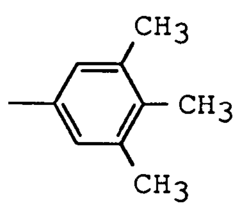 | |
| 2.127 | H | H | H | $-C_6H_4-OH-(4)$ | |
| 2.128 | H | H | H | 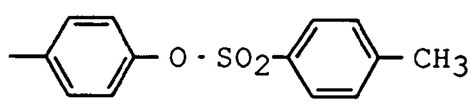 | |
| 2.129 | H | H | H | 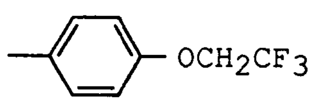 | |

Tabelle 2 (Fortsetzung)

| Verb. Nr. | $R_1$ | $R_2$ | $R_4$ | $R_3$ | Phys. Daten |
|---|---|---|---|---|---|
| 2.130 | H | H | $CH_3$ | $C_6H_5$ | |
| 2.131 | H | H | $CH_3$ | $C_6H_4$-$NO_2$-(4) | |
| 2.132 | H | H | $C_2H_5$ | $C_6H_4$-Cl-(4) | |
| 2.133 | $CH_3$ | H | H | -$C_6H_5$ | |
| 2.134 | $CH_3$ | H | H | -$C_2H_5$ | |
| 2.135 | $CH_3$ | H | H | -$CH_2$-$C_6H_5$ | |
| 2.136 | $C_2H_5$ | H | H | -$CH_2$-$C_6H_5$ | |
| 2.137 | $C_4H_9$-n | H | H | -$CH_2$-$C_6H_5$ | |
| 2.138 | H | H | $CH_3$ | -$CH_2$-$C_6H_5$ | |
| 2.139 | $CH_3$ | H | $CH_3$ | -$C_6H_5$ | |
| 2.140 | H | H | H | 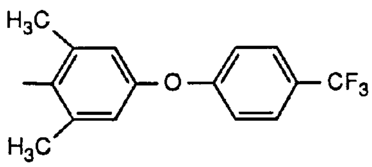 | Smp. 145-147°C |
| 2.141 | H | H | H | 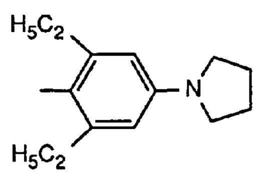 | Smp. 207-208°C |
| 2.142 | H | H | H | 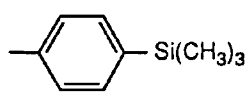 | Smp. 94-95°C |
| 2.143 | H | H | H | 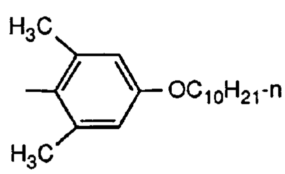 | Smp. 91-93°C |
| 2.144 | H | H | H | 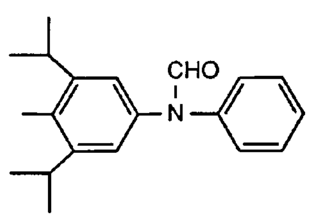 | Smp. 62-64°C |

Tabelle 2 (Fortsetzung)

| Verb. Nr. | $R_1$ | $R_2$ | $R_4$ | $R_3$ | Phys. Daten |
|---|---|---|---|---|---|
| 2.145 | H | H | H | 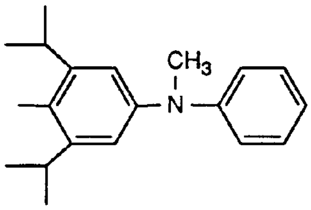 | Smp. 179-181°C |
| 2.146 | H | H | H | 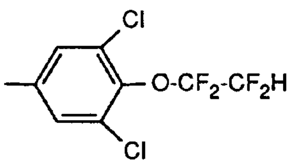 | Smp. 114-115°C |
| 2.147 | H | H | H | 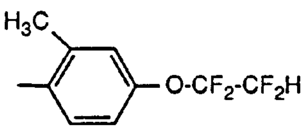 | Smp. 114-115°C |
| 2.148 | H | H | H | 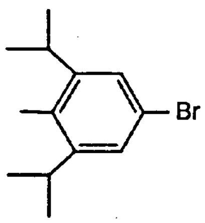 | Smp. 149-151°C |
| 2.149 | H | H | H | 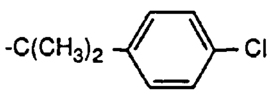 | Smp. 131-133°C |
| 2.150 | H | H | H | 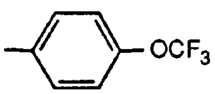 | Smp. 88-90°C |
| 2.151 | H | H | H | 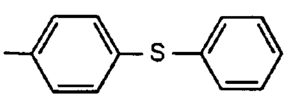 | Smp. 130-133°C |

Tabelle 2 (Fortsetzung)

| Verb. Nr. | $R_1$ | $R_2$ | $R_4$ | $R_3$ | Phys. Daten |
|---|---|---|---|---|---|
| 2.152 | H | H | H | 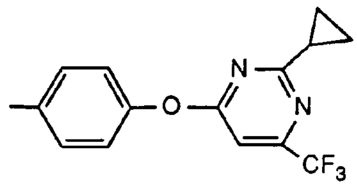 | Smp. 127-128°C |
| 2.153 | H | H | H | 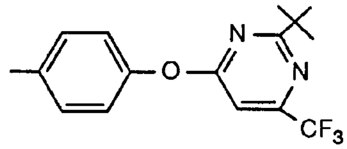 | |
| 2.154 | H | H | H | 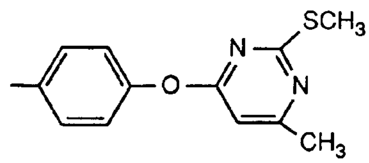 | |
| 2.155 | H | H | H | 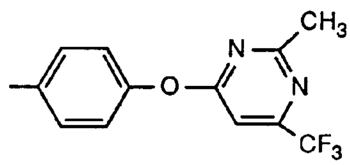 | |
| 2.156 | H | H | H | 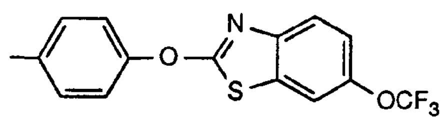 | Smp. 188-189°C |
| 2.157 | H | H | H | 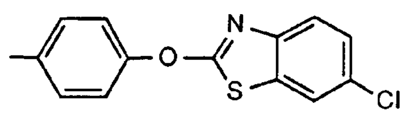 | |
| 2.158 | H | H | H | 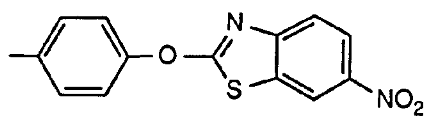 | |
| 2.159 | H | H | H | 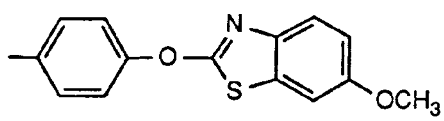 | |

Tabelle 2 (Fortsetzung)

| Verb. Nr. | $R_1$ | $R_2$ | $R_4$ | $R_3$ | Phys. Daten |
|---|---|---|---|---|---|
| 2.160 | H | H | H | 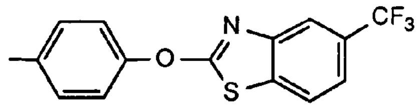 | |
| 2.161 | H | H | H | 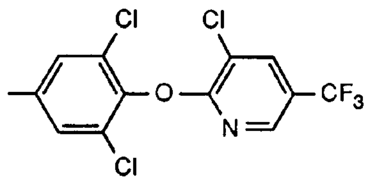 | Smp. 149-150°C |
| 2.162 | H | H | H | 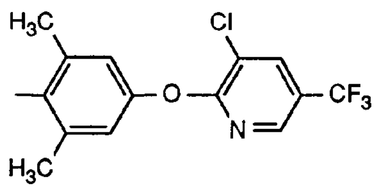 | Smp. 205-207°C |
| 2.163 | H | H | H | 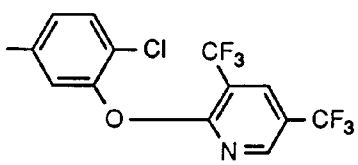 | Smp. 107-109°C |
| 2.164 | H | H | H | 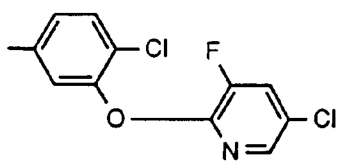 | Smp. 137-139°C |
| 2.165 | H | H | H | 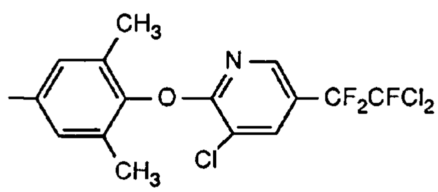 | Smp. 130-132°C |
| 2.166 | H | H | H | 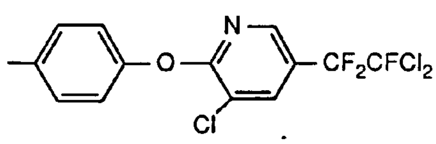 | Smp. 122-124°C |

Tabelle 2 (Fortsetzung)

| Verb. Nr. | R1 | R2 | R4 | R3 | Phys. Daten |
|---|---|---|---|---|---|
| 2.167 | H | H | H | 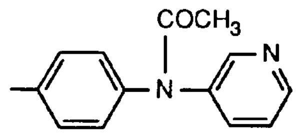 | |
| 2.168 | H | H | H | 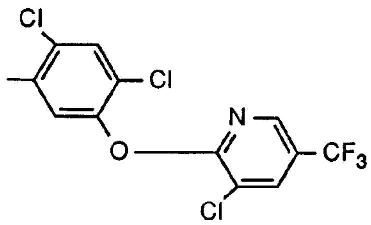 | |
| 2.169 | H | H | H | 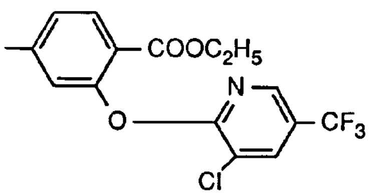 | |
| 2.170 | H | H | H | 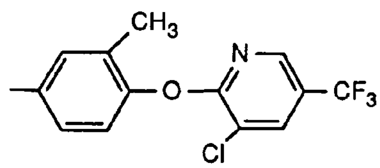 | |
| 2.171 | H | H | H | 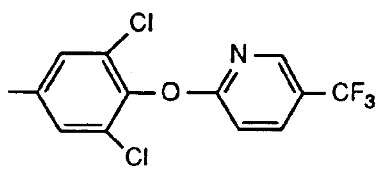 | |
| 2.172 | H | H | H | 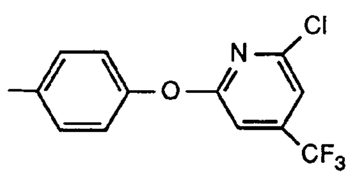 | |
| 2.173 | H | H | H | 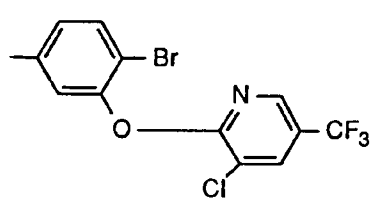 | |

Tabelle 2 (Fortsetzung)

| Verb. Nr. | $R_1$ | $R_2$ | $R_4$ | $R_3$ | Phys. Daten |
|---|---|---|---|---|---|
| 2.174 | H | H | H | 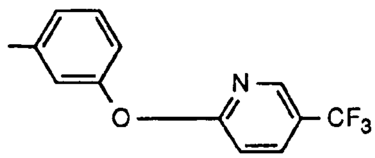 | |
| 2.175 | H | H | H | 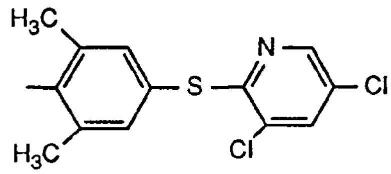 | |
| 2.176 | H | H | H | 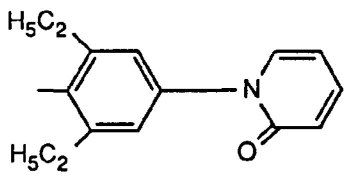 | |
| 2.177 | H | H | H | 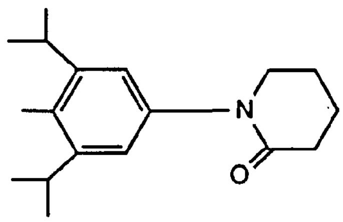 | |
| 2.178 | H | H | H | 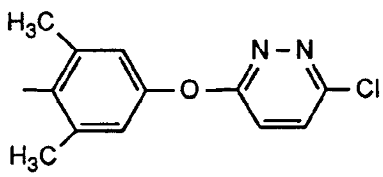 | |
| 2.179 | H | H | H | 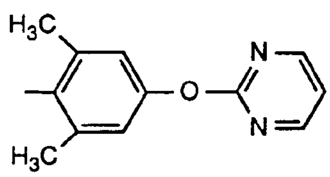 | |
| 2.180 | H | H | H | 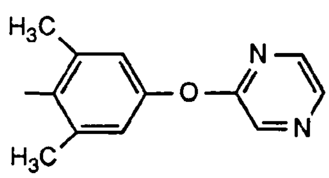 | |

Tabelle 2 (Fortsetzung)

| Verb. Nr. | $R_1$ | $R_2$ | $R_4$ | $R_3$ | Phys. Daten |
|---|---|---|---|---|---|
| 2.181 | H | H | H | 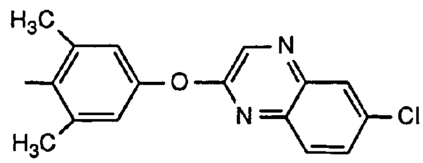 | |
| 2.182 | H | H | H | 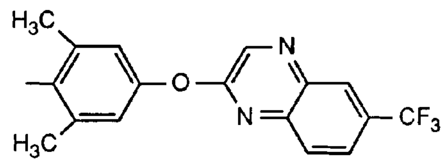 | |
| 2.183 | H | H | H | 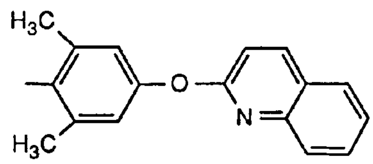 | |
| 2.184 | H | H | H | 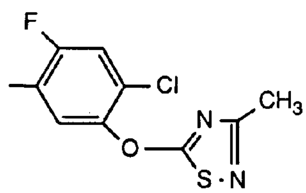 | Smp. 131-133°C |
| 2.185 | H | H | H | 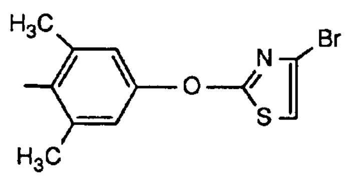 | |
| 2.186 | H | H | H | $-CH_2CH_2Cl$ | Smp. 43-44°C |
| 2.187 | H | H | H | $-CH_2-C_6H_4-N(CH_3)_2-(4)$ | Smp. 124-125°C |
| 2.188 | H | H | H | $-CH_2-C_6H_4-C(CH_3)_3-(4)$ | Smp. 121-122°C |
| 2.189 | H | H | H | $-CH_2-C_6H_4-CH_3-(4)$ | Smp. 104-105°C |
| 2.190 | H | H | H | 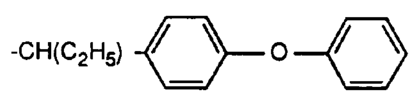 | Smp. 97-98°C |
| 2.191 | H | H | H | 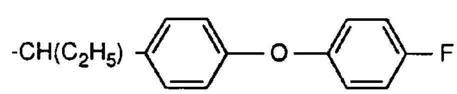 | Smp. 135°C |

Tabelle 2 (Fortsetzung)

| Verb. Nr. | $R_1$ | $R_2$ | $R_4$ | $R_3$ | Phys. Daten |
|---|---|---|---|---|---|
| 2.192 | H | H | H | 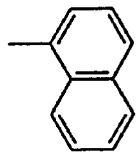 | |
| 2.193 | H | H | H | 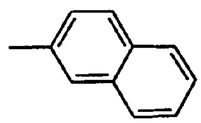 | Smp. 135-136°C |
| 2.194 | H | H | H | 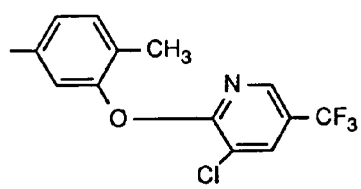 | Smp. 93-96°C |
| 2.195 | H | H | H | 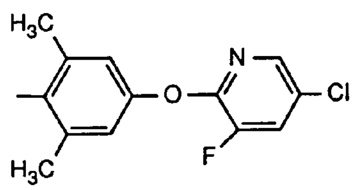 | Smp. 163-165°C |
| 2.196 | H | H | H | $-CH_2-C(CH_3)_2$ 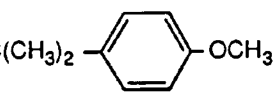 | |

Formulierungsbeispiele (% = Gewichtsprozent)

| Beispiel F1: Emulsions-Konzentrate | a) | b) | c) |
|---|---|---|---|
| Wirkstoff Nr. 1.01 oder 1.02 | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel F2: Lösungen | a) | b) | c) | d) |
|---|---|---|---|---|
| Wirkstoff Nr. 1.04 | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| Beispiel F3: Granulate | a) | b) |
|---|---|---|
| Wirkstoff Nr. 2.02 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| Beispiel F4: Stäubemittel | a) | b) |
|---|---|---|
| Wirkstoff Nr. 1.45 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoffe erhält man gebrauchsfertige Stäubemittel.

| Beispiel F5: Spritzpulver | a) | b) | c) |
|---|---|---|---|
| Wirkstoff Nr. 2.09 | 25 % | 50 % | 75 % |
| Na-Ligninsulfonat | 5 % | 5 % | - |
| Na-Laurylsulfat | 3 % | - | 5 % |
| Na-Diisobutylnaphthalin-sulfonat | - | 6 % | 10 % |
| Octylphenolpolyäthylenglykol-äther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

Beispiel F6: Emulsions-Konzentrat

| | |
|---|---|
| Wirkstoff Nr. 2.01 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Beispiel F7: Stäubemittel | a) | b) |
|---|---|---|
| Wirkstoff Nr. 2.24 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Beispiel F8: Extruder-Granulat

| | |
|---|---|
| Wirkstoff Nr. 2.08 | 10 % |
| Na-Ligninsulfonat | 2 % |
| Carboxymethylcellulose | 1 % |

| | |
|---|---|
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

Beispiel F9: Umhüllungs-Granulat

| | |
|---|---|
| Wirkstoff Nr. 2.33 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

Beispiel F10: Suspensions-Konzentrat

| | |
|---|---|
| Wirkstoff Nr. 2.07 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%-ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1: Wirkung gegen Boophilus microplus

Vollgesogene adulte Zecken Weibchen werden auf eine PVC Platte aufgeklebt und mit einem Wattebausch überdeckt. Zur Behandlung werden die Testtiere mit 10 mi einer wässrigen Testlösung, die 125 ppm des zu prüfenden Wirkstoffes enthält übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.

Verbindungen gemäss Tabellen 1 und 2 zeigen in diesem Test gute Wirkung gegen Boophilus microplus. Insbesondere die Verbindungen 1.01, 1.04, 1.05, 1.06, 1.10, 1.18, 1.21, 1.22, 1.30, 1.31, 1.33, 1.35, 1.36, 1.37, 1.38, 1.39, 1.40, 1.45, 1.46, 2.01, 2.02, 2.05, 2.07, 2.08, 2.09, 2.24, 2.28, 2.30, 2.31, 2.32, 2.33 und 2.35 zeigen eine Wirkung über 80%.

Beispiel B2: Ovizide Wirkung auf Heliothis virescens

Auf Filterpapier abgelegte Eier von Heliothis virescens werden für kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

Verbindungen gemäss Tabellen 1 und 2 zeigen in diesem Test gute Wirkung gegen Heliothis virescens.

Insbesondere die Verbindungen 1.01, 1.02, 1.04, 1.06, 1.10, 1.22, 1.31, 1.36, 1.40, 1.45, 1.46, 2.01, 2.02, 2.05, 2.07, 2.08, 2.24 und 2.28 zeigen eine Wirkung über 80%.

Beispiel B3: Wirkung gegen Aonidiella aurantii

Kartoffelknollen werden mit Wanderlarven ("Grawlern") von Aonidiella aurantii (rote Citrus-Schildlaus) besiedelt. Nach etwa 2 Wochen werden die Kartoffeln in eine wässrige Emulsions- resp. Suspensions-Spritzbrühe getaucht, die den zu prüfenden Wirkstoff in einer Konzentration von 400 ppm enthält. Nach dem Abtrocknen der so behandelten Kartoffelknollen werden diese in einem Plastikbehälter inkubiert. Zur Auswertung wird 10-12 Wochen später die Ueberlebensrate der Wanderlarven der ersten Folgegeneration der behandelten Schildlaus-Population mit derjenigen der unbehandelten Kontrollansätze verglichen.

Verbindungen gemäss Tabellen 1 und 2 zeigen in diesem Test gute Wirkung gegen Aonidiella aurantii. Insbesondere die Verbindungen 1.18, 1.21, 1.22, 1.30, 1.31, 1.32, 1.33, 1.35, 1.37, 1.38, 1.39, 1.48, 1.49, 2.01, 2.05, 2.07, 2.08, 2.30, 2.37, 2.42, 2.43, 2.44, 2.45, 2.46 und 2.48 zeigen eine Wirkung über 80%.

Beispiel 84: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Verbindungen der Tabellen 1 und 2 zeigen eine gute Wirkung gegen Nilaparvata lugens in diesem Test. Insbesondere die Verbindungen 1.10, 1.22, 1.30, 1.31, 1.33, 1.35, 1.36, 1.37, 1.38, 1.39, 1.40, 1.45, 1.48, 1.49, 2.05, 2.07, 2.08, 2.24, 2.28, 2.30, 2.31, 2.32, 2.33, 2.35 2.36, 2.39, 2.41, 2.42, 2.43, 2.45, 2.46 und 2.47 zeigen eine Wirkung über 80 %.

Beispiel B5: Wirkung gegen Tetranychus urticae

Junge Bohnenpflanzen werden mit einer Mischpopulation von Tetranychus urticae besiedelt und 1 Tag später mit einer wässrigen Emulsions-Spritzbrühe, die 400 ppm des Wirkstoffes enthält, besprüht. Die Pflanzen werden anschliessend für 6 Tage bei 25°C inkubiert und danach ausgewertet. Aus dem Vergleich der Anzahl toter Eier, Larven und Adulten auf den behandelten zu denjenigen auf den unbehandelten Pflanzen wird die prozentuale Reduktion der Population (% Wirkung) bestimmt.

Verbindungen der Tabellen 1 und 2 zeigen eine gute Wirkung gegen Tetranychus urticae in diesem Test. Insbesondere die Verbindungen 1.30, 1.33, 1.35, 1.36, 1.38, 1.39, 2.28, 2.39, 2.41, 2.43 und 2.44 zeigen eine Wirkung über 80 %.

Beispiel B6: Wirkung gegen Anthonomus grandis Adulte

Junge Baumwollpflanzen werden mit einer wässrigen Emulsions-Spritzbrühe, die 400 ppm des Wirkstoffes enthält, besprüht. Nach dem Antrocknen des Spritzbelages werden die Baumwollpflanzen mit 10 Adulten von Anthonomus grandis besiedelt und in einen Plastikbehälter gegeben. 3 Tage später erfolgt die Auswertung. Aus dem Vergleich der Anzahl toter Käfer und des Frasschadens auf den behandelten zu denjenigen auf den unbehandelten Pflanzen wird die prozentuale Reduktion der Population bezw. die prozentuale Reduktion des Frasschadens (% Wirkung) bestimmt.

Verbindungen der Tabellen 1 und 2 zeigen eine gute Wirkung gegen Anthonomus grandis in diesem Test. Insbesondere die Verbindungen 1.22, 1.36, 1.37, 1.49, 2.28, 2.32, 2.38 und 2.39 zeigen eine Wirkung über 80 %.

Beispiel B7: Wirkung gegen Aphis craccivora

Erbsenkeimlinge werden mit Aphis craccivora infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Verbindungen der Tabellen 1 und 2 zeigen eine gute Wirkung gegen Aphis craccivora in diesem Test. Insbesondere die Verbindungen 2.05, 2.07, 2.08, 2.24, 2.28, 2.35 und 2.39 zeigen eine Wirkung über 80 %.

Beispiel B8: Systemische Wirkung gegen Myzus persicae

Erbsenkeimlinge werden mit Myzus persicae infiziert, anschliessend mit den Wurzeln in eine Spritzbrühe, die 400 ppm des Wirkstoffes enthält, gestellt und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Verbindungen der Tabellen 1 und 2 zeigen eine gute Wirkung gegen Myzus persicae in diesem Test. Insbesondere die Verbindungen 2.05, 2.24 und 2.28 zeigen eine Wirkung über 80 %.

Beispiel B9: Systemische Wirkung gegen Nilaparvata lugens

Töpfe mit Reispflanzen werden in eine wässrige EmulsionsLösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.

Verbindungen der Tabellen 1 und 2 zeigen eine gute Wirkung gegen Nilaparvata lugens in diesem Test. Insbesondere die Verbindungen 1.01, 1.02, 1.04, 1.05, 1.06, 1.10, 1.18, 1.21, 1.22, 1.30, 1.31, 1.33, 1.35, 1.36, 1.37, 1.38, 1.39, 1.40, 1.45, 1.46, 1.48, 1.49, 2.01, 2.05, 2.07, 2.08, 2.09, 2.24, 2.28, 2.30, 2.31, 2.32, 2.33, 2.35, 2.36, 2.37, 2.38, 2.39, 2.40, 2.41, 2.42, 2.43, 2.44, 2.45, 2.46, 2.47 und 2.48 zeigen auch bei 12,5 ppm noch eine Wirkung über 80 %.

Beispiel B10: Ovizide Wirkung von Adoxophyes reticulana

Auf Filterpapier abgelegte Eier von Adoxophyes reticulana werden für kurze Zeit in eine acetonisch-wässrige Testlösung, die 400 ppm des zu prüfenden Wirkstoffes enthält, eingetaucht. Nach dem Antrocknen der Testtlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

Verbindungen gemäss Tabellen 1 und 2 zeigen in diesem Test gute Wirkung gegen Adoxophyes reticulana. Insbesondere die Verbindungen 1.02, 1.22, 1.31, 1.36, 2.05, 2.07, 2.08 und 2.35 zeigen eine Wirkung über 80 %.

Beispiel B11: Ovo/larvizide Wirkung auf Heliothis virescens

Auf Baumwolle abgelegte Eier von Heliothis virescens werden mit einer wässrigen Emulsions-Spritzbrühe, die 400 ppm des Wirkstoffes enthält, besprüht. Nach 8 Tagen wird der prozentuale Schlupf der Eier und die Ueberlebensraten der Raupen im Vergleich zu unbehandelten Kontrollansätzen ausgewertet (% Reduktion der Population).

Verbindungen gemäss Tabellen 1 und 2 zeigen in diesem Test gute Wirkung gegen Heliothis virescens. Insbesondere die Verbindungen 1.01, 1.02, 1.04, 1.05, 1.06, 1.07, 1.08, 1.09, 1.10, 1.18, 1.21, 1.22, 1.30, 1.31, 1.32, 1.33, 1.35, 1.36, 1.37, 1.38, 1.39, 1.40, 1.45, 1.46, 1.48, 1.49, 2.01, 2.02, 2.05, 2.07, 2.08, 2.09, 2.24, 2.28, 2.30, 2.31, 2.32, 2.33, 2.35, 2.36, 2.37, 2.38, 2.39, 2.40, 2.41, 2.42, 2.43, 2.44, 2.45, 2.46, 2.47 und 2.48 zeigen eine Wirkung über 80%.

Beispiel B12: Wirkung gegen Dermanyssus gallinae

In einem nach oben offenen Glasbehälter werden 2 bis 3 ml einer 10 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen werden kann. Nach 3 Tagen wird die Mortalität der Milben ermittelt.

Verbindungen gemäss Tabellen 1 und 2 zeigen gute Wirkung gegen Dermanyssus gallinae in diesem Test. Insbesondere die Verbindungen 1.04, 1.06, 1.10, 1.22, 1.31, 1.36, 1.40, 1.45, 2.01 und 2.02 zeigen eine Wirkung über 80 %.

Beispiel B13: Wirkung geben Heliothis virescens Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des ersten

Stadiums von Heliothis virescens besiedelt und in einen Plastikbehälter gegeben. 6 Tage später erfolgt die Auswertung. Aus dem Vergleich der Anzahl toter Raupen und des Frasschadens auf dem behandelten zu denjenigen auf den unbehandelten Pflanzen wird die prozentuale Reduktion der Population bzw. die prozentuale Reduktion des Frassschadens (% Wirkung) bestimmt.

Verbindungen gemäss Tabellen 1 und 2 zeigen eine gute Wirkung gegen Heliothis virescens in diesem Test. Insbesondere die Verbindungen 2.01, 2.07, 2.24 und 2.33 zeigen eine Wirkung über 80 %.

Beispiel B14: Ovizide Wirkung auf Cydia pomonella

Auf Filterpapier abgelegte Eier von Cydia pomonella werden für eine kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

Verbindungen gemäss Tabellen 1 und 2 zeigen in diesem Test gute Wirkung gegen Cydia pomonella.

Beispiel B15: Wirkung gegen Nephotettix cincticeps

Reispflanzen werden mit einer wässrigen Emulsions-Spritzbrühe, die 400 ppm des Wirkstoffes enthält, behandelt Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Verbindungen der Tabellen 1 und 2 zeigen eine gute Wirkung gegen Nephotettix cincticeps in diesem Test. Insbesondere die Verbindungen 1.33, 1.37, 1.38, 1.49 und 2.48 zeigen eine Wirkung über 80 %.

Beispiel B16: Wirkung gegen Ctenocephalides felis

20 bis 25 Floheier werden in eine waagerecht stehende 50-ml-Zellkulturflasche gegeben, in der zuvor 15 g Flohlarven-Nahrmedium, welches 100 ppm des zu prüfenden Wirkstoffs enthält, vorgelegt wurde. Die Testflaschen werden in einem Brutschank bei 26-27°C und 60-70 % Luftfeuchtigkeit bebrütet. Nach 21 Tagen wird die Anwesenheit von adulten Flöhen, nicht geschlüpften Puppen und Larven überprüft.

Verbindungen der Tabellen 1 und 2 zeigen eine gute Wirkung gegen Ctenocephalides felis in diesem Test.

Beispiel B17: Wirkung gegen Diabrotica balteata Eier

20 bis 50 auf Tuchfilter abgelegte Eier von D. balteata werden in eine Petrischale gegeben und mit einer wässrigen Emulsions-Spritzbrühe, die 400 ppm des Wirkstoffes enthält, behandelt. Die Petrischalen werden bei 24°C inkubiert. Nach 7 Tagen wird der prozentuale Schlupf der Eier im Vergleich zu unbehandelten Kontrollansätzen ausgewertet (% Schlupfreduktion).

Verbindungen der Tabellen 1 und 2 zeigen eine gute Wirkung gegen Diabrotica balteata in diesem Test. Insbesondere die Verbindungen 2.05, 2.08 und 2.24 zeigen eine Wirkung über 80 %.

Beispiel B18: Wirkung gegen Bemisia tabaci Eier

Buschbohnen-Pflanzen werden in Gazekäfige gestellt und mit Adulten von Bemisia tabaci (Weisse Fliege) besiedelt. Nach erfolgter Eiablage werden alle Adulten entfernt und 2 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 10 Tage nach der Wirkstoff-Applikation auf %-Schlupf im Vergleich zu den unbehandelten Kontrollansätzen.

Verbindungen gemäss Tabellen 1 und 2 zeigen in diesem Test gute Wirkung gegen Bemisia tabaci.

## Patentansprüche

### Patentansprüche für folgende Vertragsstaaten : AT, BE, CH, DE, DK, FR, GB, GR, IT, LI, LU, NL

**1.** 4-Chlor-4,4-difluorbuttersäure-Derivate Formel I

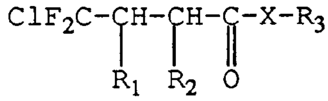

(I)

worin

$R_1$ und $R_2$ unabhängig voneinander Wasserstoff, $C_1$-$C_4$-Alkyl oder $C_1$-$C_4$-Halogenalkyl,
$R_3$ Wasserstoff oder einen organischen Rest und
X Sauerstoff oder -$NR_4$- bedeuten,
wobei $R_4$ für Wasserstoff oder $C_1$-$C_6$-Alkyl steht.

2. Verbindungen gemäss Anspruch 1, dadurch gekennzeichnet, dass $R_3$ für Wasserstoff oder für jeweils substituiertes oder unsubstituiertes $C_1$-$C_{20}$-Alkyl, $C_3$-$C_7$-Cycloalkyl, $C_3$-$C_{20}$-Alkenyl, $C_3$-$C_{20}$-Alkinyl, Benzyl oder Aryl steht.

3. Verbindungen gemäss Anspruch 1, dadurch gekennzeichnet, dass $R_3$ Wasserstoff, $C_1$-$C_{20}$-Alkyl, $C_3$-$C_7$-Cycloalkyl, $C_3$-$C_{20}$-Alkenyl, $C_3$-$C_{20}$-Alkinyl, Aryl, $C_3$-$C_{20}$-Halogenalkenyl, $C_3$-$C_{20}$-Halogenalkinyl; durch Halogen oder $C_1$-$C_4$-Alkyl substituiertes $C_3$-$C_7$-Cycloalkyl; durch Halogen, $C_1$-$C_4$-Alkyl, $C_1$-$C_4$-Halogenalkyl, $C_1$-$C_{12}$-Alkoxy, $C_1$-$C_4$-Halogenalkoxy, $C_1$-$C_4$-Alkylthio, Nitro, Cyan, Benzoyl, Halogenbenzoyl, Phenoxy, Halogenphenoxy, $C_1$-$C_4$-Alkylphenoxy, $C_1$-$C_4$-Halogenalkylphenoxy, Tri-$C_1$-$C_4$-alkylsilyl, N-Pyrrolidinyl, N-Piperidinyl, N-Pyrrolidin-2-onyl, N-Piperidin-2-onyl, $C_1$-$C_4$-Alkylamino, Di-$C_1$-$C_4$-alkylamino, Anilino, N-$C_1$-$C_4$-Alkylanilino, N-Formylanilino, N-$C_1$-$C_6$-Alkylcarbonylanilino, Phenylthio oder Halogenphenylthio substituiertes Aryl; durch einen über Sauerstoff oder Schwefel gebundenen unsubstituierten oder substituierten aromatischen oder nichtaromatischen, mono- oder bicyclischen Heterocyclus substituiertes Phenyl, wobei sowohl der Heterocyclus als auch der Phenylring jeweils durch Halogen, $C_1$-$C_4$-Alkyl, Nitro, $C_1$-$C_4$-Halogenalkyl, $C_1$-$C_4$-Halogenalkoxy, $C_1$-$C_4$-Alkoxy, $C_1$-$C_4$-Alkylthio oder Cyclopropyl substituiert sein können; oder durch Hydroxy, Halogen, Di-$C_1$-$C_4$-alkylamino, $C_1$-$C_4$-Alkoxy, $C_1$-$C_4$-Halogenalkoxy, $C_2$-$C_6$-Alkoxyalkoxy, $C_1$-$C_4$-Halogenalkylthio, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy, $C_3$-$C_7$-Cycloalkyl, Aryl, Aryloxy, Arylthio, Arylsulfonyl, Arylsulfinyl, Arylsulfonyloxy, Arylcarbonyl oder Pyridyl substituiertes $C_1$-$C_{20}$-Alkyl steht; wobei die Aryl- und Pyridylgruppen jeweils durch Halogen, $C_1$-$C_4$-Alkyl, $C_1$-$C_4$-Halo genalkyl, $C_1$-$C_4$-Alkoxy, $C_1$-$C_4$-Halogenalkoxy, $C_1$-$C_4$-Alkylthio, Nitro, Cyan, Phenoxy, Halogenphenoxy, Phenylthio oder Halogenphenylthio substituiert sein können.

4. Verbindungen gemäss Anspruch 1, dadurch gekennzeichnet, dass $R_1$ und $R_2$ unabhängig voneinander Wasserstoff oder $C_1$-$C_4$-Alkyl, vorzugsweise jedoch Wasserstoff bedeuten.

5. Verbindungen gemäss Anspruch 1, dadurch gekennzeichnet, dass X Sauerstoff, -NH-, -$NCH_3$- oder -$NC_2H_5$-, vorzugsweise jedoch Sauerstoff oder -NH- bedeutet.

6. Verbindungen gemäss Anspruch 3, dadurch gekennzeichnet, dass $R_3$ für durch in 4-Stellung durch einen über Sauerstoff gebundenen aromatischen mono- oder bicyclischen Heterocyclus aus der Reihe Pyridin, Pyrimidin oder Benzothioazol substituiertes Phenyl steht, wobei beide aromatischen Ringe unsubstituiert sind oder zusammen höchstens drei weitere Substituenten aus der Reihe Chlor, Brom, Methyl, Aethyl und Trifluormethyl tragen.

7. Verbindungen gemäss Anspruch 3, dadurch gekennzeichnet, dass $R_1$ und $R_2$ unabhängig voneinander Wasserstoff oder $C_1$-$C_4$-Alkyl, X Sauerstoff oder -$NR_4$- und $R_4$ Wasserstoff, Methyl oder Aethyl bedeuten und $R_3$ für Wasserstoff, $C_1$-$C_{20}$-Alkyl, $C_3$-$C_7$-Cycloalkyl, $C_3$-$C_{20}$-Alkenyl, $C_3$-$C_{20}$-Alkinyl, Phenyl, Naphthyl, $C_3$-$C_{20}$-Halogenalkenyl, $C_3$-$C_{20}$-Halogenalkinyl, durch Fluor, Chlor, Brom oder $C_1$-$C_3$-Alkyl substituiertes $C_3$-$C_7$-cycloalkyl, durch Fluor, Chlor, Brom oder $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, $C_1$-$C_4$-Alkylphenoxy, $C_1$-$C_4$-Halogenalkylphenoxy, Tri-$C_1$-$C_4$-alkylsilyl, N-Pyrrolidinyl, N-Piperidinyl, N-Pyrrolidin-2-onyl, N-Piperidin-2-onyl, $C_1$-$C_4$-Alkylamino, Di-$C_1$-$C_4$-alkylamino, Anilino, N-$C_1$-$C_4$-Alkylanilino, N-Formylanilino, N$C_1$-$C_6$-Alkylcarbonylanilino, Phenylthio oder Halogenphenylthio substituiertes Phenyl oder Naphthyl, oder durch Hydroxy, Fluor, Chlor, Brom, Di-$C_1$-$C_4$-alkylamino, $C_1$-$C_4$-Alkoxy, $C_1$-$C_4$-Halogenalkoxy, $C_2$-$C_6$-Alkoxyalkoxy, $C_1$-$C_4$-Halogenalkylthio, $C_1$-$C_4$-Alkylthio, $C_1C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy, $C_3$-$C_7$-Cycloalkyl, Phenyl, Phenoxy, Phenylthio, Phenylsulfonyloxy oder Pyridyl substituiertes $C_1$-$C_{20}$-Alkyl steht, wobei die Phenyl-

und Pyridylgruppen jeweils durch Fluor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, Phenylthio oder Halogenphenylthio substituiert sein können.

**8.** Verbindungen gemäss Anspruch 1, dadurch gekennzeichnet, dass $R_1$ und $R_2$ unabhängig voneinander Wasserstoff oder $C_1$-$C_4$-Alkyl, X Sauerstoff oder -$NR_4$-, $R_4$ Wasserstoff, Methyl oder Aethyl, $R_3$ Phenyl, Benzyl, Naphthyl, 3-Pyridylmethyl, oder jeweils durch Fluor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, Phenylthio oder Halogenphenylthio substituiertes Phenyl, Benzyl, Naphthyl oder 3-Pyridylmethyl bedeuten.

**9.** Verbindungen gemäss Anspruch 1, dadurch gekennzeichnet, dass $R_1$ und $R_2$ unabhängig voneinander Wasserstoff oder $C_1$-$C_4$-Alkyl, X Sauerstoff oder -$NR_4$-, $R_4$ Wasserstoff oder $C_1$-$C_4$-Alkyl, $R_3$ $C_1$-$C_{12}$-Alkyl oder durch Hydroxy, Fluor, Chlor, Brom, Dimethylamino, Methoxy, Aethoxy, Methoxyäthoxy, Aethoxyäthoxy, Methylthio, Aethylthio, Cyclopropyl, Cyclopentyl, Cyclohexyl, Phenyl oder Phenoxy substituiertes $C_1$-$C_{12}$-Alkyl bedeuten, wobei der Phenyl- oder Phenoxyrest jeweils durch Fluor, Chlor, Brom, Phenoxy, Halogenphenoxy oder Phenylthio substituiert sein kann.

**10.** Verbindungen gemäss Anspruch 1, dadurch gekennzeichnet, dass $R_1$ und $R_2$ unabhängig voneinander Wasserstoff oder $C_1$-$C_4$-Alkyl, X Sauerstoff oder -$NR_4$-, $R_4$ Wasserstoff oder $C_1$-$C_4$-Alkyl, $R_3$ $C_3$-$C_{12}$-Alkenyl, $C_3$-$C_{12}$-Alkinyl oder jeweils durch Fluor, Chlor oder Brom substituiertes $C_3$-$C_{12}$-Alkenyl oder $C_3$-$C_{12}$-Alkinyl bedeuten.

**11.** Verbindungen gemäss Anspruch 3, dadurch gekennzeichnet, dass $R_1$ und $R_2$ Wasserstoff, und X Sauerstoff, -NH-, -$NCH_3$- oder -$NC_2H_5$- bedeuten.

**12.** Verbindungen gemäss Anspruch 1 ausgewählt aus der Reihe
4-Chlor-4,4-difluorbuttersäure-methylester,
4-Chlor-4,4-difluorbuttersäure-äthylester,
4-Chlor-4,4-difluorbuttersäure-isopropylester,
4-Chlor-4,4-difluorbuttersäure-tert-butylester,
4-Chlor-4,4-difluorbuttersäure-n-butylester,
4-Chlor-4,4-difluorbuttersäure-(2,2-dimethylpropyl)-ester,
4-Chlor-4,4-difluorbuttersäure-benzylester,
4-Chlor-4,4-difluorbuttersäure-phenylester,
4-Chlor-4,4-difluorbuttersäure-[2-(4-phenoxyphenoxy)-äthyl]-ester,
4-Chlor-4,4-difluorbuttersäure-cyclohexylester,
4-Chlor-4,4-difluorbuttersäure-cyclohexylmethylester,
4-Chlor-4,4-difluorbuttersäure-cyclopropylmethylester,
4-Chlor-4,4-difluor-2-trifluormethylbuttersäure-äthylester,
4-Chlor-4,4-difluorbuttersäure-N-methylamid,
4-Chlor-4,4-difluorbuttersäure-N,N-dimethylamid,
4-Chlor-4,4-difluorbuttersäure-N,N-dihexylamid,
4-Chlor-4,4-difluorbuttersäure-N-äthylamid,
4-Chlor-4,4-difluorbuttersäure-N-isopropylamid,
4-Chlor-4,4-difluorbuttersäure-N-butylamid,
4-Chlor-4,4-difluorbuttersäure-N-tert-butylamid,
4-Chlor-4,4-difluorbuttersäure-N-benzylamid,
4-Chlor-4,4-difluorbuttersäure-anilid,
4-Chlor-4,4-difluorbuttersäure-N-methyl-N-pyrid-3-ylmethylamid,
4-Chlor-4,4-difluorbuttersäure-N-pyrid-3-ylmethylamid,
4-Chlor-4,4-difluorbuttersäure-(4-chlor-anilid),
4-Chlor-4,4-difluorbuttersäure-(4-phenoxy-anilid),
4-Chlor-4,4-difluorbuttersäure-(2-chlor-anilid),
4-Chlor-4,4-difluorbuttersäure-(4-methoxy-anilid),
4-Chlor-4,4-difluorbuttersäure-(4-methyl-anilid),
4-Chlor-4,4-difluorbuttersäure-(3-methylmercapto-anilid),
4-Chlor-4,4-difluorbuttersäure-(4-fluor-anilid),

4-Chlor-4,4-difluorbuttersäure-(4-chlor-2-nitro-anilid),
4-Chlor-4,4-difluorbuttersäure-(3-phenoxy-benzyl)-ester,
4-Chlor-4,4-difluorbuttersäure-[4-(4-fluorphenoxy)-phenyl]-ester,
4-Chlor-4,4-difluorbuttersäure-[4-(4-fluor-phenoxy)-phenoxy-äthyl]-ester,
4-Chlor-4,4-difluorbuttersäure-(4-nitro-phenyl)-ester,
4-Chlor-4,4-difluorbuttersäure-[4-(3,5-difluor-phenoxy)-phenyl]-ester,
4-Chlor-4,4-difluorbuttersäure-[4-(5-trifluormethyl-pyrid-2-yloxy)phenyl]-ester,
4-Chlor-4,4-difluorbuttersäure und
4-Chlor-4,4-difluorbuttersäureamid.

**13.** Verfahren zur Herstellung der Verbindungen der Formel I gemäss Anspruch 1, dadurch gekennzeichnet, dass man entweder

a) ein 4-Chlor-4,4-difluorbuttersäurehalogenid der Formel II

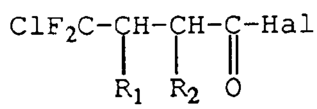

(II)

worin $R_1$ und $R_2$ die unter Formel I gegebenen Bedeutungen haben und Hal für Halogen, vorzugsweise Chlor oder Brom steht, in Gegenwart einer Base mit einer Verbindung der Formel III

$$H\text{-}X\text{-}R_3 \quad \text{(III)}$$

worin X und $R_3$ die unter Formel I gegebenen Bedeutungen haben, umsetzt, oder

b) 4-Chlor-4,4-difluorbuttersäure der Formel Ic

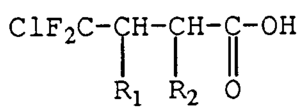

(Ic)

worin $R_1$ und $R_2$ die unter Formel I gegebenen Bedeutungen haben, in Gegenwart eines wasserabspaltenden Mittels mit einer Verbindung der Formel III umsetzt.

**14.** Schädlingsbekämpfungsmittel, welches als aktive Komponente mindestens eine Verbindung der Formel I nach Anspruch 1 enthält.

**15.** Mittel gemäss Anspruch 14, dadurch gekennzeichnet, dass es zusätzlich noch mindestens einen Träger enthält.

**16.** Verwendung einer Verbindung der Formel I nach Anspruch 1 zur Bekämpfung von Schädlingen an Tieren und Pflanzen.

**17.** Verwendung gemäss Anspruch 16, dadurch gekennzeichnet, dass es sich bei den Schädlingen um pflanzenschädigende Insekten und Arachniden handelt.

**18.** Verfahren zur Bekämpfung von tier- und pflanzenschädigenden Insekten und Arachniden, dadurch gekennzeichnet, dass man die Schädlinge oder ihren Lebensraum mit einer wirksamen Menge einer Verbindung der Formel I gemäss Anspruch 1 behandelt.

**19.** 4-Chlor-4,4-difluorbuttersäurehalogenide der Formel II

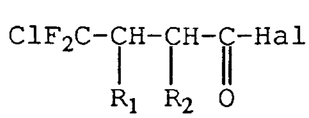

(II)

worin $R_1$ und $R_2$ unabhängig voneinander Wasserstoff, $C_1$-$C_4$-Alkyl oder $C_1$-$C_4$-Halogenalkyl bedeuten, und Hal für Halogen, vorzugsweise Chlor oder Brom, steht.

**20.** 4-Chlor-4,4-difluorcrotonsäurederivate der Formel VIII

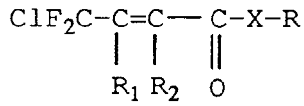

(VIII)

worin
R $C_1$-$C_6$-Alkyl,
$R_1$ und $R_2$ unabhängig voneinander Wasserstoff, $C_1$-$C_4$-Alkyl oder $C_1$-$C_4$-Halogenalkyl und
X Sauerstoff oder -$NR_4$- bedeuten,
wobei $R_4$ für Wasserstoff oder $C_1$-$C_6$-Alkyl steht, mit der Massgabe, dass in Verbindungen der Formel VIII, worin X Sauerstoff ist und R Äthyl ist, $R_2$ von Wasserstoff verschieden ist, wenn $R_1$ für $CF_2H$, $CF_2Cl$ oder $CF_3$ steht.

**21.** Verfahren zur Herstellung der Verbindungen der Formel I, dadurch gekennzeichnet, dass man ein 4-Chlor-4,4-difluorcrotonsäurederivat der Formel VIII,

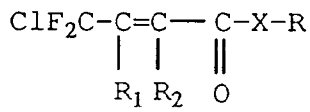

(VIII)

worin
R Wasserstoff oder $C_1$-$C_6$-Alkyl,
$R_1$ und $R_2$ unabhängig voneinander Wasserstoff, $C_1$-$C_4$-Alkyl oder $C_1$-$C_4$-Halogenalky,
und
X Sauerstoff oder -$NR_4$- bedeuten,
wobei $R_4$ für Wasserstoff oder $C_1$-$C_6$-Alkyl steht, mit Wasserstoff in Gegenwart eines Katalysators hydriert und die erhaltenen Verbindungen der Formel Id

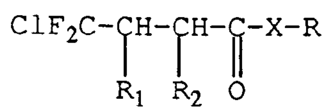

(Id)

worin $R_1$, $R_2$, X und R die oben gegebenen Bedeutungen haben, durch Hydrolyse, Umesterungsreaktion oder Umamidierungsreaktion in die übrigen Verbindungen der Formel I gemäss Anspruch 1 überführt.

**Patentansprüche für folgenden Vertragsstaat : ES**

**1.** Schädlingsbekämpfungsmittel, welches als aktive Komponente mindestens ein 4-Chlor-4,4-difluorbuttersäure-Derivat Formel I

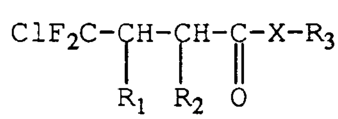

(I)

enthält, worin
$R_1$ und $R_2$ unabhängig voneinander Wasserstoff, $C_1$-$C_4$-Alkyl oder $C_1$-$C_4$-Halogenalkyl,
$R_3$ Wasserstoff oder einen organischen Rest und
X Sauerstoff oder -$NR_4$- bedeuten,
wobei $R_4$ für Wasserstoff oder $C_1$-$C_6$-Alkyl steht.

**2.** Mittel gemäss Anspruch 1, dadurch gekennzeichnet, dass $R_3$ für Wasserstoff oder für jeweils substituier-

tes oder unsubstituiertes $C_1$-$C_{20}$-Alkyl, $C_3$-$C_7$-Cycloalkyl, $C_3$-$C_{20}$-Alkenyl, $C_3$-$C_{20}$-Alkinyl, Benzyl oder Aryl steht.

3. Mittel gemäss Anspruch 1, dadurch gekennzeichnet, dass $R_3$ Wasserstoff, $C_1$-$C_{20}$-Alkyl, $C_3$-$C_7$-Cycloalkyl, $C_3$-$C_{20}$-Alkenyl, $C_3$-$C_{20}$-Alkinyl, Aryl, $C_3$-$C_{20}$-Halogenalkenyl, $C_3$-$C_{20}$-Halogenalkinyl; durch Halogen oder $C_1$-$C_4$-Alkyl substituiertes $C_3$-$C_7$-Cycloalkyl; durch Halogen, $C_1$-$C_4$-Alkyl, $C_1$-$C_4$-Halogenalkyl, $C_1$-$C_{12}$-Alkoxy, $C_1$-$C_4$-Halogenalkoxy, $C_1$-$C_4$-Alkylthio, Nitro, Cyan, Benzoyl, Halogenbenzoyl, Phenoxy, Halogenphenoxy, $C_1$-$C_4$-Alkylphenoxy, $C_1$-$C_4$-Halogenalkylphenoxy, Tri-$C_1$-$C_4$-alkylsilyl, N-Pyrrolidinyl, N-Piperidinyl, N-Pyrrolidin-2-onyl, N-Piperidin-2-onyl, $C_1$-$C_4$-Alkylamino, Di-$C_1$-$C_4$-alkylamino, Anilino, N-$C_1$-$C_4$-Alkylanilino, N-Formylanilino, N-$C_1$-$C_6$-Alkylcarbonylanilino, Phenylthio oder Halogenphenylthio substituiertes Aryl; durch einen über Sauerstoff oder Schwefel gebundenen unsubstituierten oder substituierten aromatischen oder nichtaromatischen, mono- oder bicyclischen Heterocyclus substituiertes Phenyl, wobei sowohl der Heterocyclus als auch der Phenylring jeweils durch Halogen, $C_1$-$C_4$-Alkyl, Nitro, $C_1$-$C_4$-Halogenalkyl, $C_1$-$C_4$-Halogenalkoxy, $C_1$-$C_4$-Alkoxy, $C_1$-$C_4$-Alkylthio oder Cyclopropyl substituiert sein können; oder durch Hydroxy, Halogen, Di-$C_1$-$C_4$-alkylamino, $C_1$-$C_4$-Alkoxy, $C_1$-$C_4$-Halogenalkoxy, $C_2$-$C_6$-Alkoxyalkoxy, $C_1$-$C_4$-Halogenalkylthio, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy, $C_3$-$C_7$-Cycloalkyl, Aryl, Aryloxy, Arylthio, Arylsulfonyl, Arylsulfinyl, Arylsulfonyloxy, Arylcarbonyl oder Pyridyl substituiertes $C_1$-$C_{20}$-Alkyl steht; wobei die Aryl- und Pyridylgruppen jeweils durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Nitro, Cyan, Phenoxy, Halogenphenoxy, Phenylthio oder Halogenphenylthio substituiert sein können.

4. Mittel gemäss Anspruch 1, dadurch gekennzeichnet, dass $R_1$ und $R_2$ unabhängig voneinander Wasserstoff oder $C_1$-$C_4$-Alkyl, vorzugsweise jedoch Wasserstoff bedeuten.

5. Mittel gemäss Anspruch 1, dadurch gekennzeichnet, dass X Sauerstoff, -NH-, $-NCH_3-$ oder $-NC_2H_5-$, vorzugsweise jedoch Sauerstoff oder -NH- bedeutet.

6. Mittel gemäss Anspruch 3, dadurch gekennzeichnet, dass $R_3$ für durch in 4-Stellung durch einen über Sauerstoff gebundenen aromatischen mono- oder bicyclischen Heterocyclus aus der Reihe Pyridin, Pyrimidin oder Benzothioazol substituiertes Phenyl steht, wobei beide aromatischen Ringe unsubstituiert sind oder zusammen höchstens drei weitere Substituenten aus der Reihe Chlor, Brom, Methyl, Aethyl und Trifluormethyl tragen.

7. Mittel gemäss Anspruch 3, dadurch gekennzeichnet, dass $R_1$ und $R_2$ unabhängig voneinander Wasserstoff oder $C_1$-$C_4$-Alkyl, X Sauerstoff oder $-NR_4-$ und $R_4$ Wasserstoff, Methyl oder Aethyl bedeuten und $R_3$ für Wasserstoff, $C_1$-$C_{20}$-Alkyl, $C_3$-$C_7$-Cycloalkyl, $C_3$-$C_{20}$-Alkenyl, $C_3$-$C_{20}$-Alkinyl, Phenyl, Naphthyl, $C_3$-$C_{20}$-Halogenalkenyl, $C_3$-$C_{20}$-Halogenalkinyl, durch Fluor, Chlor, Brom oder $C_1$-$C_3$-Alkyl substituiertes $C_3$-$C_7$-Cycloalkyl, durch Fluor, Chlor, Brom oder $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, $C_1$-$C_4$-Alkylphenoxy, $C_1$-$C_4$-Halogenalkylphenoxy, Tri-$C_1$-$C_4$-alkylsilyl, N-Pyrrolidinyl, N-Piperidinyl, N-Pyrrolidin-2-onyl, N-Piperidin-2-onyl, $C_1$-$C_4$-Alkylamino, Di-$C_1$-$C_4$-alkylamino, Anilino, N-$C_1$-$C_4$-Alkylanilino, N-Formylanilino, N-$C_1$-$C_6$-Alkylcarbonylanilino, Phenylthio oder Halogenphenylthio substituiertes Phenyl oder Naphthyl, oder durch Hydroxy, Fluor, Chlor, Brom, Di-$C_1$-$C_4$-alkylamino, $C_1$-$C_4$-Alkoxy, $C_1$-$C_4$-Halogenalkoxy, $C_2$-$C_6$-Alkoxyalkoxy, $C_1$-$C_4$-Halogenalkylthio, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy, $C_3$-$C_7$-Cycloalkyl, Phenyl, Phenoxy, Phenylthio, Phenylsulfonyloxy oder Pyridyl substituiertes $C_1$-$C_{20}$-Alkyl steht, wobei die Phenyl- und Pyridylgruppen jeweils durch Fluor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, Phenylthio oder Halogenphenylthio substituiert sein können.

8. Mittel gemäss Anspruch 1, dadurch gekennzeichnet, dass $R_1$ und $R_2$ unabhängig voneinander Wasserstoff oder $C_1$-$C_4$-Alkyl, X Sauerstoff oder $-NR_4-$ , $R_4$ Wasserstoff, Methyl oder Aethyl, $R_3$ Phenyl, Benzyl, Naphthyl, 3-Pyridylmethyl, oder jeweils durch Fluor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, Phenylthio oder Halogenphenylthio substituiertes Phenyl, Benzyl, Naphthyl oder 3-Pyridylmethyl bedeuten.

9. Mittel gemäss Anspruch 1, dadurch gekennzeichnet, dass $R_1$ und $R_2$ unabhängig voneinander Wasserstoff oder $C_1$-$C_4$-Alkyl, X Sauerstoff oder $-NR_4-$, $R_4$ Wasserstoff oder $C_1$-$C_4$-Alkyl, $R_3$ $C_1$-$C_{12}$-Alkyl oder

durch Hydroxy, Fluor, Chlor, Brom, Dimethylamino, Methoxy, Aethoxy, Methoxyäthoxy, Aethoxyäthoxy, Methylthio, Aethylthio, Cyclopropyl, Cyclopentyl, Cyclohexyl, Phenyl oder Phenoxy substituiertes $C_1$-$C_{12}$-Alkyl bedeuten, wobei der Phenyl- oder Phenoxyrest jeweils durch Fluor, Chlor, Brom, Phenoxy, Halogenphenoxy oder Phenylthio substituiert sein kann.

**10.** Mittel gemäss Anspruch 1, dadurch gekennzeichnet, dass $R_1$ und $R_2$ unabhängig voneinander Wasserstoff oder $C_1$-$C_4$-Alkyl, X Sauerstoff oder -$NR_4$-, $R_4$ Wasserstoff oder $C_1$-$C_4$-Alkyl, $R_3$ $C_3$-$C_{12}$-Alkenyl, $C_3$-$C_{12}$-Alkinyl oder jeweils durch Fluor, Chlor oder Brom substituiertes $C_3$-$C_{12}$-Alkenyl oder $C_3$-$C_{12}$-Alkinyl bedeuten.

**11.** Mittel gemäss Anspruch 3, dadurch gekennzeichnet, dass $R_1$ und $R_2$ Wasserstoff, und X Sauerstoff, -NH-, -$NCH_3$- oder -$NC_2H_5$- bedeuten.

**12.** Mittel gemäss Anspruch 1 enthaltend einen Wirkstoff ausgewählt aus der Reihe
4-Chlor-4,4-difluorbuttersäure-methylester,
4-Chlor-4,4-difluorbuttersäure-äthylester,
4-Chlor-4,4-difluorbuttersäure-isopropylester,
4-Chlor-4,4-difluorbuttersäure-tert-butylester,
4-Chlor-4,4-difluorbuttersäure-n-butylester,
4-Chlor-4,4-difluorbuttersäure-(2,2-dimethylpropyl)-ester,
4-Chlor-4,4-difluorbuttersäure-benzylester,
4-Chlor-4,4-difluorbuttersäure-phenylester,
4-Chlor-4,4-difluorbuttersäure-[2-(4-phenoxyphenoxy)-äthyl]-ester,
4-Chlor-4,4-difluorbuttersäure-cyclohexylester,
4-Chlor-4,4-difluorbuttersäure-cyclohexylmethylester,
4-Chlor-4,4-difluorbuttersäure-cyclopropylmethylester,
4-Chlor-4,4-difluor-2-trifluormethylbuttersäure-äthylester,
4-Chlor-4,4-difluorbuttersäure-N-methylamid,
4-Chlor-4,4-difluorbuttersäure-N,N-dimethylamid,
4-Chlor-4,4-difluorbuttersäure-N,N-dihexylamid,
4-Chlor-4,4-difluorbuttersäure-N-äthylamid,
4-Chlor-4,4-difluorbuttersäure-N-isopropylamid
4-Chlor-4,4-difluorbuttersäure-N-butylamid,
4-Chlor-4,4-difluorbuttersäure-N-tert.butylamid,
4-Chlor-4,4-difluorbuttersäure-N-benzylamid,
4-Chlor-4,4-difluorbuttersäure-anilid,
4-Chlor-4,4-difluorbuttersäure--N-methyl-N-pyrid-3-ylmethylamid
4-Chlor-4,4-difluorbuttersäure-N-pyrid-3-ylmethylamid,
4-Chlor-4,4-difluorbuttersäure-(4-chlor-anilid),
4-Chlor-4,4-difluorbuttersäure-(4-phenoxy-anilid),
4-Chlor-4,4-difluorbuttersäure-(2-chlor-anilid),
4-Chlor-4,4-difluorbuttersäure-(4-methoxy-anilid),
4-Chlor-4,4-difluorbuttersäure-(4-methyl-anilid),
4-Chlor-4,4-difluorbuttersäure-(3-methylmercapto-anilid),
4-Chlor-4,4-difluorbuttersäure-(4-fluor-anilid),
4-Chlor-4,4-difluorbuttersäure-(4-chlor-2-nitro-anilid),
4-Chlor-4,4-difluorbuttersäure-(3-phenoxy-benzyl)-ester,
4-Chlor-4,4-difluorbuttersäure-[(4-fluor-phenoxy)-phenyl]-ester,
4-Chlor-4,4-difluorbuttersäure-[(4-fluor-phenoxy)-phenoxy-äthyl]-ester,
4-Chlor-4,4-difluorbuttersäure-(4-nitro-phenyl)-ester,
4-Chlor-4,4-difluorbuttersäure-[4-(3,5-difluor-phenoxy)-phenyl]-ester,
4-Chlor-4,4-difluorbuttersäure-[4-(5-trifluormethyl-pyrid-2-yloxy)-phenyl]-ester,
4-Chlor-4,4-difluorbuttersäure und
4-Chlor-4,4-difluorbuttersäureamid.

**13.** Verfahren zur Herstellung der Verbindungen der Formel I nach Anspruch 1, dadurch gekennzeichnet, dass man entweder
a) ein 4-Chlor-4,4-difluorbuttersäurehalogenid der Formel II

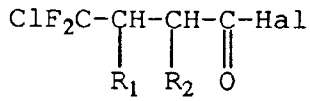 (II)

worin $R_1$ und $R_2$ die unter Formel I gegebenen Bedeutungen haben und Hal für Halogen, vorzugsweise Chlor oder Brom steht, in Gegenwart einer Base mit einer Verbindung der Formel III

$H\text{-}X\text{-}R_3$ (III)

worin X und $R_3$ die unter Formel I gegebenen Bedeutungen haben, umsetzt, oder

b) 4-Chlor-4,4-difluorbuttersäure der Formel Ic

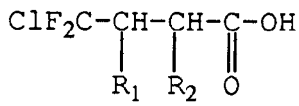 (Ic)

worin $R_1$ und $R_2$ die unter Formel I gegebenen Bedeutungen haben, in Gegenwart eines wasserabspaltenden Mittels mit einer Verbindung der Formel III umsetzt.

**14.** Mittel gemäss Anspruch 1, dadurch gekennzeichnet, dass es zusätzlich noch mindestens einen Träger enthält.

**15.** Verwendung einer Verbindung der Formel I nach Anspruch 1 zur Bekämpfung von Schädlingen an Tieren und Pflanzen.

**16.** Verwendung gemäss Anspruch 15, dadurch gekennzeichnet, dass es sich bei den Schädlingen um pflanzenschädigende Insekten und Arachniden handelt.

**17.** Verfahren zur Bekämpfung von tier- und pflanzenschädigenden Insekten und Arachniden, dadurch gekennzeichnet, dass man die Schädlinge oder ihren Lebensraum mit einer wirksamen Menge einer Verbindung der Formel I gemäss Anspruch 1 behandelt.

**18.** Verfahren zur Herstellung von 4-Chlor-4,4-difluorbuttersäurehalogeniden der Formel II

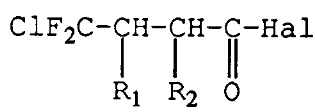 (II)

worin $R_1$ und $R_2$ unabhängig voneinander Wasserstoff, $C_1$-$C_4$-Alkyl oder $C_1$-$C_4$-Halogenalkyl bedeuten, und Hal für Halogen, vorzugsweise Chlor oder Brom, steht, dadurch gekennzeichnet, dass man eine 4-Chlor-4,4-difluorbuttersäure der Formel Ic

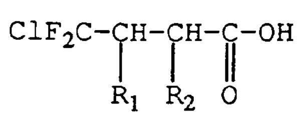 (Ic)

worin $R_1$ und $R_2$ die gegebenen Bedeutungen haben, mit einem Halogenierungsmittel umsetzt.

**19.** Verfahren zur Herstellung der 4-Chlor-4,4-difluorcrotonsäurederivate der Formel VIIIa

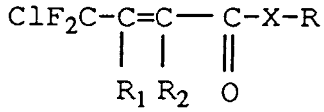 (VIII a),

worin
R $C_1$-$C_6$-Alkyl,
$R_1$ und $R_2$ jeweils Wasserstoff
und X Sauerstoff oder -$NR_4$- bedeuten,
wobei $R_4$ für Wasserstoff oder $C_1$-$C_6$-Alkyl steht, dadurch gekennzeichnet, dass man aus Verbindungen der Formel IX

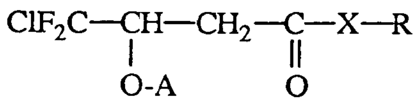 (IX),

worin A Wasserstoff oder eine Acylgruppe bedeutet, durch β-Elimination Wasser oder die Säure H-O-A abspaltet.

**20.** Verfahren zur Herstellung der Verbindungen der Formel I, dadurch gekennzeichnet, dass man ein 4-Chlor-4,4-difluorcrotonsäurederivat der Formel VIII

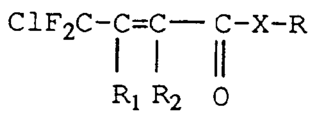 (VIII)

worin
R Wasserstoff oder $C_1$-$C_6$-Alkyl,
$R_1$ und $R_2$ unabhängig voneinander Wasserstoff, $C_1$-$C_4$-Alkyl oder $C_1$-$C_4$-Halogenalkyl,
und
X Sauerstoff oder -$NR_4$- bedeuten,
wobei $R_4$ für Wasserstoff oder $C_1$-$C_6$-Alkyl steht, mit Wasserstoff in Gegenwart eines Katalysators hydriert und die erhaltenen Verbindungen der Formel Id

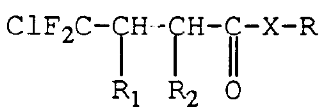 (Id)

worin $R_1$, $R_2$, X und R die oben gegebenen Bedeutungen haben, durch Hydrolyse, Umesterungsreaktion oder Umamidierungsreaktion in die übrigen Verbindungen der Formel I gemäss Anspruch 1 überführt.

## Claims

**Claims for the following Contracting States : AT, BE, CH, DE, DK, FR, GB, GR, IT, LI, LU, NL**

**1.** A 4-chloro-4,4-difluorobutyric acid derivative of formula I

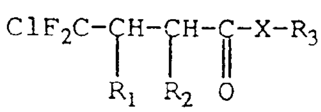 (I)

wherein
$R_1$ and $R_2$ independently of one another are hydrogen,
$C_1$-$C_4$alkyl or $C_1$-$C_4$haloalkyl,
$R_3$ is hydrogen or an organic radical and

X is oxygen or $-NR_4-$,
in which $R_4$ is hydrogen or $C_1$-$C_6$alkyl.

2. A compound according to claim 1, wherein $R_3$ is hydrogen or $C_1$-$C_{20}$alkyl, $C_3$-$C_7$cycloalkyl, $C_3$-$C_{20}$alkenyl, $C_3$-$C_{20}$alkynyl, benzyl or aryl, each of which is substituted or unsubstituted.

3. A compound according to claim 1, wherein $R_3$ is hydrogen, $C_1$-$C_{20}$alkyl, $C_3$-$C_7$cycloalkyl, $C_3$-$C_{20}$alkenyl, $C_3$-$C_{20}$alkynyl, aryl, $C_3$-$C_{20}$haloalkenyl, $C_3$-$C_{20}$haloalkynyl; $C_3$-$C_7$cycloalkyl substituted by halogen or by $C_1$-$C_4$alkyl; aryl substituted by halogen, $C_1$-$C_4$alkyl, $C_1$-$C_4$haloalkyl, $C_1$-$C_{12}$alkoxy, $C_1$-$C_4$haloalkoxy, $C_1$-$C_4$-alkylthio, nitro, cyano, benzoyl, halobenzoyl, phenoxy, halophenoxy, $C_1$-$C_4$alkylphenoxy, $C_1$-$C_4$haloalkyl-phenoxy, tri-$C_1$-$C_4$alkylsilyl, N-pyrrolidinyl, N-piperidinyl, N-pyrrolidin-2-onyl, N-piperidin-2-onyl, $C_1$-$C_4$alkylamino, di-$C_1$-$C_4$alkylamino, anilino, N-$C_1$-$C_4$alkylanilino, N-formylanilino, N-$C_1$-$C_6$alkylcarbonylanilino, phenylthio or by halophenylthio; phenyl substituted by an unsubstituted or substituted, aromatic or non-aromatic, monocyclic or bicyclic heterocycle that is bonded via oxygen or sulfur, in which both the heterocycle and the phenyl ring may each be substituted by halogen, $C_1$-$C_4$alkyl, nitro, $C_1$-$C_4$haloalkyl, $C_1$-$C_4$haloalkoxy, $C_1$-$C_4$alkoxy, $C_1$-$C_4$alkylthio or by cyclopropyl; or $C_1$-$C_{20}$alkyl substituted by hydroxy, halogen, di-$C_1$-$C_4$alkylamino, $C_1$-$C_4$alkoxy, $C_1$-$C_4$haloalkoxy, $C_2$-$C_6$alkoxyalkoxy, $C_1$-$C_4$haloalkylthio, $C_1$-$C_4$alkylthio, $C_1$-$C_4$alkylsulfinyl, $C_1$-$C_4$alkylsulfonyl, $C_1$-$C_4$alkylsulfonyloxy, $C_1$-$C_4$alkylcarbonyl, $C_1$-$C_4$alkoxycarbonyl, $C_1$-$C_6$alkylcarbonyloxy, $C_3$-$C_7$cycloalkyl, aryl, aryloxy, arylthio, arylsulfonyl, arylsulfinyl, arylsulfonyloxy, arylcarbonyl or by pyridyl, in which the aryl and pyridyl groups may each be substituted by halogen, $C_1$-$C_4$alkyl, $C_1$-$C_4$haloalkyl, $C_1$-$C_4$alkoxy, $C_1$-$C_4$haloalkoxy, $C_1$-$C_4$alkylthio, nitro, cyano, phenoxy, halophenoxy, phenylthio or by halophenylthio.

4. A compound according to claim 1, wherein $R_1$ and $R_2$ independently of one another are hydrogen or $C_1$-$C_4$alkyl, but preferably hydrogen.

5. A compound according to claim 1, wherein X is oxygen, -NH-, $-NCH_3-$ or $-NC_2H_5-$, but preferably oxygen or -NH-.

6. A compound according to claim 3, wherein $R_3$ is phenyl substituted in the 4-position by an aromatic monocyclic or bicyclic heterocycle bonded via oxygen and selected from the group pyridine, pyrimidine and benzothiazole, in which both aromatic rings are unsubstituted or together carry not more than three further substituents from the group chlorine, bromine, methyl, ethyl and trifluoromethyl.

7. A compound according to claim 3, wherein $R_1$ and $R_2$ independently of one another are hydrogen or $C_1$-$C_4$alkyl, X is oxygen or $-NR_4-$ and $R_4$ is hydrogen, methyl or ethyl and $R_3$ is hydrogen, $C_1$-$C_{20}$alkyl, $C_3$-$C_7$cycloalkyl, $C_3$-$C_{20}$alkenyl, $C_3$-$C_{20}$alkynyl, phenyl, naphthyl, $C_3$-$C_{20}$haloalkenyl, $C_3$-$C_{20}$haloalkynyl, $C_3$-$C_7$cycloalkyl substituted by fluorine, chlorine, bromine or by $C_1$-$C_3$alkyl, phenyl or naphthyl substituted by fluorine, chlorine, bromine or $C_1$-$C_3$alkyl, $C_1$-$C_3$haloalkyl, $C_1$-$C_3$alkoxy, $C_1$-$C_3$haloalkoxy, $C_1$-$C_3$alkylthio, nitro, cyano, phenoxy, halophenoxy, $C_1$-$C_4$alkylphenoxy, $C_1$-$C_4$haloalkylphenoxy, tri-$C_1$-$C_4$alkylsilyl, N-pyrrolidinyl, N-piperidinyl, N-pyrrolidin-2-onyl, N-piperidin-2-onyl, $C_1$-$C_4$alkylamino, di-$C_1$-$C_4$alkylamino, anilino, N-$C_1$-$C_4$alkylanilino, N-formylanilino, N-$C_1$-$C_6$alkylcarbonylanilino, phenylthio or by halophenylthio, or $C_1$-$C_{20}$alkyl substituted by hydroxy, fluorine, chlorine, bromine, di-$C_1$-$C_4$alkylamino, $C_1$-$C_4$-alkoxy, $C_1$-$C_4$haloalkoxy, $C_2$-$C_6$alkoxyalkoxy, $C_1$-$C_4$haloalkylthio, $C_1$-$C_4$alkylthio, $C_1$-$C_4$alkylsulfinyl, $C_1$-$C_4$alkylsulfonyl, $C_1$-$C_4$alkylsulfonyloxy, $C_1$-$C_4$alkylcarbonyl, $C_1$-$C_4$alkoxycarbonyl, $C_1$-$C_6$alkylcarbonyloxy, $C_3$-$C_7$cycloalkyl, phenyl, phenoxy, phenylthio, phenylsulfonyloxy or by pyridyl, in which the phenyl and pyridyl groups may each be substituted by fluorine, chlorine, bromine, $C_1$-$C_3$alkyl, $C_1$-$C_3$haloalkyl, $C_1$-$C_3$alkoxy, $C_1$-$C_3$haloalkoxy, $C_1$-$C_3$alkylthio, nitro, cyano, phenoxy, halophenoxy, phenylthio or by halophenylthio.

8. A compound according to claim 1, wherein $R_1$ and $R_2$ independently of one another are hydrogen or $C_1$-$C_4$alkyl, X is oxygen or $-NR_4-$, $R_4$ is hydrogen, methyl or ethyl, $R_3$ is phenyl, benzyl, naphthyl or 3-pyridylmethyl, or phenyl, benzyl, naphthyl or 3-pyridylmethyl each of which is substituted by fluorine, chlorine, bromine, $C_1$-$C_3$alkyl, $C_1$-$C_3$haloalkyl, $C_1$-$C_3$alkoxy, $C_1$-$C_3$haloalkoxy, $C_1$-$C_3$alkylthio, nitro, cyano, phenoxy, halophenoxy, phenylthio or by halophenylthio.

9. A compound according to claim 1, wherein $R_1$ and $R_2$ independently of one another are hydrogen or $C_1$-$C_4$alkyl, X is oxygen or $-NR_4-$, $R_4$ is hydrogen or $C_1$-$C_4$alkyl, $R_3$ is $C_1$-$C_{12}$alkyl or $C_1$-$C_{12}$alkyl substituted by hydroxy, fluorine, chlorine, bromine, dimethylamino, methoxy, ethoxy, methoxyethoxy, ethoxyethoxy, methylthio, ethylthio, cyclopropyl, cyclopentyl, cyclohexyl, phenyl or by phenoxy, in which the phenyl or

phenoxy radical may be substituted by fluorine, chlorine, bromine, phenoxy, halophenoxy or by phenylthio.

**10.** A compound according to claim 1, wherein $R_1$ and $R_2$ independently of one another are hydrogen or $C_1$-$C_4$alkyl, X is oxygen or $-NR_4-$, $R_4$ is hydrogen or $C_1$-$C_4$alkyl, $R_3$ is $C_3$-$C_{12}$alkenyl or $C_3$-$C_{12}$alkynyl, or $C_3$-$C_{12}$alkenyl or $C_3$-$C_{12}$alkynyl each of which is substituted by fluorine, chlorine or by bromine.

**11.** A compound according to claim 3, wherein $R_1$ and $R_2$ are hydrogen and X is oxygen, -NH-, $-NCH_3-$ or $-NC_2H_5-$.

**12.** A compound according to claim 1, selected from the group
4-chloro-4,4-difluorobutyric acid methyl ester,
4-chloro-4,4-difluorobutyric acid ethyl ester,
4-chloro-4,4-difluorobutyric acid isopropyl ester,
4-chloro-4,4-difluorobutyric acid tert-butyl ester,
4-chloro-4,4-difluorobutyric acid n-butyl ester,
4-chloro-4,4-difluorobutyric acid (2,2-dimethylpropyl) ester,
4-chloro-4,4-difluorobutyric acid benzyl ester,
4-chloro-4,4-difluorobutyric acid phenyl ester,
4-chloro-4,4-difluorobutyric acid [2-(4-phenoxyphenoxy)ethyl] ester,
4-chloro-4,4-difluorobutyric acid cyclohexyl ester,
4-chloro-4,4-difluorobutyric acid cyclohexylmethyl ester,
4-chloro-4,4-difluorobutyric acid cyclopropylmethyl ester,
4-chloro-4,4-difluoro-2-trifluoromethylbutyric acid ethyl ester,
4-chloro-4,4-difluorobutyric acid N-methylamide,
4-chloro-4,4-difluorobutyric acid N,N-dimethylamide,
4-chloro-4,4-difluorobutyric acid N,N-dihexylamide,
4-chloro-4,4-difluorobutyric acid N-ethylamide,
4-chloro-4,4-difluorobutyric acid N-isopropylamide,
4-chloro-4,4-difluorobutyric acid N-butylamide,
4-chloro-4,4-difluorobutyric acid N-tert-butylamide,
4-chloro-4,4-difluorobutyric acid N-benzylamide,
4-chloro-4,4-difluorobutyric acid anilide,
4-chloro-4,4-difluorobutyric acid N-methyl-N-pyrid-3-ylmethylamide,
4-chloro-4,4-difluorobutyric acid N-pyrid-3-ylmethylamide,
4-chloro-4,4-difluorobutyric acid (4-chloroanilide),
4-chloro-4,4-difluorobutyric acid (4-phenoxyanilide),
4-chloro-4,4-difluorobutyric acid (2-chloroanilide),
4-chloro-4,4-difluorobutyric acid (4-methoxyanilide),
4-chloro-4,4-difluorobutyric acid (4-methylanilide),
4-chloro-4,4-difluorobutyric acid (3-methylmercaptoanilide),
4-chloro-4,4-difluorobutyric acid (4-fluoroanilide),
4-chloro-4,4-difluorobutyric acid (4-chloro-2-nitroanilide),
4-chloro-4,4-difluorobutyric acid (3-phenoxybenzyl)ester,
4-chloro-4,4-difluorobutyric acid [4-(4-fluorophenoxy)phenyl] ester,
4-chloro-4,4-difluorobutyric acid [4-(4-fluorophenoxy)phenoxyethyl] ester,
4-chloro-4,4-difluorobutyric acid (4-nitrophenyl) ester,
4-chloro-4,4-difluorobutyric acid [4-(3,5-difluorophenoxy)-phenyl] ester,
4-chloro-4,4-difluorobutyric acid [4-(5-trifluoromethylpyrid-2-yloxy)-phenyl] ester,
4-chloro-4,4-difluorobutyric acid and
4-chloro-4,4-difluorobutyric acid amide.

**13.** A process for the preparation of a compound of formula I according to claim 1, which comprises either
a) reacting a 4-chloro-4,4-difluorobutyric acid halide of formula II

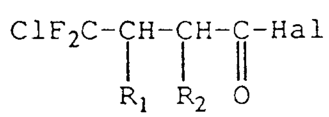

(II),

wherein $R_1$ and $R_2$ are as defined under formula I and Hal is halogen, preferably chlorine or bromine, with a compound of formula III

$$H\text{-}X\text{-}R_3 \qquad (III),$$

wherein X and $R_3$ are as defined under formula I, in the presence of a base, or
b) reacting 4-chloro-4,4-difluorobutyric acid of formula Ic

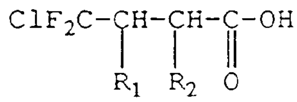 (Ic),

wherein $R_1$ and $R_2$ are as defined under formula I, with a compound of formula III in the presence of a water-removing agent.

**14.** A pesticidal composition, which comprises as active ingredient at least one compound of formula I according to claim 1.

**15.** A composition according to claim 14, which comprises in addition at least one carrier.

**16.** The use of a compound of formula I according to claim 1 for controlling pests on animals and plants.

**17.** The use according to claim 16, wherein the pests are plant-destructive insects and arachnids.

**18.** A method of controlling insects and arachnids that are harmful to animals and plants, which comprises treating the pests or the locus thereof with an effective amount of a compound of formula I according to claim 1.

**19.** A 4-chloro-4,4-difluorobutyric acid halide of formula II

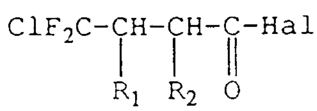 (II),

wherein $R_1$ and $R_2$ independently of one another are hydrogen, $C_1$-$C_4$alkyl or $C_1$-$C_4$haloalkyl, and Hal is halogen, preferably chlorine or bromine.

**20.** A 4-chloro-4,4-difluorocrotonic acid derivative of formula VIII

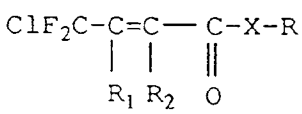 (VIII),

wherein
R is $C_1$-$C_6$alkyl,
$R_1$ and $R_2$ independently of one another are hydrogen,
$C_1$-$C_4$alkyl or $C_1$-$C_4$haloalkyl and
X is oxygen or $-NR_4-$,
in which $R_4$ is hydrogen or $C_1$-$C_6$alkyl,
with the proviso that in a compound of formula VIII wherein X is oxygen and R is ethyl, $R_2$ is other than hydrogen when $R_1$ is $CF_2H$, $CF_2Cl$ or $CF_3$.

**21.** A process for the preparation of a compound of formula I, which comprises hydrogenating a 4-chloro-4,4-difluorocrotonic acid derivative of formula VIII

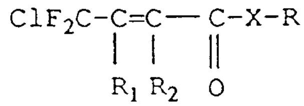

(VIII),

wherein
R is hydrogen or $C_1$-$C_6$alkyl,
$R_1$ and $R_2$ independently of one another are hydrogen,
$C_1$-$C_4$alkyl or $C_1$-$C_4$haloalkyl
and
X is oxygen or $-NR_4-$,
in which $R_4$ is hydrogen or $C_1$-$C_6$alkyl,
with hydrogen in the presence of a catalyst and converting the resulting compound of formula Id

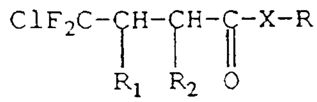

(Id),

wherein $R_1$, $R_2$, X and R are as defined above, into a different compound of formula I according to claim 1 by hydrolysis, transesterification or transamidation.

**Claims for the following Contracting State : ES**

1. A pesticidal composition, which comprises as active ingredient at least one 4-chloro-4,4-difluorobutyric acid derivative of formula I

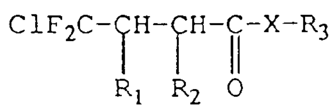

(I)

wherein
$R_1$ and $R_2$ independently of one another are hydrogen,
$C_1$-$C_4$alkyl or $C_1$-$C_4$haloalkyl,
$R_3$ is hydrogen or an organic radical and
X is oxygen or $-NR_4-$,
in which $R_4$ is hydrogen or $C_1$-$C_6$alkyl.

2. A composition according to claim 1, wherein $R_3$ is hydrogen or $C_1$-$C_{20}$alkyl, $C_3$-$C_7$cycloalkyl, $C_3$-$C_{20}$alkenyl, $C_3$-$C_{20}$alkynyl, benzyl or aryl, each of which is substituted or unsubstituted.

3. A composition according to claim 1, wherein $R_3$ is hydrogen, $C_1$-$C_{20}$alkyl, $C_3$-$C_7$cycloalkyl, $C_3$-$C_{20}$alkenyl, $C_3$-$C_{20}$alkynyl, aryl, $C_3$-$C_{20}$haloalkenyl, $C_3$-$C_{20}$haloalkynyl; $C_3$-$C_7$cycloalkyl substituted by halogen or by $C_1$-$C_4$alkyl; aryl substituted by halogen, $C_1$-$C_4$alkyl, $C_1$-$C_4$haloalkyl, $C_1$-$C_{12}$alkoxy, $C_1$-$C_4$haloalkoxy, $C_1$-$C_4$alkylthio, nitro, cyano, benzoyl, halobenzoyl, phenoxy, halophenoxy, $C_1$-$C_4$alkylphenoxy, $C_1$-$C_4$haloalkylphenoxy, tri-$C_1$-$C_4$alkylsilyl, N-pyrrolidinyl, N-piperidinyl, N-pyrrolidin-2-onyl, N-piperidin-2-onyl, $C_1$-$C_4$alkylamino, di-$C_1$-$C_4$alkylamino, anilino, N-$C_1$-$C_4$alkylanilino, N-formylanilino, N-$C_1$-$C_6$alkylcarbonylanilino, phenylthio or by halophenylthio; phenyl substituted by an unsubstituted or substituted, aromatic or non-aromatic, monocyclic or bicyclic heterocycle that is bonded via oxygen or sulfur, in which both the heterocycle and the phenyl ring may each be substituted by halogen, $C_1$-$C_4$alkyl, nitro, $C_1$-$C_4$haloalkyl, $C_1$-$C_4$haloalkoxy, $C_1$-$C_4$alkoxy, $C_1$-$C_4$alkylthio or by cyclopropyl; or $C_1$-$C_{20}$alkyl substituted by hydroxy, halogen, di-$C_1$-$C_4$alkylamino, $C_1$-$C_4$alkoxy, $C_1$-$C_4$haloalkoxy, $C_2$-$C_6$alkoxyalkoxy, $C_1$-$C_4$haloalkylthio, $C_1$-$C_4$alkylthio, $C_1$-$C_4$alkylsulfinyl, $C_1$-$C_4$alkylsulfonyl, $C_1$-$C_4$alkylsulfonyloxy, $C_1$-$C_4$alkylcarbonyl, $C_1$-$C_4$alkoxycarbonyl, $C_1$-$C_6$alkylcarbonyloxy, $C_3$-$C_7$cycloalkyl, aryl, aryloxy, arylthio, arylsulfonyl, arylsulfinyl, arylsulfonyloxy, arylcarbonyl or by pyridyl, in which the aryl and pyridyl groups may each be substituted by halogen, $C_1$-$C_4$alkyl, $C_1$-$C_4$haloalkyl, $C_1$-$C_4$alkoxy, $C_1$-$C_4$haloalkoxy, $C_1$-$C_4$alkylthio, nitro, cyano, phenoxy, halophenoxy, phenylthio or by halophenylthio.

4. A composition according to claim 1, wherein $R_1$ and $R_2$ independently of one another are hydrogen or $C_1$-$C_4$alkyl, but preferably hydrogen.

5. A composition according to claim 1, wherein X is oxygen, -NH-, -$NCH_3$- or -$NC_2H_5$-, but preferably oxygen or -NH-.

6. A composition according to claim 3, wherein $R_3$ is phenyl substituted in the 4-position by an aromatic monocyclic or bicyclic heterocycle bonded via oxygen and selected from the group pyridine, pyrimidine and benzothiazole, in which both aromatic rings are unsubstituted or together carry not more than three further substituents from the group chlorine, bromine, methyl, ethyl and trifluoromethyl.

7. A composition according to claim 3, wherein $R_1$ and $R_2$ independently of one another are hydrogen or $C_1$-$C_4$alkyl, X is oxygen or -$NR_4$- and $R_4$ is hydrogen, methyl or ethyl and $R_3$ is hydrogen, $C_1$-$C_{20}$alkyl, $C_3$-$C_7$cycloalkyl, $C_3$-$C_{20}$alkenyl, $C_3$-$C_{20}$alkynyl, phenyl, naphthyl, $C_3$-$C_{20}$haloalkenyl, $C_3$-$C_{20}$haloalkynyl, $C_3$-$C_7$cycloalkyl substituted by fluorine, chlorine, bromine or by $C_1$-$C_3$alkyl, phenyl or naphthyl substituted by fluorine, chlorine, bromine or $C_1$-$C_3$alkyl, $C_1$-$C_3$haloalkyl, $C_1$-$C_3$alkoxy, $C_1$-$C_3$haloalkoxy, $C_1$-$C_3$alkylthio, nitro, cyano, phenoxy, halophenoxy, $C_1$-$C_4$alkylphenoxy, $C_1$-$C_4$haloalkylphenoxy, tri-$C_1$-$C_4$alkylsilyl, N-pyrrolidinyl, N-piperidinyl, N-pyrrolidin-2-onyl, N-piperidin-2-onyl, $C_1$-$C_4$alkylamino, di-$C_1$-$C_4$alkylamino, anilino, N-$C_1$-$C_4$alkylanilino, N-formylanilino, N-$C_1$-$C_6$alkylcarbonylanilino, phenylthio or by halophenylthio, or $C_1$-$C_{20}$alkyl substituted by hydroxy, fluorine, chlorine, bromine, di-$C_1$-$C_4$alkylamino, $C_1$-$C_4$alkoxy, $C_1$-$C_4$haloalkoxy, $C_2$-$C_6$alkoxyalkoxy, $C_1$-$C_4$haloalkylthio, $C_1$-$C_4$alkylthio, $C_1$-$C_4$alkylsulfinyl, $C_1$-$C_4$alkylsulfonyl, $C_1$-$C_4$alkylsulfonyloxy, $C_1$-$C_4$alkylcarbonyl, $C_1$-$C_4$alkoxycarbonyl, $C_1$-$C_6$alkylcarbonyloxy, $C_3$-$C_7$cycloalkyl, phenyl, phenoxy, phenylthio, phenylsulfonyloxy or by pyridyl, in which the phenyl and pyridyl groups may each be substituted by fluorine, chlorine, bromine, $C_1$-$C_3$alkyl, $C_1$-$C_3$haloalkyl, $C_1$-$C_3$alkoxy, $C_1$-$C_3$haloalkoxy, $C_1$-$C_3$alkylthio, nitro, cyano, phenoxy, halophenoxy, phenylthio or by halophenylthio.

8. A composition according to claim 1, wherein $R_1$ and $R_2$ independently of one another are hydrogen or $C_1$-$C_4$alkyl, X is oxygen or -$NR_4$-, $R_4$ is hydrogen, methyl or ethyl, $R_3$ is phenyl, benzyl, naphthyl or 3-pyridylmethyl, or phenyl, benzyl, naphthyl or 3-pyridylmethyl each of which is substituted by fluorine, chlorine, bromine, $C_1$-$C_3$alkyl, $C_1$-$C_3$haloalkyl, $C_1$-$C_3$alkoxy, $C_1$-$C_3$haloalkoxy, $C_1$-$C_3$alkylthio, nitro, cyano, phenoxy, halophenoxy, phenylthio or by halophenylthio.

9. A composition according to claim 1, wherein $R_1$ and $R_2$ independently of one another are hydrogen or $C_1$-$C_4$alkyl, X is oxygen or -$NR_4$-, $R_4$ is hydrogen or $C_1$-$C_4$alkyl, $R_3$ is $C_1$-$C_{12}$alkyl or $C_1$-$C_{12}$alkyl substituted by hydroxy, fluorine, chlorine, bromine, dimethylamino, methoxy, ethoxy, methoxyethoxy, ethoxyethoxy, methylthio, ethylthio, cyclopropyl, cyclopentyl, cyclohexyl, phenyl or by phenoxy, in which the phenyl or phenoxy radical may be substituted by fluorine, chlorine, bromine, phenoxy, halophenoxy or by phenylthio.

10. A composition according to claim 1, wherein $R_1$ and $R_2$ independently of one another are hydrogen or $C_1$-$C_4$alkyl, X is oxygen or -$NR_4$-, $R_4$ is hydrogen or $C_1$-$C_4$alkyl, $R_3$ is $C_3$-$C_{12}$alkenyl or $C_3$-$C_{12}$alkynyl, or $C_3$-$C_{12}$alkenyl or $C_3$-$C_{12}$alkynyl each of which is substituted by fluorine, chlorine or by bromine.

11. A composition according to claim 3, wherein $R_1$ and $R_2$ are hydrogen and X is oxygen, -NH-, -$NCH_3$- or -$NC_2H_5$-.

12. A composition according to claim 1, comprising an active ingredient selected from the group
4-chloro-4,4-difluorobutyric acid methyl ester,
4-chloro-4,4-difluorobutyric acid ethyl ester,
4-chloro-4,4-difluorobutyric acid isopropyl ester,
4-chloro-4,4-difluorobutyric acid tert-butyl ester,
4-chloro-4,4-difluorobutyric acid n-butyl ester,
4-chloro-4,4-difluorobutyric acid (2,2-dimethylpropyl) ester,
4-chloro-4,4-difluorobutyric acid benzyl ester,
4-chloro-4,4-difluorobutyric acid phenyl ester,
4-chloro-4,4-difluorobutyric acid [2-(4-phenoxyphenoxy)ethyl] ester,
4-chloro-4,4-difluorobutyric acid cyclohexyl ester,
4-chloro-4,4-difluorobutyric acid cyclohexylmethyl ester,
4-chloro-4,4-difluorobutyric acid cyclopropylmethyl ester,
4-chloro-4,4-difluoro-2-trifluoromethylbutyric acid ethyl ester,
4-chloro-4,4-difluorobutyric acid N-methylamide,

4-chloro-4,4-difluorobutyric acid N,N-dimethylamide,
4-chloro-4,4-difluorobutyric acid N,N-dihexylamide,
4-chloro-4,4-difluorobutyric acid N-ethylamide,
4-chloro-4,4-difluorobutyric acid N-isopropylamide,
4-chloro-4,4-difluorobutyric acid N-butylamide,
4-chloro-4,4-difluorobutyric acid N-tert-butylamide,
4-chloro-4,4-difluorobutyric acid N-benzylamide,
4-chloro-4,4-difluorobutyric acid anilide,
4-chloro-4,4-difluorobutyric acid N-methyl-N-pyrid-3-ylmethylamide,
4-chloro-4,4-difluorobutyric acid N-pyrid-3-ylmethylamide,
4-chloro-4,4-difluorobutyric acid (4-chloroanilide),
4-chloro-4,4-difluorobutyric acid (4-phenoxyanilide),
4-chloro-4,4-difluorobutyric acid (2-chloroanilide),
4-chloro-4,4-difluorobutyric acid (4-methoxyanilide),
4-chloro-4,4-difluorobutyric acid (4-methylanilide),
4-chloro-4,4-difluorobutyric acid (3-methylmercaptoanilide),
4-chloro-4,4-difluorobutyric acid (4-fluoroanilide),
4-chloro-4,4-difluorobutyric acid (4-chloro-2-nitroanilide),
4-chloro-4,4-difluorobutyric acid (3-phenoxybenzyl) ester,
4-chloro-4,4-difluorobutyric acid [4-(4-fluorophenoxy)phenyl] ester,
4-chloro-4,4-difluorobutyric acid [4-(4-fluorophenoxy)phenoxyethyl] ester,
4-chloro-4,4-difluorobutyric acid (4-nitrophenyl) ester,
4-chloro-4,4-difluorobutyric acid [4-(3,5-difluorophenoxy)-phenyl] ester,
4-chloro-4,4-difluorobutyric acid [4-(5-trifluoromethylpyrid-2-yloxy)-phenyl] ester,
4-chloro-4,4-difluorobutyric acid and
4-chloro-4,4-difluorobutyric acid amide.

**13.** A process for the preparation of a compound of formula I according to claim 1, which comprises either
a) reacting a 4-chloro-4,4-difluorobutyric acid halide of formula II

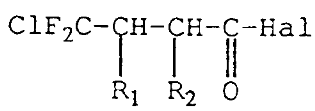 (II),

wherein $R_1$ and $R_2$ are as defined under formula I and Hal is halogen, preferably chlorine or bromine, with a compound of formula III

$H-X-R_3$ (III),

wherein X and $R_3$ are as defined under formula I, in the presence of a base, or
b) reacting 4-chloro-4,4-difluorobutyric acid of formula Ic

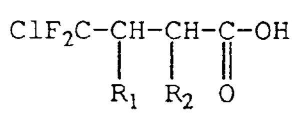 (Ic),

wherein $R_1$ and $R_2$ are as defined under formula I, with a compound of formula III in the presence of a water-removing agent.

**14.** A composition according to claim 1, which comprises in addition at least one carrier.

**15.** The use of a compound of formula I according to claim 1 for controlling pests on animals and plants.

**16.** The use according to claim 15, wherein the pests are plant-destructive insects and arachnids.

**17.** A method of controlling insects and arachnids that are harmful to animals and plants, which comprises treating the pests or the locus thereof with an effective amount of a compound of formula I according to claim 1.

**18.** A process for the preparation of a 4-chloro-4,4-difluorobutyric acid halide of formula II

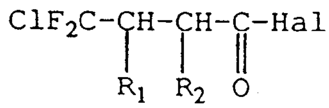

(II),

wherein $R_1$ and $R_2$ independently of one another are hydrogen, $C_1$-$C_4$alkyl or $C_1$-$C_4$haloalkyl, and Hal is halogen, preferably chlorine or bromine, which comprises reacting a 4-chloro-4,4-difluorobutyric acid of formula Ic

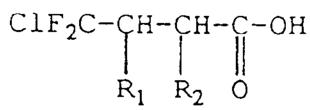

(Ic),

wherein $R_1$ and $R_2$ are as defined, with a halogenating agent.

**19.** A process for the preparation of a 4-chloro-4,4-difluorocrotonic acid derivative of formula VIIIa

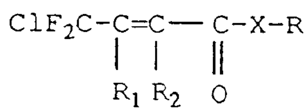

(VIIIa),

wherein
R is $C_1$-$C_6$alkyl,
$R_1$ and $R_2$ are each hydrogen and
X is oxygen or $-NR_4-$,
in which $R_4$ is hydrogen or $C_1$-$C_6$alkyl,
which comprises removing water or the acid H-O-A from a compound of formula IX

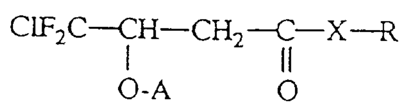

(IX),

wherein A is hydrogen or an acyl group, by β-elimination.

**20.** A process for the preparation of a compound of formula I, which comprises hydrogenating a 4-chloro-4,4-difluorocrotonic acid derivative of formula VIII

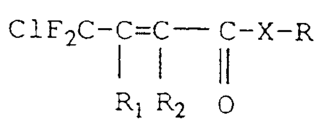

(VIII),

wherein
R is hydrogen or $C_1$-$C_6$alkyl,
$R_1$ and $R_2$ independently of one another are hydrogen,
$C_1$-$C_4$alkyl or $C_1$-$C_4$haloalkyl
and
X is oxygen or $-NR_4-$,
in which $R_4$ is hydrogen or $C_1$-$C_6$alkyl,
with hydrogen in the presence of a catalyst and converting the resulting compound of formula Id

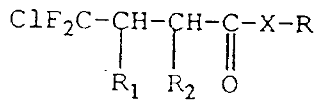 (Id),

wherein $R_1$, $R_2$, X and R are as defined above, into a different compound of formula I according to claim 1 by hydrolysis, transesterification or transamidation.

## Revendications

### Revendications pour les Etats contractants suivants : AT, BE, CH, DE, DK, FR, GB, GR, IT, LI, LU, NL

1. Dérivés de l'acide 4-chloro-4,4-difluorobutyrique de formule I

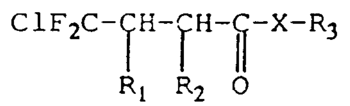 (I)

dans laquelle
$R_1$ et $R_2$, indépendamment l'un de l'autre, sont chacun un hydrogène ou un radical alkyle en $C_1$ à $C_4$ ou halogènalkyle en $C_1$ à $C_4$,
$R_3$ est un hydrogène ou un radical organique, et
X est un oxygène ou $-NR_4-$,
$R_4$ étant un hydrogène ou un radical alkyle en $C_1$ à $C_6$.

2. Composés selon la revendication 1, caractérisés en ce que $R_3$ est un hydrogène ou un radical éventuellement substitué alkyle en $C_1$ à $C_{20}$, cycloalkyle en $C_3$ à $C_7$, alcényle en $C_3$ à $C_{20}$, alcynyle en $C_3$ à $C_{20}$, benzyle ou aryle.

3. Composés selon la revendication 1, caractérisés en ce que $R_3$ est un hydrogène, un radical alkyle en $C_1$ à $C_{20}$, cycloalkyle en $C_3$ à $C_7$, alcényle en $C_3$ à $C_{20}$, alcynyle en $C_3$ à $C_{20}$, aryle, halogénalcényle en $C_3$ à $C_{20}$, halogénalcynyle en $C_3$ à $C_{20}$ ; cycloalkyle en $C_3$ à $C_7$ substitué par des substituants halogéno ou alkyle en $C_1$ à $C_4$ ; aryle substitué par des substituants halogénoalkyle en $C_1$ à $C_4$, halogénalkyle en $C_1$ à $C_4$, alcoxy en $C_1$ à $C_{12}$, halogénalcoxy en $C_1$ à $C_4$, alkylthio en $C_1$ à $C_4$, nitro, cyano, benzoyle, halogénobenzoyle, phénoxy, halogénophénoxy, (alkyle en $C_1$ à $C_4$)phénoxy, (halogénalkyle en $C_1$ à $C_4$)phénoxy, tri-(alkyle en $C_1$ à $C_4$)silyle, N-pyrrolidinyle, N-pipéridinyle, N-pyrrolidine-2-onyle, N-pipéridine-2-onyle, alkylamino en $C_1$ à $C_4$, di-(alkyle en $C_1$ à $C_4$)amino, anilino, N-(alkyle en $C_1$ à $C_4$)anilino, N-formylanilino, N-(alkyle en $C_1$ à $C_6$)carbonylanilino, phénylthio ou halogénophénylthio ; phényle substitué par un hétérocycle mono- ou dicyclique, aromatique ou non aromatique, éventuellement substitué, lié par l'intermédiaire d'un atome d'oxygène ou de soufre, auquel cas l'hétérocycle, tout le comme le noyau phényle, peuvent être chacun substitués par des substituants halogéno, alkyle en C1 à $C_4$, nitro, halogénalkyle en $C_1$ à $C_4$, halogénalcoxy en $C_1$ à $C_4$, alcoxy en $C_1$ à $C_4$, alkylthio en $C_1$ à $C_4$ ou cyclopropyle ; ou encore alkyle en $C_1$ à $C_{20}$ substitué par des substituants hydroxy, halogéno, di-(alkyle en $C_1$ à $C_4$)amino, alcoxy en $C_1$ à $C_4$, halogénalcoxy en $C_1$ à $C_4$, alcoxyalcoxy en $C_2$ à $C_6$, halogénalkylthio en $C_1$ à $C_4$, alkylthio en $C_1$ à $C_4$, alkylsulfinyle en $C_1$ à $C_4$, alkylsulfonyle en $C_1$ à $C_4$, alkylsulfonyloxy en $C_1$ à $C_4$, (alkyle en $C_1$ à $C_4$)carbonyle, (alcoxy en $C_1$ à $C_4$)carbonyle, (alkyle en $C_1$ à $C_6$)carbonyloxy, cycloalkyle en $C_3$ à $C_7$, aryle, aryloxy, arylthio, arylsulfonyle, arylsulfinyle, arylsulfonyloxy, arylcarbonyle ou pyridyle ; les groupes aryle et pyridyle pouvant chacun être substitués par des substituants halogéno, alkyle en $C_1$ à $C_4$, halogénalkyle en $C_1$ à $C_4$, alcoxy en $C_1$ à $C_4$, halogénalcoxy en $C_1$ à $C_4$, alkylthio en $C_1$ à $C_4$, nitro, cyano, phénoxy, halogénophénoxy, phénylthio ou halogénophénylthio.

4. Composés selon la revendication 1, caractérisés en ce que $R_1$ et $R_2$, indépendamment l'un de l'autre, sont chacun un hydrogène ou un radical alkyle en $C_1$ à $C_4$, mais de préférence un hydrogène.

**5.** Composés selon la revendication 1, caractérisés en ce que X est un oxygène, -NH-, -$NCH_3$- ou -$NC_2H_5$-, mais de préférence un oxygène ou -NH-.

**6.** Composés selon la revendication 3, caractérisés en ce que $R_3$ est un radical phényle substitué en position 4 par un hétérocycle mono- ou bicyclique aromatique, lié par l'intermédiaire d'un oxygène, et choisi parmi la pyridine, la pyrimidine ou le benzothiazole, les deux noyaux aromatiques étant non substitués, ou portant ensemble au plus trois substituants supplémentaires choisis parmi le chlore, le brome, et les radicaux méthylo, éthyle et trifluorométhyle.

**7.** Composés selon la revendication 3, caractérisés en ce que $R_1$ et $R_2$, indépendamment l'un de l'autre, sont chacun un hydrogène ou un radical alkyle en $C_1$ à $C_4$, X est un oxygène ou -$NR_4$- et $R_4$ est un hydrogène ou le radical méthyle ou éthyle, et $R_3$ est un hydrogène, un radical alkyle en $C_1$ à $C_{20}$, cycloalkyle en $C_3$ à $C_7$, alcényle en $C_3$ à $C_{20}$, alcynyle en $C_3$ à $C_{20}$, phényle, naphtyle, halogénalcényle en $C_3$ à $C_{20}$, halogénalcynyle en $C_3$ à $C_{20}$ ; cycloalkyle en $C_3$ à $C_7$ substitué par des substituants fluoro, chloro, bromo ou alkyle en $C_1$ à $C_3$ ; phényle ou naphtyle substitués par des substituants fluoro, chloro, bromo, ou alkyle en $C_1$ à $C_3$, halogénalkyle en $C_1$ à $C_3$, alcoxy en $C_1$ à $C_3$, halogénalcoxy en $C_1$ à $C_3$, alkylthio en $C_1$ à $C_3$, nitro, cyano, phénoxy, halogénophénoxy, (alkyle en $C_1$ à $C_4$)phénoxy, (halogénalkyle en $C_1$ à $C_4$)phénoxy, tri(alkyle en $C_1$ à $C_4$)silyle, N-pyrrolidinyle, N-pipéridinyle, N-pyrrolidine-2-onyle, N-pipéridine-2-onyle, alkylamino en $C_1$ à $C_4$, di-(alkyle en $C_1$ à $C_4$)amino, anilino, N-(alkyle en $C_1$ à $C_4$)amino, N-formylanilino, N-(alkyle en $C_1$ à $C_6$)carbonylanilino, phénylthio ou halogénophénylthio, ou encore alkyle en $C_1$ à $C_{20}$ substitué par des substituants hydroxy, fluoro, chloro, bromo, di-(alkyle en $C_1$ à $C_4$)amino, alcoxy en $C_1$ à $C_4$, halogénalcoxy en $C_1$ à $C_4$, alcoxyalcoxy en $C_2$ à $C_6$, halogénalkylthio en $C_1$ à $C_4$, alkylthio en $C_1$ à $C_4$, alkylsulfinyle en $C_1$ à $C_4$, alkylsulfonyle en $C_1$ à $C_4$, alkylsulfonyloxy en $C_1$ à $C_4$, (alkyle en $C_1$ à $C_4$)carbonyle, (alcoxy en $C_1$ à $C_4$)carbonyle, (alkyle en $C_1$ à $C_6$)carbonyloxy, cycloalkyle en $C_3$ à $C_7$, phényle, phénoxy, phénylthio, phénylsulfonyloxy ou pyridyle, chacun des groupes phényle et pyridyle pouvant être substitué par des substituants fluoro, chloro, bromo, alkyle en $C_1$ à $C_3$, halogénalkyle en $C_1$ à $C_3$, alcoxy en $C_1$ à $C_3$, halogénalcoxy en $C_1$ à $C_3$, alkylthio en $C_1$ à $C_3$, nitro, cyano, phénoxy, halogénophénoxy, phénylthio ou halogénophénylthio.

**8.** Composés selon la revendication 1, caractérisés en ce que $R_1$ et $R_2$, indépendamment l'un de l'autre, sont chacun un hydrogène ou un radical alkyle en $C_1$ à $C_4$, X est un oxygène où -$NR_4$-, $R_4$ est un hydrogène ou le radical méthyle ou éthyle, $R_3$ est un radical phényle, benzyle, naphtyle, 3-pyridylméthyle, ou encore un radical phényle, benzyle, naphtyle ou 3-pyridylméthyle, chacun substitués par des substituants fluoro, chloro, bromo, alkyle en $C_1$ à $C_3$, halogénalkyle en $C_1$ à $C_3$, alcoxy en $C_1$ à $C_3$, halogénalcoxy en $C_1$ à $C_3$, alkylthio en $C_1$ à $C_3$, nitro, cyano, phénoxy, halogénophénoxy, phénylthio ou halogénophénylthio.

**9.** Composés selon la revendication 1, caractérisés en ce que $R_1$ et $R_2$, indépendamment l'un de l'autre, sont chacun un hydrogène ou un radical alkyle en $C_1$ à $C_4$, X est un oxygène ou -$NR_4$-, $R_4$ est un hydrogène ou un radical alkyle en $C_1$ à $C_4$, est un radical alkyle en $C_1$ à $C_{12}$, ou encore un radical alkyle en $C_1$ à $C_{12}$ substitué par des substituants hydroxy, fluoro, chloro, bromo, diméthylamino, méthoxy, éthoxy, méthoxyéthoxy, éthoxyéthoxy, méthylthio, éthylthio, cyclopropyle, cyclopentyle, cyclohexyle, phényle ou phénoxy, les radicaux phényle ou phénoxy pouvant chacun être substitués par des substituants fluoro, chloro, bromo, phénoxy, halogénophénoxy ou phénylthio.

**10.** Composés selon la revendication 1, caractérisés en ce que $R_1$ et $R_2$, indépendamment l'un de l'autre, sont chacun un hydrogène ou un radical alkyle en $C_1$ à $C_4$, X est un oxygène ou -$NR_4$-, $R_4$ est un hydrogène ou un radical alkyle en $C_1$ à $C_4$, $R_3$ est un radical alcényle en $C_3$ à $C_{12}$, alcynyle en $C_3$ à $C_{12}$ ou encore alcényle en $C_3$ à $C_{12}$ ou alcynyle en $C_3$ à $C_{12}$ substitués par des substituants fluoro, chloro ou bromo.

**11.** Composés selon la revendication 3, caractérisés en ce que $R_1$ et $R_2$ sont chacun un hydrogène, et X est un oxygène ou -NH-, -$NCH_3$- ou -$NC_2H_5$-.

**12.** Composés selon la revendication 1, choisis parmi les composés suivants :
4-chloro-4,4-difluorobutyrate de méthyle,
4-chloro-4,4-difluorobutyrate d'éthyle,
4-chloro-4,4-difluorobutyrate d'isopropyle,
4-chloro-4,4-difluorobutyrate de tert-butyle,
4-chloro-4,4-difluorobutyrate de n-butyle,
4-chloro-4,4-difluorobutyrate de (2,2-diméthylpropyle),

4-chloro-4,4-difluorobutyrate de benzyle,
4-chloro-4,4-difluorobutyrate de phényle,
4-chloro-4,4-difluorobutyrate de [2-(4-phénoxyphénoxy)éthyle],
4-chloro-4,4-difluorobutyrate de cyclohexyle,
4-chloro-4,4-difluorobutyrate de cyclohexylméthyle,
4-chloro-4,4-difluorobutyrate de cyclopropylméthyle,
4-chloro-4,4-difluoro-2-trifluorométhylbutyrate d'éthyle,
4-chloro-4,4-difluorobutyro-N-méthylamide,
4-chloro-4,4-difluorobutyro-N,N-diméthylamide,
4-chloro-4,4-difîuorobutyro-N,N-dihexylamide,
4-chloro-4,4-difluorobutyro-N-éthylamide,
4-chloro-4,4-difluorobutyro-N-isopropylamide,
4-chloro-4,4-difluorobutyro-N-butylamide,
4-chloro-4,4-difluorobutyro-N-tert-butylamide,
4-chloro-4,4-difluorobutyro-N-benzylamide,
4-chloro-4,4-difluorobutyro-anilide,
4-chloro-4,4-difluorobutyro-N-méthyl-N-pyrid-3-ylméthylamide,
4-chloro-4,4-difluorobutyro-N-pyrid-3-ylméthylamide,
4-chloro-4,4-difluorobutyro-(4-chloro-anilide),
4-chloro-4,4-difluorobutyro-(4-phénoxy-anilide),
4-chloro-4,4-difluorobutyro-(2-chloro-anilide),
4-chloro-4,4-difluorobutyro-(4-méthoxy-anilide),
4-chloro-4,4-difluorobutyro-(4-méthyl-anilide),
4-chloro-4,4-difluorobutyro-(3-méthylmercapto-anilide),
4-chloro-4,4-difluorobutyro-(4-fluoro-anilide),
4-chloro-4,4-difluorobutyro-(4-chloro-2-nitro-anilide),
4-chloro-4,4-difluorobutyrate de (3-phénoxy-benzyle),
4-chloro-4,4-difluorobutyrate de (4-(4-fluoro-phénoxy)phényle],
4-chloro-4,4-difluorobutyrate de [4-(4-fluoro-phénoxy)phénoxy-éthyle],
4-chloro-4,4-difluorobutyrate de (4-nitro-phényle),
4-chloro-4,4-difluorobutyrate de [4-(3,5-difluorophénoxy)-phényl]-ester,
4-chloro-4,4-difluorobutyrate de (4-(5-trifluorométhylpyrid-2-yloxy)-phényle],
acide 4-chloro-4,4-difluorobutyrique et 4-chloro-4,4-difluorobutyramide.

**13.** Procédé pour préparer les composés de formule I selon la revendication 1, caractérisé en ce que :

a) on fait réagir un halogénure de 4-chloro-4,4-difluorobutyryle de formule II

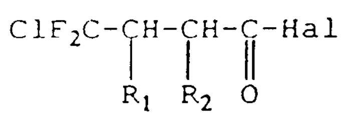

(II)

dans laquelle $R_1$ et $R_2$ ont les significations données pour la formule I et Hal représente un halogène, de préférence le chlore ou le brome, en présence d'une base, avec un composé de formule III

$$H\text{-}X\text{-}R_3 \qquad (III)$$

dans X et $R_3$ ont les significations données pour la formule I, ou bien

b) on fait réagir de l'acide 4-chloro-4,4-difluorobutyrique de formule Ic

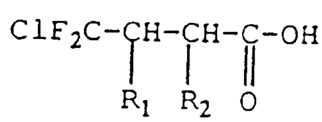

(Ic)

dans laquelle $R_1$ et $R_2$ ont les significations données pour la formule I, en présence d'un agent éliminant l'eau, avec un composé de formule III.

**14.** Produit pesticide contenant comme matière active au moins un composé de formule I selon la revendication 1.

**15.** Pesticide selon la revendication 14, caractérisé en ce qu'il contient, en outre, au moins un support.

**16.** Utilisation d'un composé de formule I selon la revendication pour lutter contre les ravageurs des animaux et des plantes.

**17.** Utilisation selon la revendication 16, caractérisée en ce que, pour ce qui concerne les ravageurs, il s'agit d'insectes et d'araignées ennemis des plantes.

**18.** Procédé pour maîtriser les insectes et araignées ennemis des animaux et des plantes, caractérisé en ce qu'on traite les ravageurs, ou leur espace vital, avec une quantité efficace d'un composé de formule I selon la revendication 1.

**19.** Halogénures de 4-chloro-4,4-difluorobutyryle de formule II

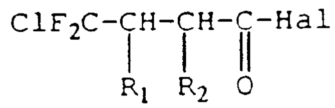

(II)

dans laquelle $R_1$ et $R_2$, indépendamment l'un de l'autre, sont chacun un hydrogène ou un radical alkyle en $C_1$ à $C_4$ ou halogénalkyle en $C_1$ à $C_4$, et Hal est un halogène, de préférence le chlore ou le brome.

**20.** Dérivés de l'acide 4-chloro-4,4-difluorocrotonique de formule VIII

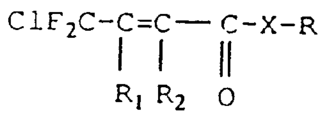

(VIII)

dans laquelle
R est un radical alkyle en $C_1$ à $C_6$,
$R_1$ et $R_2$, indépendamment l'un de l'autre, sont chacun un hydrogène ou un radical alkyle en $C_1$ à $C_4$ ou halogénalkyle en $C_1$ à $C_4$, et X est un oxygène ou $-NR_4-$,
$R_4$ représentant un hydrogène ou un radical alkyle en $C_1$ à $C_6$, du moment que, dans les composés de formule VIII dans laquelle X est un oxygène et R est le radical éthyle, $R_2$ est différent d'un oxygène hydrogène et lorsque $R_1$ est $CF_2H$, $CF_2Cl$ ou $CF_3$.

**21.** Procédé pour préparer les composés de formule I, caractérisé en ce qu'on hydrogène avec de l'hydrogène en présence d'un catalyseur, un dérivé de l'acide 4-chloro-4,4-difluorocrotonique de formule VIII

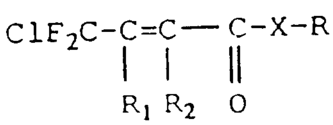

(VIII)

dans laquelle
R est un hydrogène ou un radical alkyle en $C_1$ à $C_6$,
$R_1$ et $R_2$, indépendamment l'un de l'autre, sont chacun un hydrogène ou un radical alkyle en $C_1$ à $C_4$ ou halogénalkyle en $C_1$ à $C_4$,
et
X est un oxygène ou $-NR_4-$,
$R_4$ étant un hydrogène ou un radical alkyle en $C_1$ à $C_6$, puis, par hydrolyse, par une réaction de transestérification ou de transamidation, on convertit en les autres composés de formule I selon la revendication I les composés obtenus de formule Id

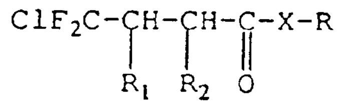

(Id)

dans laquelle $R_1$, $R_2$, X et R ont les significations données ci-dessus.

**Revendications pour l'Etat Contractant suivant : ES**

1. Pesticide, qui contient comme matière active au moins un dérivé de l'acide 4-chloro-4,4-difluorobutyrique de formule I

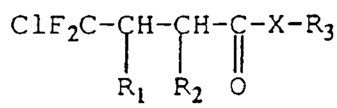

(I)

dans laquelle
$R_1$ et $R_2$, indépendamment l'un de l'autre, sont chacun un hydrogène ou un radical alkyle en $C_1$ à $C_4$ ou halogènalkyle en $C_1$ à $C_4$,
$R_3$ est un hydrogène ou un radical organique, et
X est un oxygène ou $-NR_4-$,
$R_4$ étant un hydrogène ou un radical alkyle en $C_1$ à $C_6$.

2. Pesticide selon la revendication 1, caractérisé en ce que $R_3$ est un hydrogène ou un radical éventuellement substitué alkyle en $C_1$ à $C_{20}$, cycloalkyle en $C_3$ à $C_7$, alcényle en $C_3$ à $C_{20}$, alcynyle en $C_3$ à $C_{20}$, benzyle ou aryle .

3. Pesticide selon la revendication 1, caractérisé en ce que $R_3$ est un hydrogène, un radical alkyle en $C_1$ à $C_{20}$, cycloallkyle en $C_3$ à $C_7$, alcényle en $C_3$ à $C_{20}$, alcynyle en $C_3$ à $C_{20}$, aryle, halogénalcenyle en $C_3$ à $C_{20}$, halogénalcynyle en $C_3$ à $C_{20}$ ; cycloalkyle en $C_3$ à $C_7$ substitué par des substituants halogéno ou alkyle en $C_1$ à $C_4$ ; aryle substitué par des substituants halogéno, alkyle en $C_1$ à $C_4$, halogénalkyle en $C_1$ à $C_4$, alcoxy en $C_1$ à $C_{12}$, halogénalcoxy en $C_1$ à $C_4$, alkylthio en $C_1$ à $C_4$, nitro, cyano, benzoyle, halogénobenzoyle, phénoxy, halogénophénoxy, (alkyle en $C_1$ à $C_4$)phénoxy, (halogéalkyle en $C_1$ à $C_4$)phénoxy, tri-(alkyle en $C_1$ à $C_4$)silyle, N-pyrrolidinyle, N-pipéridinyle, N-pyrrolidine-2-onyle, N- pipéridine-2-onyle, alkylamino en $C_1$ à $C_4$, di-(alkyle en $C_1$ à $C_4$)amino, anilino, N-(alkyle en $C_1$ à $C_4$)anilino, N-formylanilino, N-(alkyle en $C_1$ à $C_6$)carbonylanilino, phénylthio ou halogénophénylthio ; phényle substitué par un hétérocycle mono- ou dicyclique, aromatique ou non aromatique, éventuellement substitué, lié par l'intermédiaire d'un atome d'oxygène ou de soufre, auquel cas l'hétérocycle, tout le comme le noyau phényle, peuvent être chacun substitués par des substituants halogéno, alkyle en $C_1$ à $C_4$, nitro, halogénalkyle en $C_1$ à $C_4$, halogénalcoxy en $C_1$ à $C_4$, alcoxy en $C_1$ à $C_4$, alkylthio en $C_1$ à $C_4$ ou cyclopropyle ; ou encore alkyle en $C_1$ à $C_{20}$ substitué par des substituants hydroxy, halogéno, di- (alkyle en $C_1$ à $C_4$)amino, alcoxy en $C_1$ à $C_4$, halogénalcoxy en $C_1$ à $C_4$, alcoxyalcoxy en $C_2$ à $C_6$, halogénalkylthio en $C_1$ à $C_4$, alkylthio en $C_1$ à $C_4$, alkylsulfinyle en $C_1$ à $C_4$, alkylsulfonyle en $C_1$ à $C_4$, alkylsulfonyloxy en $C_1$ à $C_4$, (alkyle en $C_1$ à $C_4$)carbonyle, (alcoxy en $C_1$ à $C_4$)carbonyle, (alkyle en $C_1$ à $C_6$)carbonyloxy, cycloalkyle en $C_3$ à $C_7$, aryle, aryloxy, arylthio, arylsulfonyle, arylsulfinyle, arylsulfonyloxy, arylcarbonyle ou pyridyle ; les groupes aryle et pyridyle pouvant chacun être substitués par des substituants halogéno, alkyle en $C_1$ à $C_4$, halogénalkyle e n $C_1$ à $C_4$, alcoxy en $C_1$ à $C_4$, halogénalcoxy en $C_1$ à $C_4$, alkylthio en $C_1$ à $C_4$, nitro, cyano, phénoxy, halogénophénoxy, phénylthio ou halogénophénylthio.

4. Pesticide selon la revendication 1, caractérisé en ce que $R_1$ et $R_2$, indépendamment l'un de l'autre, sont chacun un hydrogène ou un radical alkyle en $C_1$ à $C_4$, mais de préférence un hydrogène.

5. Pesticide selon la revendication 1, caractérisé en ce que X est un oxygène, -NH-, $-NCH_3-$ ou $-NC_2H_5-$, mais de préférence un oxygène ou -NH-.

6. Pesticide selon la revendication 3, caractérisé en ce que $R_3$ est un radical phényle substitué en position 4 par un hétérocycle mono- ou bicyclique aromatique, lié par l'intermédiaire d'un oxygène, et choisi parmi

la pyridine, la pyrimidine ou le benzothiazole, les deux noyaux aromatiques étant non substitués, ou portant ensemble au plus trois substituants supplémentaires choisis parmi le chlore, le brome, et les radicaux méthyle, éthyle et trifluorométhyle.

7. Pesticide selon la revendication 3, caractérisé en ce que $R_1$ et $R_2$, indépendamment l'un de l'autre, sont chacun un hydrogène ou un radical alkyle en $C_1$ à $C_4$, X est un oxygène ou $-NR_4-$ et $R_4$ est un hydrogène ou le radical méthyle ou éthyle, et $R_3$ est un hydrogène, un radical alkyle en $C_1$ à $C_{20}$, cycloalkyle en $C_3$ à $C_7$, alcényle en $C_3$ à $C_{20}$, alcynyle en $C_3$ à $C_{20}$, phényle, naphtyle, halogénalcényle en $C_3$ à $C_{20}$, halogénalcynyle en $C_3$ à $C_{20}$ ; cycloalkyle en $C_3$ à $C_7$ substitué par des substituants fluoro, chloro, bromo ou alkyle en $C_1$ à $C_3$ ; phényle ou naphtyle substitués par des substituants fluoro, chloro, bromo, ou alkyle en $C_1$ à $C_3$, halogénalkyle en $C_1$ à $C_3$, alcoxy en $C_1$ à $C_3$, halogénalcoxy en $C_1$ à $C_3$, alkylthio en $C_1$ à $C_3$, nitro, cyano, phénoxy, halogénophénoxy, (alkyle en $C_1$ à $C_4$)phénoxy, (halogénalkyle en $C_1$ à $C_4$)phénoxy, tri (alkyle en $C_1$ à $C_4$)silyle, N-pyrrolidinyle, N-pipéridinyle, N-pyrrolidine-2-onyle, N-pipéridine-2-onyle, alkylamino en $C_1$ à $C_4$, di-(alkyle en $C_1$ à $C_4$)amino, anilino, N-(alkyle en $C_1$ à $C_4$)amino, N-formylanilino, N-(alkyle en $C_1$ à $C_6$)carbonylanilino, phénylthio ou halogénophénylthio, ou encore alkyle en $C_1$ à $C_{20}$ substitué par des substituants hydroxy, fluoro, chloro, bromo, di-(alkyle en $C_1$ à $C_4$)amino, alcoxy en $C_1$ à $C_4$, halogénalcoxy en $C_1$ à $C_4$, alcoxyalcoxy en $C_2$ à $C_6$, halogénalkylthio en $C_1$ à $C_4$, alkylthio en $C_1$ à $C_4$, alkylsulfinyle en $C_1$ à $C_4$, alkylsulfonyle en $C_1$ à $C_4$, alkylsulfonyloxy en $C_1$ à $C_4$, (alkyle en $C_1$ à $C_4$)carbonyle, (alcoxy en $C_1$ à $C_4$)carbonyle, (alkyle en $C_1$ à $C_6$)carbonyloxy, cycloalkyle en $C_3$ à $C_7$, phényle, phénoxy, phénylthio, phénylsulfonyloxy ou pyridyle, chacun des groupes phényle et pyridyle pouvant être substitué par des substituants fluoro, chloro, bromo, alkyle en $C_1$ à $C_3$, halogénalkyle en $C_1$ à $C_3$, alcoxy en $C_1$ à $C_3$, halogénalcoxy en $C_1$ à $C_3$, alkylthio en $C_1$ à $C_3$, nitro, cyano, phénoxy, halogénophénoxy, phénylthio ou halogénophénylthio.

8. Pesticide selon la revendication 1, caractérisé en ce que $R_1$ et $R_2$, indépendamment l' un de l'autre, sont chacun un hydrogène ou un radical alkyle en $C_1$ à $C_4$, X est un oxygène ou $-NR_4-$, $R_4$ est un hydrogène ou le radical méthyle ou éthyle, $R_3$ est un radical phényle, benzyle, naphtyle, 3-pyridylméthyle, ou encore un radical phényle, benzyle, naphtyle ou 3-pyridylméthyle, chacun substitués par des substituants fluoro, chloro, bromo, alkyle en $C_1$ à $C_3$, halogénalkyle en $C_1$ à $C_3$, alcoxy en $C_1$ à $C_3$, halogénalcoxy en $C_1$ à $C_3$, alkylthio en $C_1$ à $C_3$, nitro, cyano, phénoxy, halogénophénoxy, phénylthio ou halogénophénylthio.

9. Pesticide selon la revendication 1, caractérisé en ce que $R_1$ et $R_2$, indépendamment l' un de l'autre, sont chacun un hydrogène ou un radical alkyle en $C_1$ à $C_4$, X est un oxygène ou $-NR_4-$, $R_4$ est un hydrogène ou un radical alkyle en $C_1$ à $C_4$, est un radical alkyle en $C_1$ à $C_{12}$, ou encore un radical alkyle en $C_1$ à $C_{12}$ substitué par des substituants hydroxy, fluoro, chloro, bromo, diméthylamino, méthoxy, éthoxy, méthoxyéthoxy, éthoxyéthoxy, méthylthio, éthylthio, cyclopropyle, cyclopentyle, cyclohexyle, phényle ou phénoxy, les radicaux phényle ou phénoxy pouvant chacun être substitués par des substituants fluoro, chloro, bromo, phénoxy, halogénophénoxy ou phénylthio.

10. Pesticide selon la revendication 1, caractérisé en ce que $R_1$ et $R_2$, indépendamment l'un de l'autre, sont chacun un hydrogène ou un radical alkyle en $C_1$ à $C_4$, X est un oxygène ou $-NR_4-$, $R_4$ est un hydrogène ou un radical alkyle en $C_1$ à $C_4$, $R_3$ est un radical alcényle en $C_3$ à $C_{12}$, alcynyle en $C_3$ à $C_{12}$ ou encore alcényle en $C_3$ à $C_{12}$ ou alcynyle en $C_3$ à $C_{12}$ substitués par des substituants fluoro, chloro ou bromo.

11. Pesticide selon la revendication 3, caractérisé en ce que $R_1$ et $R_2$ sont chacun un hydrogène, et X est un oxygène ou -NH-, $-NCH_3-$ ou $-NC_2H_5-$.

12. Pesticide selon la revendication 1, choisi parmi les composés suivants :
4-chloro-4,4-difluorobutyrate de méthyle,
4-chloro-4,4-difluorobutyrate d'éthyle,
4-chloro-4,4-difluorobutyrate d'isopropyle,
4-chloro-4,4-difluorobutyrate de tert-butyle,
4-chloro-4,4-difluorobutyrate de n-butyle,
4-chloro-4,4-difluorobutyrate de (2,2-diméthylpropyle),
4-chloro-4,4-difluorobutyrate de benzyle,
4-chloro-4,4-difluorobutyrate de phényle,
4-chloro-4,4-difluorobutyrate de [2-(4-phénoxyphénoxy)éthyle],
4-chloro-4,4-difluorobutyrate de cyclohexyle,
4-chloro-4,4-difluorobutyrate de cyclohexylméthyle,

4-chloro-4,4-difluorobutyrate de cyclopropylméthyle,
4-chloro-4,4-difluoro-2-trifluorométhylbutyrate d'éthyle,
4-chloro-4,4-difluorobutyro-N-méthylamide,
4-chloro-4,4-difluorobutyro-N,N-diméthylamide,
4-chloro-4,4-difluorobutyro-N,N-dihexylamide,
4-chloro-4,4-difluorobutyro-N-éthylamide,
4-chloro-4,4-difluorobutyro-N-isopropylamide,
4-chloro-4,4-difluorobutyro-N-butylamide,
4-chloro-4,4-difluorobutyro-N-tert-butylamide,
4-chloro-4,4-difluorobutyro-N-benzylamide,
4-chloro-4,4-difluorobutyro-anilide,
4-chloro-4,4-difluorobutyro-N-méthyl-N-pyrid-3-ylméthylamide,
4-chloro-4,4-difluorobutyro-N-pyrid-3-ylméthylamide,
4-chloro-4,4-difluorobutyro-(4-chloro-anilide),
4-chloro-4,4-difluorobutyro-(4-phénoxy-anilide),
4-chloro-4,4-difluorobutyro-(2-chloro-anilide),
4-chloro-4,4-difluorobutyro-(4-méthoxy-anilide),
4-chloro-4,4-difluorobutyro-(4-méthyl-anilide),
4-chloro-4,4-difluorobutyro-(3-méthylmercapto-anilide),
4-chloro-4,4-difluorobutyro-(4-fluoro-anilide),
4-chloro-4,4-difluorobutyro-(4-chloro-2-nitro-anilide),
4-chloro-4,4-difluorobutyrate de (3-phénoxy-benzyle),
4-chloro-4,4-difluorobutyrate de [4-(4-fluoro-phénoxy)phényle],
4-chloro-4,4-difluorobutyrate de [4-(4-fluoro-phénoxy)phénoxy-éthyle],
4-chloro-4,4-difluorobutyrate de (4-nitro-phényle),
4-chloro-4,4-difluorobutyrate de [4-(3,5-difluorophénoxy)-phényl]-ester,
4-chloro-4,4-difluorobutyrate de [4-(5-trifluorométhylpyrid-2-yloxy)-phényle],
acide 4-chloro-4,4-difluorobutyrique et 4-chloro-4,4-difluorobutyramide.

**13.** Procédé pour préparer les composés de formule I selon la revendication 1, caractérisé en ce que :
a) on fait réagir un halogénure de 4-chloro-4,4-difluorobutyryle de formule II

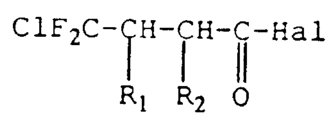

(II)

dans laquelle $R_1$ et $R_2$ ont les significations données pour la formule I et Hal représente un halogène, de préférence le chlore ou le brome, en présence d'une base, avec un composé de formule III

$$H\text{-}X\text{-}R_3 \qquad (III)$$

dans X et $R_3$ ont les significations données pour la formule I, ou bien
b) on fait réagir de l'acide 4-chloro-4,4-difluorobutyrique de formule Ic

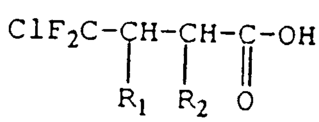

(Ic)

dans laquelle $R_1$ et $R_2$ ont les significations données pour la formule I, en présence d'un agent éliminant l'eau, avec un composé de formule III.

**14.** Pesticide selon la revendication 14, caractérisé en ce qu'il contient, en outre, au moins un support.

**15.** Utilisation d'un composé de formule I selon la revendication pour lutter contre les ravageurs des animaux et des plantes.

**16.** Utilisation selon la revendication 16, caractérisée en ce que, pour ce qui concerne les ravageurs, il s'agit

d'insectes et d'araignées ennemis des plantes.

**17.** Procédé pour maîtriser les insectes et araignées ennemis des animaux et des plantes, caractérisé en ce qu'on traite les ravageurs ou leur espace vital avec une quantité efficace d'un composé de formule I selon la revendication 1.

**18.** Procédé pour préparer des halogénures de 4-chloro-4,4-difluorobutyryle de formule II

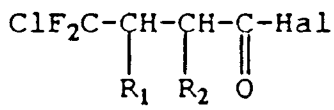

(II)

dans laquelle $R_1$ et $R_2$, indépendamment l'un de l'autre, sont chacun un hydrogène ou un radical alkyle en $C_1$ à $C_4$ ou halogénalkyle en $C_1$ à $C_4$ et Hal est un halogène, de préférence le chlore ou le brome, caractérisé en ce qu'on fait réagir avec un agent d'halogénation un acide 4-chloro-4,4-difluorobutyrique de formule Ic

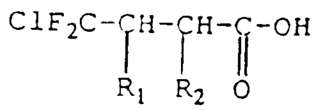

(Ic)

dans laquelle $R_1$ et $R_2$ ont les significations données ci-dessus.

**19.** Procédé pour préparer les dérivés de l'acide 4-chloro-4,4-difluorocrotonique de formule VIIIa

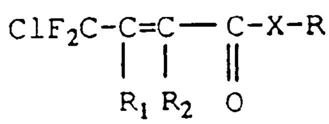

dans laquelle
R est un radical alkyle en $C_1$ à $C_6$,
$R_1$ et $R_2$ sont chacun un hydrogène et X est un oxygène ou -$NR_4$-,
$R_4$ étant un hydrogène ou un radical alkyle en $C_1$ à $C_6$, caractérisé en ce que, par élimination β, on supprime l'eau ou l'acide H-O-A des composés de formule IX

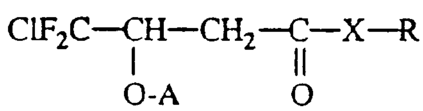

(IX)

dans laquelle A est un hydrogène ou un groupe acyle.

**20.** Procédé pour préparer les composés de formule I, caractérisé en ce qu'on hydrogène avec de l'hydrogène, en présence d'un catalyseur un dérivé de l'acide 4-chloro-4,4-difluorocrotonique de formule VIII

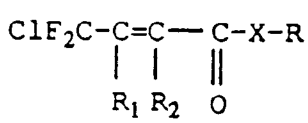

(VIII)

dans laquelle
R est un radical alkyle en $C_1$ à $C_6$,

$R_1$ et $R_2$, indépendamment l'un de l'autre, sont chacun un hydrogène ou un radical alkyle en $C_1$ à $C_4$ ou halogénalkyle en $C_1$ à $C_4$,
et
et X est un oxygène ou $-NR_4-$,
$R_4$ représentant un hydrogène ou un radical alkyle en $C_1$ à $C_6$, et par hydrolyse, transestérification ou transamidation, on convertit, en les autres composés de formule I selon la revendication 1, les composés obtenus de formule Id

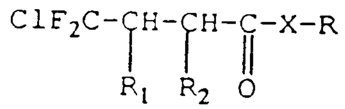

(Id)

dans laquelle $R_1$, $R_2$, X et R ont les significations données ci-dessus.